(19) Europäisches Patentamt
European Patent Office
Office européen des brevets

(11) EP 1 246 790 B1

(12) EUROPÄISCHE PATENTSCHRIFT

(45) Veröffentlichungstag und Bekanntmachung des Hinweises auf die Patenterteilung:
**04.05.2005 Patentblatt 2005/18**

(21) Anmeldenummer: **00990799.9**

(22) Anmeldetag: **20.12.2000**

(51) Int Cl.[7]: **C07C 215/50**, C07C 217/58, A61K 31/136, A61K 31/16, A61K 31/165, A61K 31/195, C07D 295/08, C07C 219/28, C07C 225/16, C07C 229/38, C07C 237/48, C07C 243/38, C07C 311/37, C07D 295/12, C07D 295/14, A61P 25/04, A61P 29/00

(86) Internationale Anmeldenummer:
**PCT/EP2000/012972**

(87) Internationale Veröffentlichungsnummer:
**WO 2001/047866 (05.07.2001 Gazette 2001/27)**

(54) **SUBSTITUIERTE 1- UND 2-NAPHTHOL-MANNICHBASEN**

SUBSTITUTED 1 AND 2 NAPHTHOL MANNICH BASES

COMPOSES 1-2-NAPHTOL/BASES DE MANNICH SUBSTITUES

(84) Benannte Vertragsstaaten:
**AT BE CH CY DE DK ES FI FR GB GR IE IT LI LU MC NL PT SE TR**
Benannte Erstreckungsstaaten:
**LT LV SI**

(30) Priorität: **27.12.1999 DE 19963179**

(43) Veröffentlichungstag der Anmeldung:
**09.10.2002 Patentblatt 2002/41**

(73) Patentinhaber: **Grünenthal GmbH
52078 Aachen (DE)**

(72) Erfinder:
- **GERLACH, Matthias
63636 Brachttal (DE)**
- **MAUL, Corinna
52066 Aachen (DE)**

(74) Vertreter: **Kutzenberger, Helga, Dr. et al
Kutzenberger & Wolff,
Theodor-Heuss-Ring 23
50668 Köln (DE)**

(56) Entgegenhaltungen:
**EP-A- 0 864 559**

- **CARDELLICCHIO C ET AL: "The Betti base: absolute configuration and routes to a family of related chiral nonracemic bases" TETRAHEDRON: ASYMMETRY,NL,ELSEVIER SCIENCE PUBLISHERS, AMSTERDAM, Bd. 9, Nr. 20, 23. Oktober 1998 (1998-10-23), Seiten 3667-3675, XP004141842 ISSN: 0957-4166**
- **DATABASE WPI Section Ch, Week 199635 Derwent Publications Ltd., London, GB; Class B05, AN 1996-354450 XP002166569 & WO 96 22276 A (NIPPON SHINYAKU CO LTD), 25. Juli 1996 (1996-07-25)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft substituierte 1- und 2-Naphthol-Mannichbasen, Verfahren zu deren Herstellung, Arzneimittel enthaltend diese Verbindungen sowie die Verwendung dieser Verbindungen zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie z.B. das Morphin, sind bei der Therapie starker bis sehr starker Schmerzen wirksam. Als unerwünschte Begleiterscheinungen weisen sie jedoch unter anderem Atemdepression, Erbrechen, Sedierung, Obstipation sowie eine Toleranzentwicklung auf.

Tramadolhydrochlorid - (1 RS, 2RS) - 2-[(Dimethylamino)methyl]-1-(3-methoxyphenyl)-cyclohexanol - nimmt unter den zentral wirksamen Analgetika eine Sonderstellung ein, da dieser Wirkstoff eine starke Schmerzhemmung ohne die für Opioide bekannten Nebenwirkungen hervorruft. (J. Pharmacol. Exptl. Ther. 267, 33 (1993)): WO 96 222 76 beschreibt 1-Phenyl-2-arylethylamine mit analgetischer Wirkung. Nach weiteren schmerzhemmenden Mitteln wird weltweit geforscht.

Aufgabe der vorliegenden Erfindung war es daher, neue Verbindungen zur Verfügung zu stellen, die sich insbesondere als Wirkstoffe in Arzneimitteln eignen.

Diese Wirkstoffe sollen sich insbesondere zur Schmerztherapie und zur Behandlung von inflammatorischen und allergischen Reaktionen, Drogenund/oder Alkoholmißbrauch, Diarrhoe, Gastritis, Ulcera, cardiovaskulären Erkrankungen, Harninkontinenz, Depressionen, Schockzuständen, Migräne, Narkolepsie, Übergewicht, Asthma, Glaukom und/oder hyperkinetischem Syndrom eignen. Erfindungsgemäß wird diese Aufgabe durch die Bereitstellung substituierter 1- und 2-Naphthol-Mannichbasen der nachstehenden allgemeinen Formel I gelöst, die eine ausgeprägte analgetische Wirkung aufweisen und die sich darüber hinaus zur Behandlung von inflammatorischen und allergischen Reaktionen, Drogen- und/oder Alkoholmißbrauch, Diarrhoe, Gastritis, Ulcera, cardiovaskulären Erkrankungen, Harninkontinenz, Depressionen, Schockzuständen, Migräne, Narkolepsie, Übergewicht, Asthma, Glaukom und/oder hyperkinetischem Syndrom eignen.

Gegenstand der Erfindung sind daher substituierte 1- und 2- Naphthol-Mannichbasen der allgemeinen Formel I,

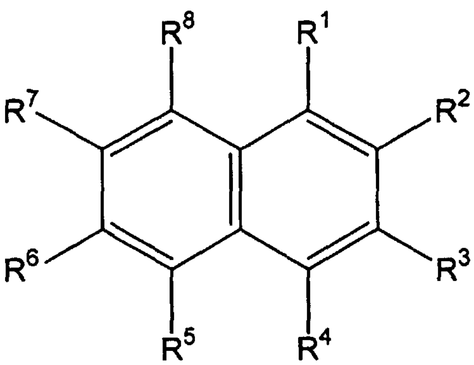

I

worin
$R^1 = CH(R^9)N(R^{10})(R^{11})$ und $R^2 = OR^{12}$ bedeuten
oder
$R^1 = OR^{12}$ und $R^2 = CH(R^9)N(R^{10})(R^{11})$ bedeuten,
und jeweils die Reste
$R^3$ bis $R^8$, gleich oder verschieden, = H, F, Cl, Br, $CF_3$, CN, $NO_2$, $SO_2NH_2$, $SO_2NHR^{13}$, $NHR^{13}$, $SR^{15}$, $OR^{16}$, $CO(OR^{20})$, $CH_2CO(OR^{21})$, $CO(R^{22})$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise = H, F, Cl, Br, $SO_2NH_2$, $NHR^{13}$, $CO(R^{22})$, $OR^{16}$, $CO(OR^{20})$, einen $C_{1-6}$-Alkyl- oder einen über eine $C_{1-2}$-Alkylen-Gruppe gebundenen Aryl-Rest, besonders bevorzugt H, $NHR^{13}$,

$CO(R^{22})$, $OR^{16}$, $CO(OR^{20})$, bedeuten,

$R^9$ einen Aryl-Rest, einen Heteroaryl-Rest oder einen Alkyl-Rest ohne acides Proton in α-Stellung, vorzugsweise einen unsubstituierten Phenyl-Rest oder einen wenigstens einfach mit $C_{1-4}$-Alkyl-, $C_{1-3}$-Alkoxy-, Halogen-, $CF_3$-, CN-, O-Phenyl- oder OH substituierten Phenyl-Rest, besonders bevorzugt einen unsubstituierten Phenyl-Rest oder einen 2-Methoxy-phenyl-, 3-Methoxy-phenyl-, 4-Methoxy-phenyl-, 2-Methyl-phenyl-, 3-Methyl-phenyl-, 4-Methyl-phenyl-, 2-tert-Butyl-phenyl-, 3-tert-Butyl-phenyl-, 4-tert-Butyl-phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 5-Brom-2-Fluorphenyl-, 2-Chlor-4-Fluor-phenyl-, 2-Chlor-5-Fluor-phenyl-, 2-Chlor-6-Fluorphenyl-, 4-Brom-2-Fluor-phenyl-, 3-Brom-4-Fluor-phenyl-, 3-Brom-2-Fluorphenyl-, 2,3-Dichlor-phenyl-, 2,4-Dichlor-phenyl-, 2,5-Dichlorphenyl-, 3,4-Dichlor-phenyl-, 2,3-Dimethyl-phenyl-, 2,4-Dimethyl-phenyl-, 2,5-Dimethylphenyl-, 2,3-Dimethoxy-phenyl-, 2,4-Dimethoxy-phenyl-, 2,5-Dimethoxy-phenyl-, 3,4-Dimethoxy-phenyl-, 3,4,5-Trimethoxy-phenyl-, 2-Trifluormethyl-phenyl, 3-Trifluormethyl-phenyl oder einen 4-Trifluormethyl-phenyl-Rest, ganz besonders bevorzugt einen unsubstituierten Phenyl-Rest, bedeutet,

$R^{10}$, $R^{11}$, gleich oder verschieden, einen verzweigten, unverzweigten, gesättigten, ungesättigten, unsubstituierten oder wenigstens einfach substituierten $C_{1-6}$-Alkyl-Rest oder einen unsubstituierten oder wenigstens einfach substituierten Phenyl-, Benzyl- oder Phenethyl-Rest, vorzugsweise einen gesättigten, unsubstituierten oder wenigstens einfach substituierten $C_{1-6}$-Alkyl-Rest, besonders bevorzugt einen $CH_3$-Rest, bedeuten,

oder $R^{10}$ und $R^{11}$ zusammen $(CH_2)_n$ mit n = eine ganze Zahl von 3 bis 6 oder $(CH_2)_2O(CH_2)_2$, vorzugsweise $(CH_2)_n$ mit n = 4 oder 5, bedeuten,

$R^{12}$ = H, $COR^{22}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise = H, einen $C_{1-6}$-Alkyl- oder einen über eine $C_{1-2}$-Alkylen-Gruppe gebundenen Aryl-Rest bedeutet,

$R^{13}$ = H, $COR^{14}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise = H, einen $C_{1-6}$-Alkyl- oder einen über eine $C_{1-2}$-Alkylen-Gruppe gebundenen Aryl-Rest, besonders bevorzugt = H, bedeutet,

$R^{14}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^{15}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^{16}$ = H, $CO(R^{17})$, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise H, einen $C_{1-6}$-Alkyl-, einen über eine $C_{1-2}$-Alkylen-Gruppe gebundenen Aryl-Rest oder $CO(R^{17})$, besonders bevorzugt H oder $CO(R^{17})$, bedeutet,

$R^{17}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise einen $C_{1-6}$-Alkyl-, einen über eine $C_{1-2}$-Alkylen-Gruppe gebundenen Aryl-Rest oder einen gegebenenfalls mit F-, Cl-, Br-, $C_{1-4}$-Alkyl-, $C_{1-3}$-Alkoxy- substituierten Phenyl-Rest, besonders bevorzugt einen gegebenenfalls mit F-, Cl-, Br-, $C_{1-4}$-Alkyl-, $C_{1-3}$-Alkoxy- substituierten Phenyl-Rest, bedeutet,

$R^{18}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise einen $C_{1-6}$-Alkyl-, einen über eine $C_{1-2}$-Alkylen-Gruppe gebundenen Aryl-Rest oder einen gegebenenfalls mit F-, Cl-, Br-, $C_{1-4}$-Alkyl-, oder $C_{1-3}$-Alkoxy- substituierten Phenyl- oder Naphthyl-Rest, besonders bevorzugt einen gegebenenfalls mit F-, Cl-, Br-,

$C_{1-4}$-Alkyl-, $C_{1-3}$-Alkoxy- substituierten Phenyl-Rest, bedeutet,

$R^{20}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise H, einen $C_{1-6}$-Alkyl-, einen über eine $C_{1-2}$-Alkylen-Gruppe gebundenen Aryl-Rest oder einen gegebenenfalls mit F-, Cl-, Br-, $C_{1-4}$-Alkyl-, oder $C_{1-3}$-Alkoxysubstituierten Phenyl-Rest, besonders bevorzugt H oder einen gegebenenfalls mit F-, Cl-, Br-, $C_{1-4}$-Alkyl-, $C_{1-3}$-Alkoxy- substituierten Phenyl-Rest, bedeutet,

$R^{21}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise = H, einen

$C_{1-6}$-Alkyl- oder einen über eine $C_{1-2}$-Alkylen-Gruppe gebundenen Aryl-Rest bedeutet,

$R^{22}$ = H, $NHNH_2$, $NHR^{18}$, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise H, einen $C_{1-6}$-Alkyl-, einen über eine $C_{1-2}$-Alkylen-Gruppe gebundenen Aryl-Rest, $NHNH_2$, $NHR'^{8}$ oder einen gegebenenfalls mit F-, Cl-, Br-, $C_{1-4}$-Alkyl-, oder $C_{1-3}$-Alkoxy- substituierten Phenyl-Rest, besonders bevorzugt $NHNH_2$, $NHR^{18}$ oder einen gegebenenfalls mit F-, Cl-, Br-, $C_{1-4}$-Alkyl-, oder $C_{1-3}$-Alkoxy- substituierten Phenyl-Rest, ganz besonders bevorzugt $NHNH_2$ oder $NHR^{18}$, bedeutet

und/oder deren Racemate, Enantiomere, Diastereomere und/oder entsprechende Basen und/oder entsprechende Salze von physiologisch verträglichen Säuren,

wobei

die Racemate der Verbindungen, in denen die Reste $R^1 = CH(R^9)N(R^{10})(R^{11})$ und $R^2 = OR^{12}$ sind und jeweils die Reste $R^3$ bis $R^8$ und $R^{12}$ = H, der Rest $R^9$ = Phenyl, 2-Chlorphenyl, 4-Methoxyphenyl, 3-Fluorphenyl, 3-Chlorphenyl, 3-Bromphenyl, 4-Bromphenyl, 2-Fluorphenyl, 2-Bromphenyl, Benzo-1,3-dioxol, 4-$CH_3$OCO-phenyl- oder 2-Methoxyphenyl und die Reste $R^{10}$ und $R^{11}$ zusammen = $(CH_2)_5$ bedeuten
oder
die Reste $R^3$ bis $R^8$ und $R^{12}$ = H, der Rest $R^9$ = Phenyl, 4-Methoxyphenyl, 4-Dimethylaminophenyl-, 4-Hydroxy-2,3-Di-tert-Butylphenyl, 2,3-Dihydrobenzodioxan, 4-Nitrophenyl- oder Benzo-1,3-dioxol und die Reste $R^{10}$ und $R^{11}$ zusammen = $(CH_2)_2O(CH_2)_2$ bedeuten
oder
die Reste $R^3$ bis $R^8$ und $R^{12}$ = H, der Rest $R^9$ = 4-Methoxyphenyl und die Reste $R^{10}$ und $R^{11}$ zusammen = $(CH_2)_4$ bedeuten
oder
der Rest $R^3 = CO(OR^{20})$, die Reste $R^4$ bis $R^8$ und $R^{12}$ = H, der Rest $R^9$ = Phenyl, 4-Methoxyphenyl, 4-Methylphenyl, 4-Nitrophenyl, p-Benzaldehyd, die Reste $R^{10}$ und $R^{11}$ zusammen = $(CH_2)_5$ und der Rest $R^{20} = CH_3$ bedeuten
oder
die Reste $R^3$ bis $R^8$ und $R^{12}$ = H, der Rest $R^9$ = Phenyl und beide Reste $R^{10}$ und $R^{11}$ jeweils = $CH_3$, $C_2H_5$ oder n-$C_3H_7$ bedeuten
oder
die Reste $R^3$ bis $R^8$ und $R^{12}$ = H, der Rest $R^9$ = 4-Methoxyphenyl oder Benzo-1,3-dioxol und die Reste $R^{10}$ und $R^{11}$ jeweils = $CH_3$ bedeuten
oder
die Reste $R^3$ bis $R^5$, $R^7$, $R^8$, $R^{12}$ = H, der Rest $R^6$ = Br, der Rest $R^9$ = Phenyl und die Reste $R^{10}$ und $R^{11}$ zusammen = $(CH_2)_5$ bedeuten
oder
die Reste $R^3$ bis $R^5$, $R^7$, $R^8$, $R^{12}$ = H, der Rest $R^6$ = Br, der Rest $R^9$ = 4-Hydroxy-3,5-Di-tert-Butylphenyl und die Reste $R^{10}$ und $R^{11}$ zusammen = $(CH_2)_2O(CH_2)_2$ bedeuten
oder
die Reste $R^3$ bis $R^8$ und $R^{12}$ = H, der Rest $R^9$ = Phenyl und die Reste $R^{10}$ und $R^{11}$ jeweils = $CH_3$ bedeuten als Hydrochlorid
oder
die Reste $R^3$ bis $R^8$ und $R^{12}$ = H, der Rest $R^9$ = Phenyl oder 4-Methoxyphenyl und die Reste $R^{10}$ und $R^{11}$ zusammen = $(CH_2)_5$ bedeuten als Hydrochlorid
oder
der Rest $R^3 = CO(OR^{20})$, die Reste $R^4$ bis $R^8$ und $R^{12}$ = H, der Rest $R^9$ = Phenyl, die Reste $R^{10}$ und $R^{11}$ zusammen = $(CH_2)_5$ und der Rest $R^{20} = CH_3$ bedeuten als Hydrochlorid
oder
die Reste $R^3$ bis $R^8$ und $R^{12}$ = H, der Rest $R^9$ = Thiophen und die Reste $R^{10}$ und $R^{11}$ zusammen = $(CH_2)_2O(CH_2)_2$ bedeuten
oder
die Reste $R^3$ bis $R^8$ = H, der Rest $R^{12} = CH_3$, Der Rest $R^9$ = Thiophen, 4-Methoxyphenyl oder 3,4-Dimethoxyphenyl und die Reste $R^{10}$ und $R^{11}$ zusammen = $(CH_2)_2O(CH_2)_2$ bedeuten
und die Enantiomere der Verbindung der allgemeinen Formel I, in der $R^1 = CH(R^9)N(R^{10})(R^{11})$ und $R^2 = OR^{12}$ ist und die Reste $R^3$ bis $R^8$, $R^{12}$ = H, $R^9$ = Phenyl, $R^{10}$ und $R^{11}$ zusammen = $(CH_2)_5$ bedeuten
(+)-1-($\alpha$-N,N-Dimethylaminobenzyl)-2-naphtol sowie das entsprechende Tartrat,
(-)-1-($\alpha$-N,N-Dimethylaminobenzyl)-2-naphtol sowie das entsprechende Tartrat, und
(-)-[(2-Methoxynaphth-1-yl)benzyl]-dimethylamin
sowie
die Racemate der Verbindungen in denen die Reste $R^1 = OR^{12}$ und $R^2 = CH(R^9)N(R^{10})(R^{11})$ sind und jeweils die Reste $R^3$ bis $R^8$ und $R^{12}$ = H, der Rest $R^9$ = Phenyl, 2-Bromphenyl, 3-Bromphenyl oder 4-Bromphenyl und die Reste $R^{10}$ und $R^{11}$ zusammen = $(CH_2)_5$ bedeuten
oder
$R^3$ bis $R^8$ und $R^{12}$ = H, der Rest $R^9$ = Phenyl oder 2-Nitrophenyl und die Reste $R^{10}$ und $R^{11}$ zusammen = $(CH_2)_2O(CH_2)_2$ bedeuten
oder
$R^3$, $R^4$, $R^6$, $R^8$ und $R^{12}$ = H, die Reste $R^5$, $R^7$ = $CH_3$, der Rest $R^9$ = Phenyl oder 4-Methoxyphenyl und die Reste $R^{10}$ und $R^{11}$ zusammen = $(CH_2)_5$ bedeuten
oder
$R^3$ bis $R^6$, $R^8$, $R^{12}$ = H, der Rest $R^7$ = $CH_3$, der Rest $R^9$ = 4-Methoxyphenyl oder Phenyl und die Reste $R^{10}$, $R^{11}$

zusammen = $(CH_2)_5$ bedeuten
oder
$R^3$ bis $R^8$ und $R^{12}$ = H, der Rest $R^9$ = Phenyl, der Rest $R^{10}$ = $CH_3$ und der Rest $R^{11}$ = $C_6H_{11}$ oder die Reste $R^{10}$ und $R^{11}$ jeweils = $CH_3$ bedeuten
oder
$R^3$ bis $R^6$, $R^8$, $R^{12}$ = H, der Rest $R^7$ = $CH_3$, der Rest $R^9$ = Phenyl oder 4-Methoxyphenyl und die Reste $R^{10}$ und $R^{11}$ zusammen = $(CH_2)_2O(CH_2)_2$ bedeuten
oder
$R^3$, $R^4$, $R^6$, $R^8$, $R^{12}$ = H, die Reste $R^5$ und $R^7$ = $CH_3$, der Rest $R^9$ = 4-Methoxyphenyl und die Reste $R^{10}$ und $R^{11}$ zusammen = $(CH_2)_2O(CH_2)_2$ bedeuten
oder
$R^3$ bis $R^8$, $R^{12}$ = H, der Rest $R^9$ = Phenyl und die Reste $R^{10}$ und $R^{11}$ zusammen = $(CH_2)_2O(CH_2)_2$ bedeuten als Hydrochlorid
ausgenommen sind.

Unter Alkyl-Resten werden vorzugsweise mindestens einfach mit Halogen-, CN-, $CF_3$- und/oder OH-, besonders bevorzugt mit F-, Cl-, Br- oder OH-, substituierte Kohlenwasserstoff-Reste verstanden. Enthalten diese mehr als einen Substituenten, so können diese Substituenten gleich oder verschieden sein. Die Alkyl-Reste können verzweigt, unverzweigt oder cyclisch sein. Besonders bevorzugt sind die Alkyl-Reste Methyl, Ethyl, Propyl, 1-Methylethyl, Butyl, 1-Methylpropyl, 2-Methylpropyl, 1,1-Dimethylpropyl, 1,2-Dimethylpropyl, 2,2-Dimethylpropyl, Hexyl, 1-Methylpentyl, Heptyl, Nonyl oder Decanyl.

Unter einem Aryl-Rest werden vorzugsweise mindestens einfach, mit einem OH-, einem Halogen-, vorzugsweise F, Br oder Cl, einem $CF_3$-, einem CN-, einem $C_{1-6}$-Alkyl-, einem $C_{1-6}$-Alkoxy- oder einem Phenyl-Rest substituierte Phenyl- oder Naphthyl-Reste verstanden. Die unsubstituierten oder substituierten Phenyl-Reste können auch mit weiteren Ringen kondensiert sein. Besonders bevorzugt sind die Aryl-Reste 2-, 3-, 4-Bromphenyl, 4-Brom-2-fluorphenyl, 5-Brom-2-fluorphenyl, 3-Brom-4-fluorphenyl, 4-tert.-Butylphenyl, 2-Chlor-4-fluorphenyl, 2-Chlor-6-fluorphenyl, 4-Cyanophenyl, 2,3-Dichlorphenyl, 2,4-Dichlorphenyl, 2,6-Dichlorphenyl, 3,4-Dichlorphenyl, 2,3-Dimethoxyphenyl, 3,4-Dimethoxyphenyl, 2,4-Dimethylphenyl, 2,5-Dimethylphenyl, 2-, 3-, 4-Fluorphenyl, 2-Methoxyphenyl, 2-,3-,4-Methylphenyl, 3-Phenoxyphenyl, 2-,4-Trifluormethylphenyl oder 3,4,5-Trimethoxyphenyl.

Unter einem Heteroaryl-Rest werden aromatische Verbindungen verstanden, die wenigstens ein Heteroatom, vorzugsweise Stickstoff- und/oder Sauerstoff- und/oder Schwefel, besonders bevorzugt Stickstoff und/oder Sauerstoff aufweisen und die vorzugsweise mit einem Halogen-, einem CN-, einem $CF_3$- oder OH-Rest substituiert sein können. Ganz besonders bevorzugt ist der Heteroaryl-Rest ein mit substituierter oder unsubstituierter Thiophen-, Pyrrolyl- oder Furfuryl-Rest.

Besonders bevorzugt sind folgende substituierte 1- und 2-Naphthol-Mannichbasen:

6-(Dimethylaminophenylmethyl)-5-hydroxy-naphthalin-1-sulfonsäureamid

4-Amino-2-(dimethylaminophenylmethyl)-naphthalin-1-ol

4-(Dimethylaminophenylmethyl)-3-hydroxy-naphthalin-2-carbonsäurehydrazid

4-(Dimethylaminophenylmethyl)-3-hydroxy-naphthalin-2-carbonsäuremethylester

4-(Dimethylaminophenylmethyl)-3-hydroxy-naphthalin-2-carbonsäure

4-(Dimethylaminophenylmethyl)-3-hydroxy-naphthalin-2-carbonsäurephenylester

[5-(Dimethylaminophenylmethyl)-6-hydroxy-naphthalin-2-yl]-phenylmethanon

3-Amino-1-(dimethylaminophenylmethyl)-naphthalin-2-ol

4-(Dimethylaminophenylmethyl)-3-hydroxy-naphthalin-2-carbonsäure-(2-methoxy-phenyl)-amid

4-(Dimethylaminophenylmethyl)-3-hydroxy-naphthalin-2-carbonsäure-*o*-tolylamid

4-(Dimethylaminophenylmethyl)-3-hydroxy-naphthalin-2-carbonsäurenaphthalin-1-ylamid

4-(Dimethylaminophenylmethyl)-3-hydroxy-7-methoxy-naphthalin-2-carbonsäure

5-(Dimethylaminophenylmethyl)-6-hydroxy-naphthalin-2-carbonsäure

1-(Dimethylaminophenylmethyl)-7-methoxy-naphthalin-2-ol

1-(Dimethylaminophenylmethyl)-6-methoxy-naphthalin-2-ol

5-(Dimethylaminophenylmethyl)-6-hydroxy-naphthalin-1-carbonsäure

4-(Dimethylaminophenylmethyl)-3-hydroxy-7-methoxy-naphthalin-2-carboxylat-Natriumsalz

4-Chlor-2-(morpholin-4-yl-*o*-tolylmethyl)-naphthalin-1-ol

4-Chlor-2-(piperidin-1-yl-*o*-tolylmethyl)-naphthalin-1-ol

4-Chlor-2-[(2-chlorophenyl)-piperidin-1-yl-methyl]-naphthalin-1-ol

4-Chlor-2-[(2,3-dimethoxyphenyl)-morpholin-4-yl-methyl]-naphthalin-1-ol

5-Amino-2-[(2-chlorophenyl)-piperidin-1-yl-methyl]-naphthalin-1-ol

5-Amino-2-[(2,3-dimethoxyphenyl)-morpholin-4-yl-methyl]-naphthalin-1-ol

3-Hydroxy-4-(piperidin-1-yl-*o*-tolylmethyl)-naphthalin-2-carbonsäurehydrazid

7-Methoxy-1-(morpholin-4-yl-*o*-tolylmethyl)-naphthalin-2-ol

1-[(2-Chlorophenyl)-piperidin-1-yl-methyl]-7-methoxy-naphthalin-2-ol

1-[(2,3-Dimethoxyphenyl)-morpholin-4-yl-methyl]-7-methoxy-naphthalin-2-ol

6-Brom-1-[(2-methoxyphenyl)-morpholin-4-yl-methyl]-naphthalin-2-ol

6-Hydroxy-5-[(2-methoxyphenyl)-morpholin-4-yl-methyl]-naphthalin-1-carbonsäure

7-Methoxy-1-[(2-methoxyphenyl)-morpholin-4-yl-methyl]-naphthalin-2-ol

6-Methoxy-1-[(2-methoxyphenyl)-morpholin-4-yl-methyl]-naphthalin-2-ol

4-Chlor-2-[(2-methoxyphenyl)-piperidin-1-yl-methyl]-naphthalin-1-ol

6-Brom-1-[(2-methoxyphenyl)-piperidin-1-yl-methyl]-naphthalin-2-ol

6-Methoxy-1-[(2-methoxyphenyl)-piperidin-1-yl-methyl]-naphthalin-2-ol

7-Methoxy-1-[(2-methoxyphenyl)-piperidin-1-yl-methyl]-naphthalin-2-ol

5-Chlor-2-[dimethylamino-(2-methoxyphenyl)-methyl]-naphthalin-1-ol

{[1-(4-Methoxybenzyloxy)-naphthalin-2-yl]-phenylmethyl}-dimethylamin

{[2-(4-Methoxybenzyloxy)-naphthalin-1-yl]-phenylmethyl}-dimethylamin

4-Methoxybenzoesäure 1-(dimethylaminophenylmethyl)-naphthalin-2-yl-ester

2-Chlorobenzoesäure-1-(dimethylaminophenylmethyl)-naphthalin-2-yl-ester

1-(Morpholin-4-yl-phenylmethyl)-naphthalin-2-ol

1-(Phenylpiperidin-1-yl-methyl)-naphthalin-2-ol

2-[(4-Fluor-phenyl)-pyrrolidin-1-yl-methyl]-naphthalin-1-ol.

Ein weiterer Gegenstand der Erfindung sind Verfahren zur Herstellung von substituierten 1- und 2-Naphthol-Mannichbasen der allgemeinen Formel I, in denen der Rest $R^{12}$ für H steht und die Reste $R^1$ bis $R^{11}$, die Reste $R^{13}$ bis $R^{18}$ und die Reste $R^{20}$ bis $R^{22}$ die Bedeutung gemäß der allgemeinen Formel I haben, die dadurch gekennzeichnet sind, daß man
aromatische Aldehydverbindungen, heteroaromatische Aldehydverbindungen und/oder aliphatische Aldehydverbindungen der allgemeinen Formel II,

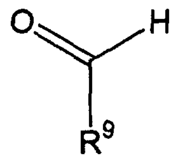

II

in denen $R^9$ die Bedeutung gemäß der allgemeinen Formel I hat, in Lösung, vorzugsweise in einem organischen Lösungsmittel, besonders bevorzugt in Toluol, in Gegenwart einer Base, vorzugsweise Kaliumcarbonat oder Borsäureanhydrid, bevorzugt bei einer Temperatur von -10 °C bis + 110°C mit sekundären Aminen der allgemeinen Formel III,

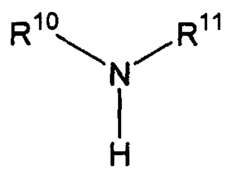

III

in denen die Reste $R^{10}$ und $R^{11}$ die Bedeutung gemäß der allgemeinen Formel I haben,
zu Aminalverbindungen der allgemeinen Formel IV

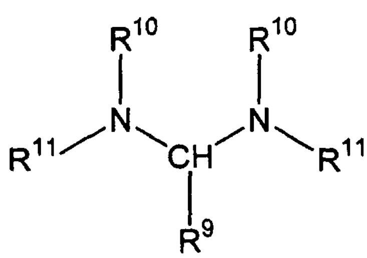

IV

umsetzt, und diese Aminalverbindungen der allgemeinen Formel IV ohne weitere Aufreinigung mit einem Säurechlorid, vorzugsweise mit Acetylchlorid, in absolutem Lösungsmittel, vorzugsweise in Diethylether zu Iminiumsalzen der all-

gemeinen Formel V

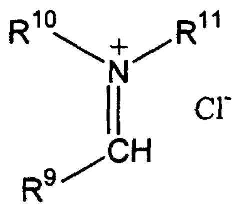

V

umsetzt, und diese Iminiumsalze der allgemeinen Formel V ohne weitere Aufreinigung in Lösung, vorzugsweise in Acetonitril mit substituierten und/oder unsubstituierten Naphtholverbindungen der allgemeinen Formel VI,

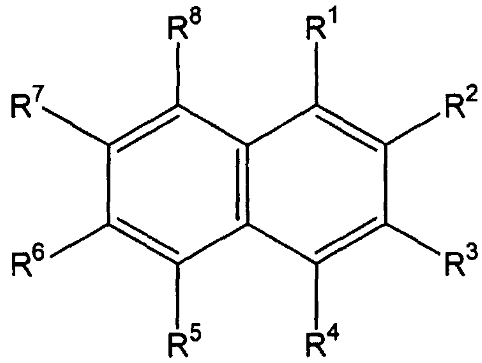

VI

worin $R^1$ = H und $R^2$ = OH oder $R^1$ = OH und $R^2$ = H bedeuten und die Reste $R^3$ bis $R^8$, $R^{13}$ bis $R^{18}$ und $R^{20}$ bis $R^{22}$ die Bedeutung gemäß der allgemeinen Formel I haben, umsetzt, und die so erhaltenen 1- und 2-Naphtholverbindungen der allgemeinen Formel I, in denen der Rest $R^{12}$ für H steht und die Reste $R^1$ bis $R^{11}$, die Reste $R^{13}$ bis $R^{18}$ und die Reste $R^{20}$ bis $R^{22}$ die Bedeutung gemäß der allgemeinen Formel I haben, durch Extraktion reinigt und nach den üblichen Methoden isoliert.

Ein weiterer Gegenstand der vorliegenden Erfindung sind auch Verfahren zur Herstellung von substituierten 1- und 2-Naphthol-Mannichbasen der allgemeinen Formel I, in denen der Rest $R^{12}$ = $COR^{22}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet und die Reste $R^1$ bis $R^{11}$, $R^{13}$ bis $R^{18}$ und $R^{20}$ bis $R^{22}$ die Bedeutung gemäß der allgemeinen Formel I haben, die dadurch gekennzeichnet sind, daß man

aromatische Aldehydverbindungen, heteroaromatische Aldehydverbindungen und/oder aliphatische Aldehydverbindungen der allgemeinen Formel II,

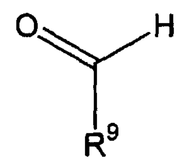

II

in denen $R^9$ die Bedeutung gemäß der allgemeinen Formel I hat, in Lösung, vorzugsweise in einem organischen Lösungsmittel, besonders bevorzugt in Toluol, in Gegenwart einer Base, vorzugsweise Kaliumcarbonat oder Borsäureanhydrid, bevorzugt bei einer Temperatur von -10 bis +110 °C mit sekundären Aminen der allgemeinen Formel III,

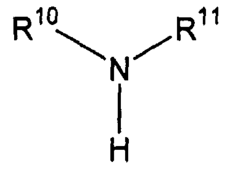

III

in denen die Reste $R^{10}$ und $R^{11}$ die Bedeutung gemäß der allgemeinen Formel I haben,
zu Aminalverbindungen der allgemeinen Formel IV

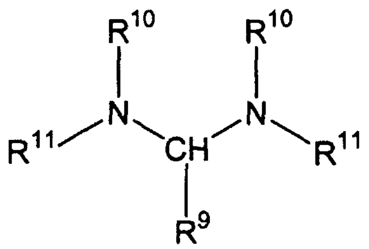

IV

umsetzt, und diese Aminalverbindungen der allgemeinen Formel IV ohne weitere Aufreinigung mit einem Säurechlorid, vorzugsweise mit Acetylchlorid, in absolutem Lösungsmittel, vorzugsweise in Diethylether zu Iminiumsalzen der allgemeinen Formel V

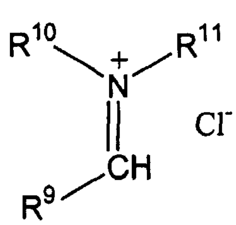

V

umsetzt, und diese Iminiumsalze der allgemeinen Formel V ohne weitere Aufreinigung in Lösung, vorzugsweise in

Acetonitril mit substituierten und/oder unsubstituierten Naphtholverbindungen der allgemeinen Formel VI,

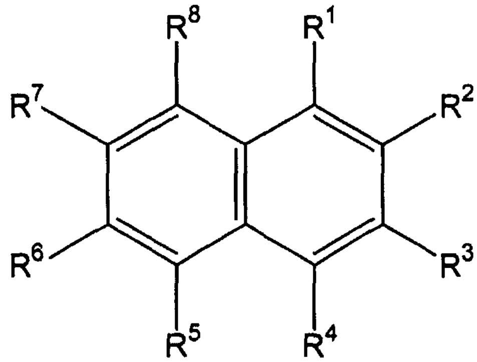

VI

worin $R^1$ = H und $R^2$ = OH oder $R^1$ = OH und $R^2$ = H bedeuten und jeweils die Reste $R^3$ bis $R^8$, $R^{13}$ bis $R^{18}$ und $R^{20}$ bis $R^{22}$ die Bedeutung gemäß der allgemeinen Formel I haben, umsetzt, und die so erhaltenen Verbindungen der allgemeinen Formel VI, worin $R^1$ = $CH(R^9)N(R^{10})(R^{11})$ und $R^2$ = OH oder $R^1$ = OH und $R^2$ = $CH(R^9)N(R^{10})(R^{11})$ bedeuten und jeweils die Reste $R^3$ bis $R^{11}$, $R^{13}$ bis $R^{18}$ und $R^{20}$ bis $R^{22}$ die Bedeutung gemäß der allgemeinen Formel I haben, in Lösung, vorzugsweise in Dimethylformamid, mit Verbindungen der allgemeinen Formel $XR^{12'}$, worin X = Cl, Br oder I, vorzugsweise Cl bedeutet und $R^{12'}$ für $COR^{22}$, einen $C_{1-10}$-Alkyl-, einen Aryl-, einen Heteroaryl- oder für einen über eine $C_{1-6}$-Alkylen-Gruppe gebundenen Aryl- oder Heteroaryl-Rest, vorzugsweise für einen $C_{1-6}$-Alkyl-Rest oder einen über eine $C_{1-2}$-Alkylen-Gruppe gebundenen Aryl-Rest steht, in Gegenwart einer Base, vorzugsweise Triethylamin oder Kalium-tert-Butylat, vorzugsweise bei einer Temperatur von 10 bis 150 °C umsetzt, und die so erhaltenen 1- und 2-Naphthol-Mannichbasen der allgemeinen Formel I, in denen der Rest $R^{12}$ für $COR^{22}$, einen $C_{1-10}$-Alkyl-, einen Aryl-, einen Heteroaryl- oder für einen über eine $C_{1-6}$-Alkylen-Gruppe gebundenen Aryl- oder Heteroaryl-Rest, vorzugsweise für einen $C_{1-6}$-Alkyl-Rest oder einen über eine $C_{1-2}$-Alkylen-Gruppe gebundenen Aryl-Rest steht und die Reste $R^1$ bis $R^{11}$, $R^{13}$ bis $R^{18}$ und $R^{20}$ bis $R^{22}$ die Bedeutung gemäß der allgemeinen Formel I haben, durch Filtration, vorzugsweise durch Filtration über ein Scavenger-Harz, besonders bevorzugt durch Filtration über polymergebundenenes Tris(2-aminoethyl)amin (Novabiochem, Bad Soden) und/oder 3-(3-Mercaptophenyl)-propanamidomethylpolystyrol (Argonaut, Muttenz, Schweiz) reinigt.

Vorzugsweise erfolgt die Synthese der erfindungsgemäßen substituierten 1- und 2-Naphthol-Mannichbasen auf einer automatischen Anlage der Firma Zymark gemäß Figur 1 und Figur 2 wie untenstehend beschrieben.

Die erfindungsgemäßen substituierten 1- und 2-Naphthol-Mannichbasen der allgemeinen Formel I lassen sich mit physiologisch verträglichen Säuren, vorzugsweise mit Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in einer dem Fachmann an sich bekannten Weise in ihre Salze überführen. Vorzugsweise wird die Salzbildung in einem Lösungsmittel, besonders bevorzugt in Diethylether, Diisopropylether, Essigsäurealkylester, vorzugsweise Essigsäureethylester, Aceton und/oder 2-Butanon durchgeführt. Ganz besonders bevorzugt erfolgt die Salzbildung mit Trimethylchlorsilan in Methylethylketon.

Die erfindungsgemäßen substituierten 1- und 2-Naphthol-Mannichbasen der allgemeinen Formel I sind toxikologisch unbedenklich und stellen daher geeignete pharmazeutische Wirkstoffe dar.

Ein weiterer Gegenstand der Erfindung sind daher auch Arzneimittel, die als Wirkstoff wenigstens eine substituierte 1- und/oder 2-Naphthol-Mannichbase der allgemeinen Formel I und gegebenenfalls weitere Wirkstoffe und/oder Hilfsstoffe enthalten.

Vorzugsweise kann das Arzneimittel auch ein Gemisch aus Enantiomeren wenigstens einer substituierten 1-Naphthol-Mannichbase und/oder 2-Naphthol-Mannichbase der allgemeinen Formel I enthalten, wobei das Gemisch die Enantiomere vorzugsweise nicht in äquimolaren Mengen aufweist. Besonders bevorzugt beträgt der relative Anteil eines der Enantiomere 5 bis 45 Mol-%, ganz besonders bevorzugt 10 bis 40 Mol-%, bezogen auf das Gesamtgemisch der Enantiomere.

Vorzugsweise werden die Arzneimittel zur Behandlung/Bekämpfung von Schmerzen und/oder inflammatorischen Reaktionen und/oder allergischen Reaktionen und/oder Drogenmißbrauch und/oder Alkoholmißbrauch und/oder Diarrhoe und/oder Gastritis und/oder Ulcera und/oder cardiovaskulären Erkrankungen und/oder Harninkontinenz und/oder Depressionen und/oder Schockzuständen und/oder Migräne und/oder Narkolepsie und/oder Übergewicht und/ oder Asthma und/oder Glaukom und/oder hyperkinetischem Syndrom eingesetzt.

Die Verwendung wenigstens einer erfindungsgemäßen substituierten 1- und/oder 2-Naphthol-Mannichbase der allgemeinen Formel I zur Herstellung eines Arzneimittels zur Behandlung/Bekämpfung von Schmerzen und/oder inflammatorischen Reaktionen und/oder allergischen Reaktionen und/oder Drogenmißbrauch und/oder Alkoholmißbrauch und/oder Diarrhoe und/oder Gastritis und/oder Ulcera und/oder cardiovaskulären Erkrankungen und/oder Harninkontinenz und/oder Depressionen und/oder Schockzuständen und/oder Migräne und/oder Narkolepsie und/oder Übergewicht und/oder Asthma und/oder Glaukom und/oder hyperkinetischem Syndrom ist ebenfalls Gegenstand der vorliegenden Erfindung.

Zur Zubereitung entsprechender pharmazeutischer Formulierungen werden neben mindestens einer substituierten 1- und/oder 2-Naphthol-Mannichbase der allgemeinen Formel I Trägermaterialien, Füllstoffe, Lösungsmittel, Verdünnungsmittel, Farbstoffe und/oder Bindemittel eingesetzt. Die Auswahl der Hilfsstoffe hängt davon ab, ob das Arzneimittel oral, intravenös, intraperitoneal, intradermal, intramuskulär, intranasal, buccal oder örtlich, zum Beispiel auf Infektionen an der Haut, der Schleimhäute und an den Augen, appliziert werden soll. Für die orale Applikation eignen sich die Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays.

Die erfindungsgemäßen substituierten 1-bzw. 2-Naphthol-Mannichbasen der allgemeinen Formel I in einem Depot in gelöster Form oder in einem Pflaster, gegebenenfalls unter Zusatz von die Hautpenetration fördernden Mittel, stellen geeignete perkutane Applikationszubereitungen dar. Aus oralen oder perkutanen Zubereitungsformen können die erfindungsgemäßen Verbindungen der allgemeinen Formel I verzögert freigesetzt werden.

Die an den Patienten zu verabreichende Wirkstoffmenge variiert in Abhängigkeit vom Gewicht des Patienten, von der Applikationsart, der Indikation und dem Schweregrad der Erkrankung.

**Pharmakologische Untersuchungen:**

1.) In vitro-Tests

Die breite Austestung der erfindungsgemäßen 1- und 2-Naphthol-Mannichbasen auf ihre Wirksamkeit erfolgte nach den üblichen High Throughput Screening Methoden, wie sie in John P. Devlin, High Throughput Screening, 1997, Marcel Dekker Inc. beschrieben sind. Sie werden hiermit als Referenz eingeführt.

Die Wirkung der erfindungsgemäßen 1- und 2-Naphthol-Mannichbasen wird insbesondere durch die Affinität zur N-Methyl-D-Aspartat-(NMDA)-Rezeptorfamilie, zu $\alpha$-adrenergen Rezeptoren und opioiden Rezeptoren bestimmt.

2.) Analgesieprüfung im Writhing-Test an der Maus

Die vertiefte Untersuchung auf analgetische Wirksamkeit wurde im Phenylchinon-induzierten Writhing an der Maus (modifiziert nach I.C. Hendershot, J. Forsaith, J. Pharmacol. Exp. Ther. 125, 237-240 (1959)) durchgeführt. Dazu wurden männliche NMRI-Mäuse im Gewicht von 25-30 g verwendet. Gruppen von 10 Tieren pro Substanzdosis erhielten 10 Minuten nach intravenöser Gabe der Prüfsubstanzen 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Als Kontrolle wurden Tiere mitgeführt, die nur physiologische Kochsalzlösung erhielten.

Die Substanzen wurden in der Standarddosis von 10 mg/kg getestet. Die Hemmung der Writhingreaktionen durch eine Substanz wurde nach folgender Formel berechnet:

$$\%\,\text{Hemmung} = 100 - \left[\frac{\text{Writhingreaktion behan. Tiere}}{\text{Writhingreaktion Kontrolle}} \times 100\right]$$

Die folgenden Beispiele dienen der Erläuterung der Erfindung.

**Beispiele:**

**Allgemeine Synthesevorschriften zur Herstellung von Aminalverbindungen der allgemeinen Formel IV:**

Allgemeine Synthesevorschrift 1:

Zu 2,7 Äquivalenten einer 40%-igen Lösung des jeweiligen sekundären Amins mit der allgemeinen Formel III wurden langsam unter Rühren bei 20°C 1,0 Äquivalente der jeweiligen aromatischen, heteroaromatischen oder aliphatischen Aldehydverbindung der allgemeinen Formel II getropft. Dann wurde die Lösung weitere 30 Minuten bei einer Temperatur von 80°C nachgerührt, anschließend auf Raumtemperatur abgekühlt und mit 0,57 Äquivalenten Kaliumcarbonat versetzt. Hierbei bildeten sich zwei Phasen, die voneinander getrennt wurden, wobei die wässrige Phase dreimal mit jeweils 100 ml Essigester extrahiert wurde. Die vereinigten, organischen Phasen wurden über Kaliumcarbonat getrocknet und vom Lösungsmittel befreit. Die so erhaltenen Aminalverbindungen der allgemeinen Formel IV wurden dann ohne weitere Aufreinigung in den nachfolgenden Reaktionen eingesetzt.

Allgemeine Synthesevorschrift 2:

Zu einer Lösung von 1,0 Äquivalenten der jeweiligen aromatischen, heteroaromatischen oder aliphatischen Aldehydverbindung der allgemeinen Formel II in 80 ml absolutem Toluol wurden 1,6 Äquivalente Borsäureanhydrid gegeben. Anschließend wurde eine Lösung von 2,4 Äquivalenten eines sekundären Amins der allgemeinen Formel III in 85 ml absolutem Toluol unter starkem Rühren zugegeben. Das Starten der Reaktion ließ sich an einem deutlichen Temperaturanstieg erkennen. Dann wurde die Reaktionslösung für weitere zwei Stunden bei einer Temperatur von 45 bis 50°C nachgerührt. Nach dem Abkühlen auf Raumtemperatur wurde das überschüssige Borsäureanhydrid abgetrennt und das Filtrat vom Lösungsmittel befreit. Die so erhaltenen Aminalverbindungen der allgemeinen Formel IV wurden ohne weitere Aufreinigung in den nachfolgenden Reaktionen eingesetzt.

**Allgemeine Synthesevorschrift zur Synthese von Iminiumsalzen der allgemeinen Formel V:**

Allgemeine Synthesevorschrift 3:

Eine Lösung von 1,0 Äquivalenten Acetylchlorid in absolutem Diethylether wurde langsam unter Rühren zu 1,0 Äquivalenten einer eisgekühlten Lösung bzw. Suspension der nach der allgemeinen Synthesevorschrift 1 oder 2 hergestellten Aminalverbindung der allgemeinen Formel IV getropft. Anschließend wurde das Reaktionsgemisch über Nacht bei ca. 20 °C nachgerührt. Hierbei entstand ein Niederschlag, der unter Stickstoff abgesaugt und anschließend im Ölpumpenvakuum getrocknet wurde. Die so erhaltenen Iminiumsalze der allgemeinen Formel V wurden ohne weitere Aufreinigung in den nachfolgenden Reaktionen eingesetzt.

**Allgemeine Synthesevorschrift zur Synthese von 1- und 2- Naphthol-Mannichbasen der allgemeinen Formel I:**

Allgemeine Synthesevorschrift 4:

Die Synthese der erfindungsgemäßen Naphthol-Mannichbasen erfolgte auf einer automatischen Anlage der Firma Zymark gemäß Figur 1 bzw. Figur 2:

Dabei umfaßt Figur 1 eine Capper-Station (Ziff. 1) zum Verschließen der Reaktionsröhrchen, einen Roboter 1 (Ziff. 2) und einen Roboter 2 (Ziff. 3), wobei der Roboter 1 die Reaktionsröhrchen bzw. die entsprechenden Racks bewegt und der Roboter 2 die Reagenzien in die Reaktionsröhrchen pipettiert, einen temperierbaren Reaktorblock (Ziff. 4), Rührblöcke (Ziff. 5) und eine Filtrationsstation (Ziff. 6), in der die Reaktionslösung abfiltriert wird.

Figur 2 umfaßt ebenfalls einen Roboter 1 (Ziff. 1) und einen Roboter 2 (Ziff. 2), die beide die Glasröhrchen mit den Syntheseprodukten auf die verschiedenen Stationen bewegen. Bei den Stationen handelt es sich im einzelnen um einen Vortexer (Ziff. 3) zum Durchmischen der Proben und zum Zudosieren von Lösungen oder Lösungsmitteln, einen Spin-Reaktor (Ziff. 4) zur Durchmischung von Proben, eine Phasendetektionsstation (Ziff. 5) zur Detektion der Phasengrenze und Phasentrennung sowie eine Station (Ziff. 6) zum Trocknen der Syntheseprodukte über Salzkartuschen.

Zur Synthese wurde ein Rundbodenröhrchen aus Glas (Durchmesser 16 mm, Länge 125 mm) mit Gewinde manuell mit einem Rührer versehen und auf der Capper-Station (Ziff. 1) gemäß Figur 1 mit einem Schraubdeckel mit Septum verschlossen. Das Röhrchen wurde von Roboter 1 (Ziff. 2) in den auf 25°C temperierten Reaktorblock (Ziff.

4) gestellt. Roboter 2 (Ziff. 3) pipettierte nacheinander folgende Reagenzien hinzu:

1.) 1 ml einer 0,1 M Lösung von 1- oder 2-Naphthol oder einer substituierten 1- oder 2-Naphtholverbindung der allgemeinen Formel VI und 14 µl Triethylamin in Acetonitril

2.) 1,2 ml einer 0,1 M Lösung eines Iminiumsalzes der allgemeinen Formel V in Acetonitril.

Die Iminiumsalze wurden zuvor wie in den nachfolgenden Beispielen angegeben hergestellt. Das Reaktionsgemisch wurde im Anschluss bei 90°C in einem der Rührblöcke (Ziff. 5) 960 min lang gerührt. Danach wurde die Reaktionslösung an der Filtrations-Station (Ziff. 6) abfiltriert. Das Röhrchen wurde dabei zweimal mit je 1 ml Dichlormethan und 200 µl Wasser gespült. Das Rack mit den Röhrchen wurde anschließend manuell auf eine automatische Aufarbeitungsanlage gemäß Figur 2 gestellt. Dort wurde das Reaktionsgemisch auf einem Vortexer (Ziff. 3) mit 2 ml Wasser und 2 ml Essigester versetzt.

Mit 1 ml wässriger 5%-iger Salzsäure-Lösung stellte man das Gemisch auf einen pH-Wert gleich 1. Im Spin-Reaktor (Ziff. 4) wurde zehn Minuten lang gründlich gemischt und durch die langsame Abnahme der Drehbewegung eine deutliche Phasengrenze ausgebildet. Diese Phasengrenze wurde auf der Phasendetektionsstation (Ziff. 5) optisch detektiert und die wässrige Phase abpipettiert. Im nächsten Schritt wurde diese mit 2 ml Essigester versetzt und mit 1 ml gesättigter wässriger Natriumhydrogencarbonat-Lösung auf einen pH-Wert = 11 eingestellt. Im Spin-Reaktor (Ziff. 4) wurde erneut zehn Minuten lang gründlich gemischt und anschließend die organische Phase abpipettiert. Im nächsten Schritt wurde die wässrige Phase nochmals mit 1,5 ml Essigester versetzt. Die Lösung wurde geschüttelt, zentrifugiert und die organische Phase abpipettiert. Die vereinigten organischen Phasen wurden über 2,4 g $MgSO_4$ (granuliert) getrocknet. Das Lösungsmittel wurde in einer Vakuumzentrifuge entfernt.

**Allgemeine Synthesevorschrift zur Synthese von alkylierten 1- und 2-Naphthol-Mannichbasen der allgemeinen Formel I:**

Allgemeine Synthesevorschrift 5:

Eine Lösung von 1,0 Äquivalenten 1- und/ oder 2-Naphthol Mannich Base der allgemeinen Formel I mit R12 = H in absolutem *N,N*-Dimethylformamid wurde 15 Minuten mit 1.0 Äquivalenten Kaliumtert.butylat behandelt, im Anschluss mit 1,0 Äquivalenten Alkylierungsreagenz (R12-Hal) versetzt und für weitere 24 Stunden bei ca. 20°C nachgerührt. Hierzu gab man anschließend 3,0 Äquivalente 3-(3-Mercaptophenyl)-propanamidomethylpolystyrol, ließ weitere drei Stunden miteinanderreagieren, filtrierte vom PS-Harz ab und konzentrierte das Filtrat im Vakuum auf. Den so erhaltenen Rückstand nahm man in einem 1:1 Dichlormethan/ Wasser-Gemisch auf, rührte 30 Minuten, trennte die Phasen, wobei die wässrige Phase dreimal mit jeweils 20 mL Dichlormethan extrahiert wurde. Die vereinigten, organischen Phasen wurden über Magnesiumsulfat getrocknet und vom Lösungsmittel befreit.

**Allgemeine Synthesevorschrift zur Synthese von acylierten 1- und 2-Naphthol-Mannichbasen der allgemeinen Formel I:**

Allgemeine Synthesevorschrift 6:

Eine Lösung von 1,0 Äquivalenten 1- und/ oder 2-Naphthol Mannich Base der allgemeinen Formel I mit R12 = H in absolutem *N,N*-Dimethylformamid wurde 15 Minuten mit 1.0 Äquivalenten Kaliumtert.butylat behandelt, im Anschluss mit 1,0 Äquivalenten Aclierungsreagenz (R12-Hal) versetzt und für weitere 24 Stunden bei ca. 20°C nachgerührt. Hierzu gab man anschließend 3,0 polymergebundenenes Tris(2-aminoethyl)amin, ließ weitere drei Stunden miteinanderreagieren, filtrierte vom PS-Harz ab und konzentrierte das Filtrat im Vakuum auf. Den so erhaltenen Rückstand nahm man in einem 1:1 Dichlormethan/ Wasser-Gemisch auf, rührte 30 Minuten, trennte die Phasen, wobei die wässrige Phase dreimal mit jeweils 20 mL Dichlormethan extrahiert wurde. Die vereinigten, organischen Phasen wurden über Magnesiumsulfat getrocknet und vom Lösungsmittel befreit.

**Synthese von 1- und 2-Naphthol-Mannichbasen der allgemeinen Formel I:**

Beispiel 1:

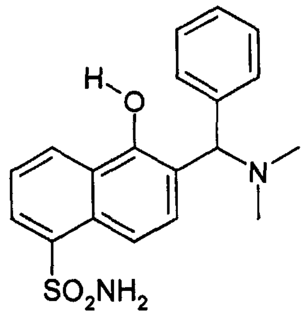

6-(Dimethylaminophenylmethyl)-5-hydroxy-naphthalin-1-sulfonsäureamid

1.Stufe

Benzyliden-dimethyl-ammoniumchlorid

Die Umsetzung von 32,0 ml (0,213 mol) Dimethylaminlösung und 8,0 ml (0,079 mol) Benzaldehyd gemäß der allgemeinen Synthesevorschrift 1 und nachfolgende Reaktion mit 4,7 ml (0,079 mol) Acetylchlorid gemäß der allgemeinen Synthesevorschrift 3 ergaben 9,5 g (entsprechend 70.7 % der theoretisch berechneten Ausbeute) an Benzyliden-dimethyl-ammoniumchlorid.

2. Stufe

6-(Dimethylaminophenylmethyl)-5-hydroxy-naphthalin-1-sulfonsäureamid

Die Herstellung erfolgte gemäß der allgemeinen Synthesevorschrift 4 aus 5-Hydroxy-1-naphthalinsulfonamid und Benzyliden-dimethyl-ammoniumchlorid.

Der Nachweis der Struktur erfolgte mittels ESI-MS: berechnete Masse 356,45 g/mol, gefundene Masse M+H = 357,3 g/mol.

Beispiel 2:

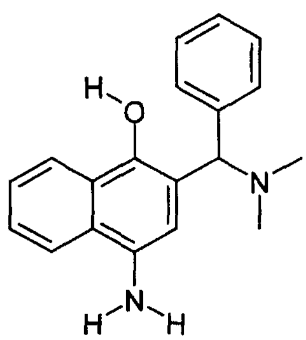

4-Amino-2-(dimethylaminophenylmethyl)-naphthalin-1-ol

Die Herstellung erfolgte gemäß der allgemeinen Synthesevorschrift 4 aus 1-Amino-4-naphthol und Benzyliden-dimethyl-ammoniumchlorid, welches gemäß Beispiel 1 hergestellt worden ist.

Der Nachweis der Struktur erfolgte mittels ESI-MS: berechnete Masse 292,38 g/mol, gefundene Masse M+H = 293,8.

Beispiel 3:

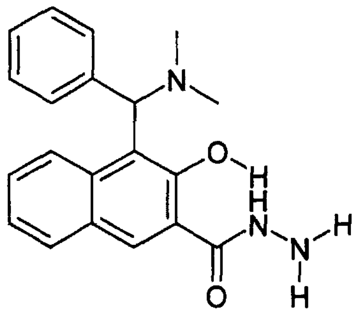

4-(Dimethylaminophenylmethyl)-3-hydroxy-naphthalin-2-carbonsäurehydrazid

Die Herstellung erfolgte gemäß der allgemeinen Synthesevorschrift 4 aus 2-Hydroxy-3-naphthoesäurehydrazid und Benzyliden-dimethyl-ammoniumchlorid, welches gemäß Beispiel 1 hergestellt worden ist.

Der Nachweis der Struktur erfolgte mittels ESI-MS: berechnete Masse 335,41 g/mol, gefundene Masse M+H = 336,3.

Beispiel 4:

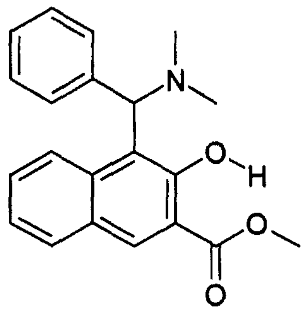

4-(Dimethylaminophenylmethyl)-3-hydroxy-naphthalin-2-carbonsäuremethylester

Die Herstellung erfolgte gemäß der allgemeinen Synthesevorschrift 4 aus Methyl-3-hydroxy-2-naphthoat und Benzyliden-dimethyl-ammoniumchlorid, welches gemäß Beispiel 1 hergestellt worden ist.

Der Nachweis der Struktur erfolgte mittels ESI-MS: berechnete Masse 335,41 g/mol, gefundene Masse M+H = 336,5.

Beispiel 5:

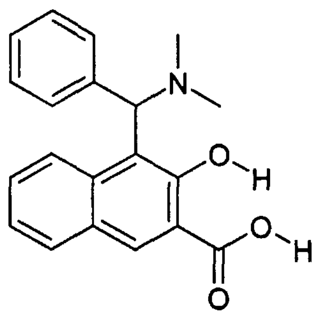

4-(Dimethylaminophenylmethyl)-3-hydroxy-naphthalin-2-carbonsäure

Die Herstellung erfolgte gemäß der allgemeinen Synthesevorschrift 4 aus 2-Hydroxy-3-naphthoesäure und Benzyliden-dimethyl-ammoniumchlorid, welches gemäß Beispiel 1 hergestellt worden ist.

Der Nachweis der Struktur erfolgte mittels ESI-MS: berechnete Masse 321,38 g/mol, gefundene Masse M+H = 322,2.

Beispiel 6:

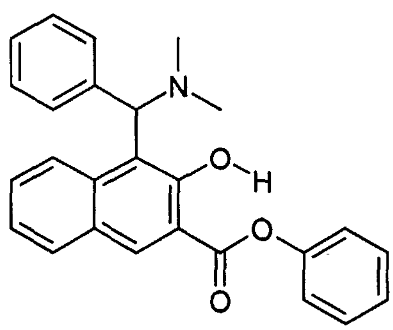

4-(Dimethylaminophenylmethyl)-3-hydroxy-naphthalin-2-carbonsäurephenylester

Die Herstellung erfolgte gemäß der allgemeinen Synthesevorschrift 4 aus 2-Hydroxy-3-naphthoesäurephenylester und Benzyliden-dimethyl-ammoniumchlorid, welches gemäß Beispiel 1 hergestellt worden ist.

Der Nachweis der Struktur erfolgte mittels ESI-MS: berechnete Masse 397,48 g/mol, gefundene Masse M+H = 398,2.

Beispiel 7:

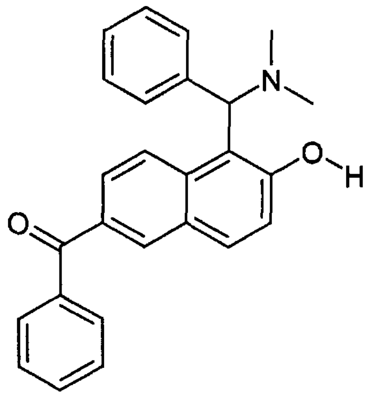

[5-(Dimethylaminophenylmethyl)-6-hydroxy-naphthalin-2-yl]-phenyl-methanon

Die Herstellung erfolgte gemäß der allgemeinen Synthesevorschrift 4 aus 6-Benzoyl-2-naphthol und Benzyliden-dimethyl-ammoniumchlorid, welches gemäß Beispiel 1 hergestellt worden ist.

Der Nachweis der Struktur erfolgte mittels ESI-MS: berechnete Masse 381,48 g/mol, gefundene Masse M+H = 382,2.

Beispiel 8:

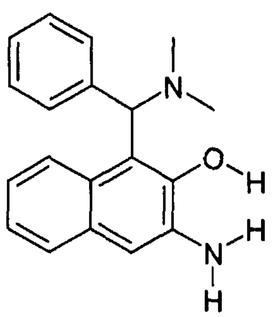

3-Amino-1-(dimethylaminophenylmethyl)-naphthalin-2-ol

Die Herstellung erfolgte gemäß der allgemeinen Synthesevorschrift 4 aus 3-Amino-2-naphthol und Benzyliden-dimethyl-ammoniumchlorid, welches gemäß Beispiel 1 hergestellt worden ist.

Der Nachweis der Struktur erfolgte mittels ESI-MS: berechnete Masse 292,38 g/mol, gefundene Masse M+H = 293,3; M+H-$NMe_2$ = 249,3.

Beispiel 9:

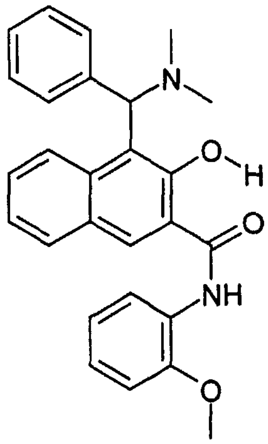

4-(Dimethylaminophenylmethyl)-3-hydroxy-naphthalin-2-carbonsäure-(2-methoxy-phenyl)-amid

Die Herstellung erfolgte gemäß der allgemeinen Synthesevorschrift 4 aus 3-Hydroxy-N-(2-methoxyphenyl)-2-naphthalincarboxamid und Benzyliden-dimethyl-ammoniumchlorid, welches gemäß Beispiel 1 hergestellt worden ist.
Der Nachweis der Struktur erfolgte mittels ESI-MS: berechnete Masse 426,52 g/mol, gefundene Masse M+H = 427,0.

Beispiel 10:

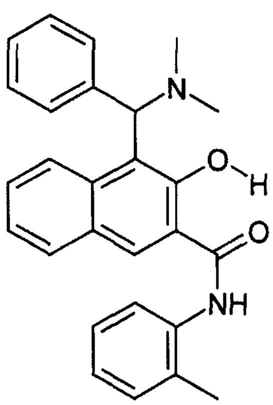

4-(Dimethylaminophenylmethyl)-3-hydroxy-naphthalin-2-carbonsäure-*o*-tolylamid

Die Herstellung erfolgte gemäß der allgemeinen Synthesevorschrift 4 aus 3-Hydroxy-N-(o-tolyl)-2-naphthalincarboxamid und Benzyliden-dimethyl-ammoniumchlorid, welches gemäß Beispiel 1 hergestellt worden ist.
Der Nachweis der Struktur erfolgte mittels ESI-MS, berechnete Masse 410,52 g/mol, gefundene Masse M+H = 412,0.

Beispiel 11:

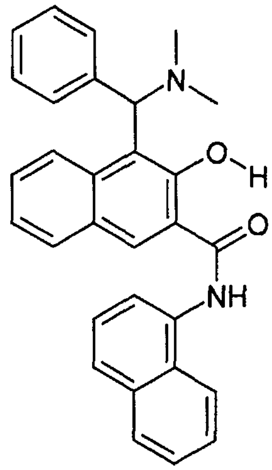

4-(Dimethylaminophenylmethyl)-3-hydroxy-naphthalin-2-carbonsäurenaphthalin-1-ylamid

Die Herstellung erfolgte gemäß der allgemeinen Synthesevorschrift 4 aus 3-Hydroxy-N-(naphthyl)-2-naphthalincarboxamid und Benzyliden-dimethyl-ammoniumchlorid, welches gemäß Beispiel 1 hergestellt worden ist.
Der Nachweis der Struktur erfolgte mittels ESI-MS: berechnete Masse 446,55 g/mol, gefundene Masse M+H = 447,8.

Beispiel 12:

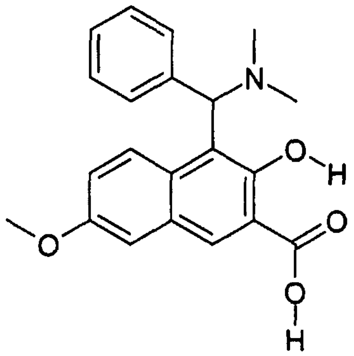

4-(Dimethylaminophenylmethyl)-3-hydroxy-7-methoxy-naphthalin-2-carbonsäure

Die Herstellung erfolgte gemäß der allgemeinen Synthesevorschrift 4 aus 3-Hydroxy-7-methoxy-2-naphthoesäure und Benzyliden-dimethyl-ammoniumchlorid, welches gemäß Beispiel 1 hergestellt worden ist.
Der Nachweis der Struktur erfolgte mittels ESI-MS, berechnete Masse 351,41 g/mol, gefundene Masse M+H = 352,3.

Beispiel 13:

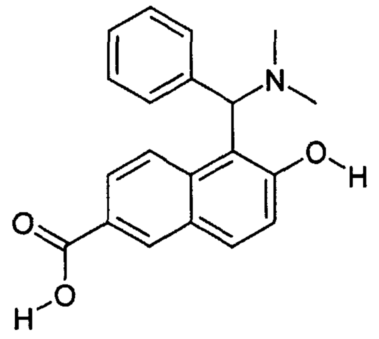

5-(Dimethylaminophenylmethyl)-6-hydroxy-naphthalin-2-carbonsäure

Die Herstellung erfolgte gemäß der allgemeinen Synthesevorschrift 4 aus 6-Hydroxy-2-naphthoesäure und Benzyliden-dimethyl-ammoniumchlorid, welches gemäß Beispiel 1 hergestellt worden ist.

Der Nachweis der Struktur erfolgte mittels ESI-MS: berechnete Masse 321,38 g/mol, gefundene Masse M+H = 322,1.

Beispiel 14:

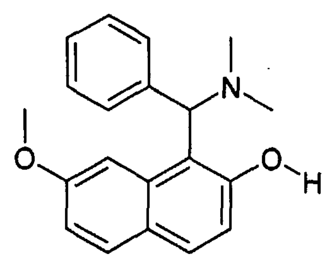

1-(Dimethylaminophenylmethyl)-7-methoxynaphthalin-2-ol

Die Herstellung erfolgte gemäß der allgemeinen Synthesevorschrift 4 aus 7-Methoxy-2-naphthol und Benzyliden-dimethyl-ammoniumchlorid, welches gemäß Beispiel 1 hergestellt worden ist.

Der Nachweis der Struktur erfolgte mittels ESI-MS: berechnete Masse 307,36 g/mol, gefundene Masse M+H = 308,4.

Beispiel 15:

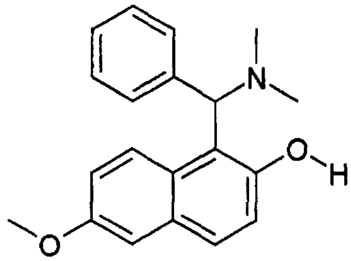

1-(Dimethylaminophenylmethyl)-6-methoxynaphthalin-2-ol

Die Herstellung erfolgte gemäß der allgemeinen Synthesevorschrift 4 aus 6-Methoxy-2-naphthol und Benzyliden-dimethyl-ammoniumchlorid, welches gemäß Beispiel 1 hergestellt worden ist.

Der Nachweis der Struktur erfolgte mittels ESI-MS: berechnete Masse 307,4 g/mol, gefundene Masse M+H = 308,3.

Beispiel 16:

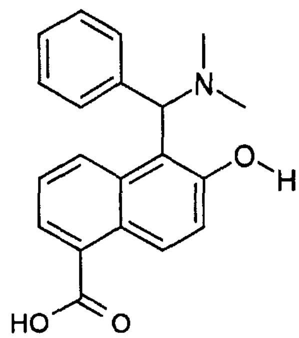

5-(Dimethylaminophenylmethyl)-6-hydroxynaphthalin-1-carbonsäure

Die Herstellung erfolgte gemäß der allgemeinen Synthesevorschrift 4 aus 6-Hydroxy-1-naphthoesäure und Benzyliden-dimethyl-ammoniumchlorid, welches gemäß Beispiel 1 hergestellt worden ist.

Der Nachweis der Struktur erfolgte mittels ESI-MS: berechnete Masse 321,38 g/mol, gefundene Masse M+H = 322,2.

Beispiel 17:

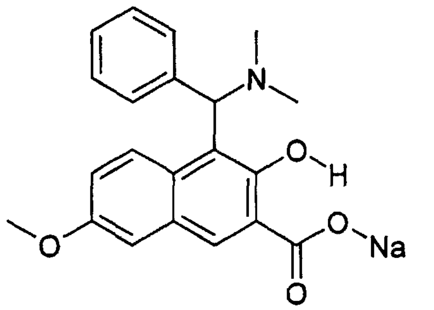

4-(Dimethylaminophenylmethyl)-3-hydroxy-7-methoxynaphthalin-2-carboxylat-Natriumsalz

Die Herstellung erfolgte gemäß der allgemeinen Synthesevorschrift 4 aus dem Natriumsalz der 3-Hydroxy-7-methoxy-2-naphthoesäure und Benzyliden-dimethyl-ammoniumchlorid, welches gemäß Beispiel 1 hergestellt worden ist.

Der Nachweis der Struktur erfolgte mittels ESI-MS: berechnete Masse 373,39 g/mol, gefundene Masse M+H-Na = 352,0.

Beispiel 18:

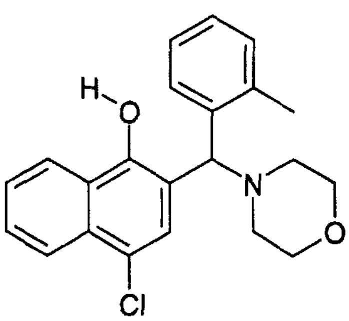

4-Chlor-2-(morpholin-4-yl-*o*-tolylmethyl)-naphthalin-1-ol

1. Stufe

4-(2-Methyl-benzyliden)-morpholin-4-ium-chlorid

Die Umsetzung von 8,5 g (0,100 mol) Morpholin und 7,0 g (0,050 mol) 2-Methoxybenzaldehyd gemäß der allgemeinen Synthesevorschrift 2 und nachfolgende Reaktion mit 3,9 g (0,050 mol) Acetylchlorid gemäß der allgemeinen Synthesevorschrift 3 ergab 7,1 g (entsprechend 58 % der theoretisch berechneten Ausbeute) an 4-(2-Methylbenzyliden)-morpholin-4-ium-chlorid.

2.Stufe

4-Chlor-2-(morpholin-4-yl-*o*-tolylmethyl)-naphthalin-1-ol

Die Herstellung erfolgte gemäß der allgemeinen Synthesevorschrift 4 aus 4-Chlor-1-naphthol und 4-(2-Methyl-benzyliden)-morpholin-4-ium-chlorid.
Der Nachweis der Struktur erfolgte mittels ESI-MS: berechnete Masse 367,88 g/mol, gefundene Masse M+H = 368,1.

Beispiel 19:

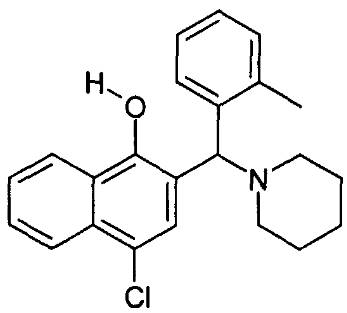

4-Chlor-2-(piperidin-1-yl-*o*-tolylmethyl)-naphthalin-1-ol

1. Stufe

1-(2-Methyl-benzyliden)-piperidinium-chlorid

Die Umsetzung von 9,5 ml (0,096 mol) Piperidin und 4,7 ml (0,040 mol) 2-Methylbenzaldehyd gemäß der allgemeinen Synthesevorschrift 2 und nachfolgende Reaktion mit 2,4 ml (0,040 mol) Acetylchlorid gemäß der allgemeinen Synthesevorschrift 3 ergaben 5,8 g (entsprechend 65 % der theoretisch berechneten Ausbeute) an 1-(2-Methyl-benzyliden)-piperidiniumchlorid.

2. Stufe

4-Chlor-2-(piperidin-1-yl-*o*-tolylmethyl)-naphthalin-1-ol

Die Herstellung erfolgte gemäß der allgemeinen Synthesevorschrift 4 aus 1-(2-Methyl-benzyliden)-piperidinium-chlorid und 4-Chlor-1-naphthol.
Der Nachweis der Struktur erfolgte mittels ESI-MS: berechnete Masse 365,91 g/mol, gefundene Masse M+H = 366,2.

Beispiel 20:

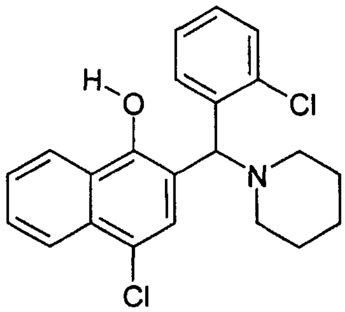

4-Chlor-2-[(2-chlorophenyl)-piperidin-1-yl-methyl]-naphthalin-1-ol

1. Stufe

1-(2-Chlor-benzyliden)-piperidinium-chlorid

Die Umsetzung von 8,5 g (0,100 mol) Piperidin und 7,0 g (0,050 mol) 2-Chlorbenzaldehyd gemäß der allgemeinen Synthesevorschrift 2 und nachfolgende Reaktion mit 3,9 g (0,050 mol) Acetylchlorid gemäß der allgemeinen Synthesevorschrift 3 ergaben 7,1 g (entsprechend 58 % der theoretisch berechneten Ausbeute) an 1-(2-Methyl-benzyliden)-piperidiniumchlorid.

2. Stufe

4-Chlor-2-[(2-chlorophenyl)-piperidin-1-yl-methyl]-naphthalin-1-ol

Die Herstellung erfolgte gemäß der allgemeinen Synthesevorschrift 4 aus 1-(2-Chlor-benzyliden)-piperidinium-chlorid und 4-Chlor-1-naphthol.

Der Nachweis der Struktur erfolgte mittels ESI-MS: berechnete Masse 386,32 g/mol, gefundene Masse M+H = 386,1.

Beispiel 21:

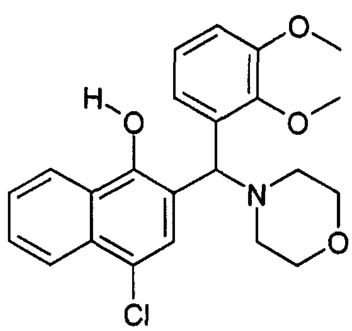

4-Chlor-2-[(2,3-dimethoxyphenyl)-morpholin-4-yl-methyl]-naphthalin-1-ol

1. Stufe

4-(2,3-Dimethoxy-benzyliden)-morpholin-4-ium-chlorid

Die Umsetzung von 7,3 ml (0,084 mol) Morpholin und 5,8 g (0,035 mol) 2,3-Dimethoxybenzaldehyd gemäß der allgemeinen Synthesevorschrift 2 und nachfolgende Reaktion mit 2,1 ml (0,035 mol) Acetylchlorid gemäß der allgemeinen Synthesevorschrift 3 ergaben 5,6 g (entsprechend 59 % der theoretisch berechneten Ausbeute) an 4-(2,3-Dimethoxy-benzyliden)-morpholin-4-ium-chlorid.

2. Stufe

4-Chlor-2-[(2,3-dimethoxyphenyl)-morpholin-4-yl-methyl]-naphthalin-1-ol

Die Herstellung erfolgte gemäß der allgemeinen Synthesevorschrift 4 aus 4-(2,3-Dimethoxy-benzyliden)-morpholin-4-ium-chlorid und 4-Chlor-1-naphthol.

Der Nachweis der Struktur erfolgte mittels ESI-MS: berechnete Masse 413,91 g/mol, gefundene Masse M+H = 414,0.

Beispiel 22:

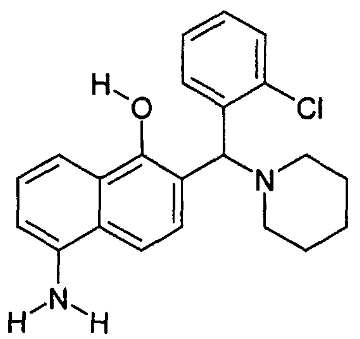

5-Amino-2-[(2-chlorophenyl)-piperidin-1-yl-methyl]-naphthalin-1-ol

Die Herstellung erfolgte gemäß der allgemeinen Synthesevorschrift 4 aus 5-Amino-1-naphthol und 1-(2-Chlor-benzyliden)-piperidinium-chlorid, welches gemäß Beispiel 20 hergestellt worden ist.

Der Nachweis der Struktur erfolgte mittels ESI-MS: berechnete Masse 366,89 g/mol, gefundene Masse M+H = 367,4.

Beispiel 23:

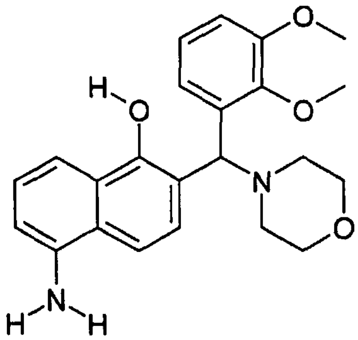

5-Amino-2-[(2,3-dimethoxyphenyl)-morpholin-4-yl-methyl]-naphthalin-1-ol

Die Herstellung erfolgte gemäß der allgemeinen Synthesevorschrift 4 aus 5-Amino-1-naphthol und 4-(2,3-Dimethoxy-benzyliden)-morpholin-4-ium-chlorid, welches gemäß Beispiel 21 hergestellt worden ist.

Der Nachweis der Struktur erfolgte mittels ESI-MS: berechnete Masse 394,47 g/mol, gefundene Masse M+H = 395,1.

Beispiel 24:

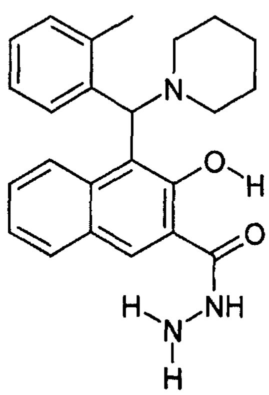

3-Hydroxy-4-(piperidin-1-yl-*o*-tolylmethyl)-naphthalin-2-carbonsäurehydrazid

1. Stufe

1-(2-Methyl-benzyliden)-piperidinium-chlorid

Die Umsetzung von 9,5 ml (0,096 mol) Piperidin und 4,7 ml (0,040 mol) 2-Methylbenzaldehyd gemäß der allgemeinen Synthesevorschrift 2 und nachfolgende Reaktion mit 2,4 ml (0,040 mol) Acetylchlorid gemäß der allgemeinen Synthesevorschrift 3 ergaben 5,8 g (entsprechend 65 % der theoretisch berechneten Ausbeute) an 1-(2-Methylbenzyliden)-piperidiniumchlorid.

2. Stufe

3-Hydroxy-4-(piperidin-1-yl-*o*-tolylmethyl)-naphthalin-2-carbonsäurehydrazid

Die Herstellung erfolgte gemäß der allgemeinen Synthesevorschrift 4 aus 1-(2-Methyl-benzyliden)-piperidinium-chlorid und 2-Hydroxy-3-naphthoesäurehydrazid.
Der Nachweis der Struktur erfolgte mittels ESI-MS: berechnete Masse 389,5 g/mol, gefundene Masse M-H = 388,5.

Beispiel 25:

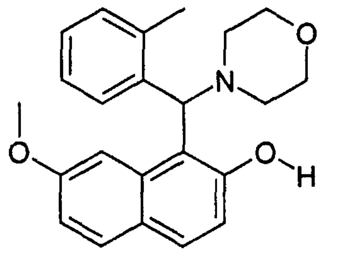

7-Methoxy-1-(morpholin-4-yl-*o*-tolylmethyl)-naphthalin-2-ol

Die Herstellung erfolgte gemäß der allgemeinen Synthesevorschrift 4 aus 7-Methoxy-2-naphthol und 4-(2-Methyl-benzyliden)-morpholin-4-ium-chlorid, welches gemäß Beispiel 18 hergestellt worden ist.
Der Nachweis der Struktur erfolgte mittels ESI-MS, berechnete Masse 389,41 g/mol, gefundene Masse M+H = 389,5.

Beispiel 26:

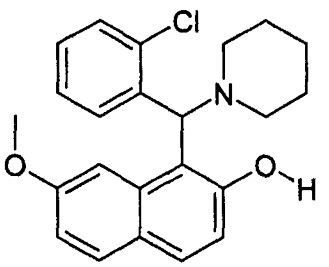

1-[(2-Chlorophenyl)-piperidin-1-yl-methyl]-7-methoxy-naphthalin-2-ol

Die Herstellung erfolgte gemäß der allgemeinen Synthesevorschrift 4 aus 7-Methoxy-2-naphthol und 1-(2-Chlor-benzyliden)-piperidinium-chlorid, welches gemäß Beispiel 20 hergestellt worden ist.
Der Nachweis der Struktur erfolgte mittels ESI-MS: berechnete Masse 381,91 g/mol, gefundene Masse M+H = 382,2.

Beispiel 27:

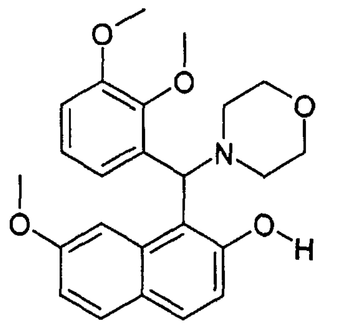

1-[(2,3-Dimethoxyphenyl)-morpholin-4-yl-methyl]-7-methoxy-naphthalin-2-ol

Die Herstellung erfolgte gemäß der allgemeinen Synthesevorschrift 4 aus 7-Methoxy-2-naphthol und 4-(2,3-Dimethoxy-benzyliden)-morpholin-4-ium-chlorid, welches gemäß Beispiel 21 hergestellt worden ist.
Der Nachweis der Struktur erfolgte mittels ESI-MS: berechnete Masse 409,49 g/mol, gefundene Masse M+H = 409,9.

Beispiel 28:

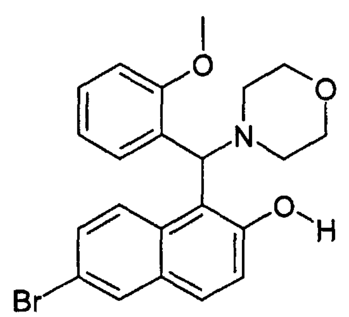

6-Brom-1-[(2-methoxyphenyl)-morpholin-4-yl-methyl]-naphthalin-2-ol

1. Stufe

4-(2-Methoxy-benzyliden)-morpholin-4-ium-chlorid

Die Umsetzung von 18,8 ml (0,216 mol) Morpholin und 12,4 g (0,09 mol) 2-Methoxvbenzaldehvd gemäß der allgemeinen Synthesevorschrift 2 und nachfolgende Reaktion mit 5,3 ml (0,110 mol) Acetylchlorid gemäß der allgemeinen Synthesevorschrift 3 ergaben 7,61 g (entsprechend 38 % der theoretisch berechneten Ausbeute) an 4-(2-Methoxy-benzyliden)-morpholin-4-ium-chlorid.

2. Stufe

6-Brom-1-[(2-methoxyphenyl)-morpholin-4-yl-methyl]-naphthalin-2-ol

Die Herstellung erfolgte gemäß der allgemeinen Synthesevorschrift 4 aus 4-(2-Methoxy-benzyliden)-morpholin-4-ium-chlorid und 6-Brom-2-naphthol.

Der Nachweis der Struktur erfolgte mittels ESI-MS: berechnete Masse 428,33 g/mol, gefundene Masse M+H = 428,1/430,0.

Beispiel 29:

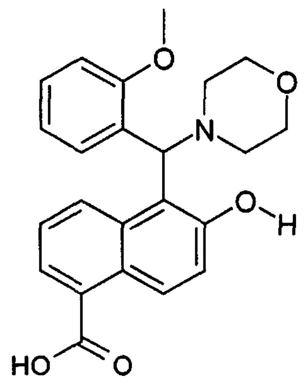

6-Hydroxy-5-[(2-methoxyphenyl)-morpholin-4-yl-methyl]-naphthalin-1-carbonsäure

Die Herstellung erfolgte gemäß der allgemeinen Synthesevorschrift 4 aus 6-Hydroxy-1-naphthoesäure und 4-(2-Methoxy-benzyliden)-morpholin-4-iumchlorid, welches gemäß Beispiel 28 hergestellt worden ist.

Der Nachweis der Struktur erfolgte mittels ESI-MS: berechnete Masse 393,44 g/mol, gefundene Masse M+H = 394,1.

Beispiel 30:

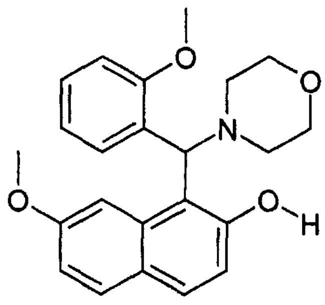

7-Methoxy-1-[(2-methoxyphenyl)-morpholin-4-yl-methyl]-naphthalin-2-ol

Die Herstellung erfolgte gemäß der allgemeinen Synthesevorschrift 4 aus 7-Methoxy-2-naphthol und 4-(2-Methoxy-benzyliden)-morpholin-4-ium-chlorid, welches gemäß Beispiel 28 hergestellt worden ist.

Der Nachweis der Struktur erfolgte mittels ESI-MS: berechnete Masse 379,46 g/mol, gefundene Masse M+H = 380,2.

Beispiel 31:

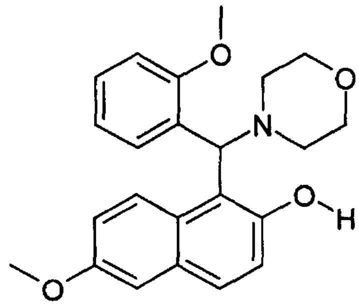

6-Methoxy-1-[(2-methoxyphenyl)-morpholin-4-yl-methyl]-naphthalin-2-ol

Die Herstellung erfolgte gemäß der allgemeinen Synthesevorschrift 4 aus 6-Methoxy-2-naphthol und 4-(2-Methoxy-benzyliden)-morpholin-4-ium-chlorid, welches gemäß Beispiel 28 hergestellt worden ist.

Der Nachweis der Struktur erfolgte mittels ESI-MS: berechnete Masse 379,46 g/mol, gefundene Masse M+H = 380,1.

Beispiel 32:

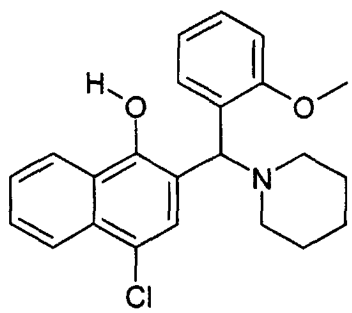

4-Chlor-2-[(2-methoxyphenyl)-piperidin-1-yl-methyl]-naphthalin-1-ol

1. Stufe

1-(2-Methoxy-benzyliden)-piperidinium-chlorid

Die Umsetzung von 18,4 g (0,216 mol) Piperidin und 25,9 g (0,090 mol) 2-Methoxybenzaldehyd gemäß der allgemeinen Synthesevorschrift 2 und nachfolgende Reaktion mit 5,3 ml (0,11 mol) Acetylchlorid gemäß der allgemeinen Synthesevorschrift 3 ergaben 13,4 g (entsprechend 62 % der theoretisch berechneten Ausbeute) an 1-(2-Methoxy-benzyliden)-piperidiniumchlorid.

2. Stufe

4-Chlor-2-[(2-methoxyphenyl)-piperidin-1-yl-methyl]-naphthalin-1-ol

Die Herstellung erfolgte gemäß der allgemeinen Synthesevorschrift 4 aus 1-(2-Methoxy-benzyliden)-piperidinium-chlorid und 4-Chlor-1-naphthol.

Der Nachweis der Struktur erfolgte mittels ESI-MS: berechnete Masse 381,91 g/mol, gefundene Masse M+H-

Piperidin = 297,2.

Beispiel 33:

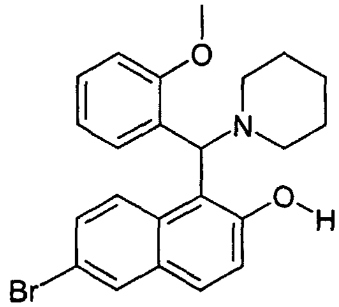

6-Brom-1-[(2-methoxyphenyl)-piperidin-1-yl-methyl]-naphthalin-2-ol

Die Herstellung erfolgte gemäß der allgemeinen Synthesevorschrift 4 aus 6-Brom-2-naphthol und 1-(2-Methoxy-benzyliden)-piperidinium-chlorid, welches gemäß Beispiel 32 hergestellt worden ist.

Der Nachweis der Struktur erfolgte mittels ESI-MS: berechnete Masse 426,36 g/mol, gefundene Masse M+H = 426,1/428,2.

Beispiel 34:

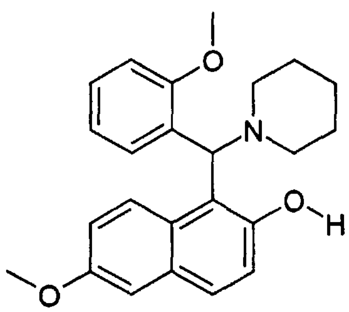

6-Methoxy-1-[(2-methoxyphenyl)-piperidin-1-yl-methyl]-naphthalin-2-ol

Die Herstellung erfolgte gemäß der allgemeinen Synthesevorschrift 4 aus 6-Methoxy-2-naphthol und 1-(2-Methoxy-benzyliden)-piperidinium-chlorid, welches gemäß Beispiel 32 hergestellt worden.

Der Nachweis der Struktur erfolgte mittels ESI-MS: berechnete Masse 377,49 g/mol, gefundene Masse M+H = 378,2.

Beispiel 35:

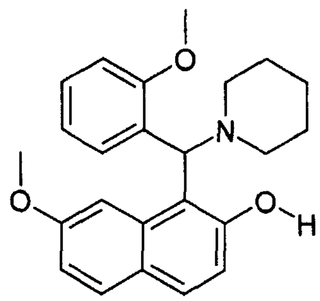

7-Methoxy-1-[(2-methoxyphenyl)-piperidin-1-yl-methyl]-naphthalin-2-ol

Die Herstellung erfolgte gemäß der allgemeinen Synthesevorschrift 4 aus 7-Methoxy-2-naphthol und 1-(2-Methoxy-benzyliden)-piperidinium-chlorid, welches gemäß Beispiel 32 hergestellt worden ist.

Der Nachweis der Struktur erfolgte mittels ESI-MS: berechnete Masse 377,49 g/mol, gefundene Masse M+H = 378,2.

Beispiel 36:

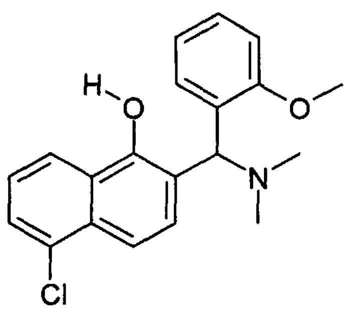

5-Chlor-2-[dimethylamino-(2-methoxyphenyl)-methyl]-naphthalin-1-ol

1. Stufe

(2-methoxy-benzyliden)-dimethyl-ammonium-chlorid

Die Umsetzung von 17,0 ml (0,135 mol) Dimethylaminlösung und 6,8 ml (0,050 mol) 2-Methoxybenzaldehyd gemäß der allgemeinen Synthesevorschrift 1 und nachfolgende Reaktion mit 3,0 ml (0,050 mol) Acetylchlorid gemäß der allgemeinen Synthesevorschrift 3 ergaben 4,8 g (entsprechend 48 % der theoretisch berechneten Ausbeute) an (2-methoxy-benzyliden)-dimethylammonium-chlorid.

2. Stufe

5-Chlor-2-[dimethylamino-(2-methoxyphenyl)-methyl]-naphthalin-1-ol

Die Herstellung erfolgte gemäß der allgemeinen Synthesevorschrift 4 aus (2-methoxy-benzyliden)-dimethyl-ammonium-chlorid und 5-Chlor-1-naphthol.

Der Nachweis der Struktur erfolgte mittels ESI-MS: berechnete Masse 341,84 g/mol, gefundene Masse M+H-

$NMe_2$ = 297,2.

Beispiel 37:

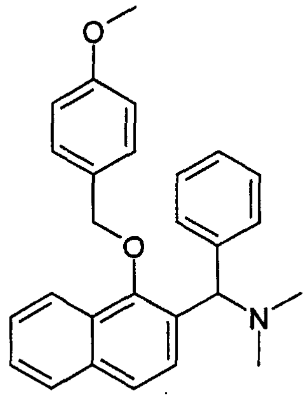

{[1-(4-Methoxy-benzyloxy)-naphthalin-2-yl]-phenylmethyl}-dimethylamin

Die Herstellung erfolgte gemäß der allgemeinen Synthesevorschriften 4 und 5 aus 1-Naphthol und Benzyliden-dimethyl-ammoniumchlorid und 4-Methoxybenzylchlorid.

Der Nachweis der Struktur erfolgte mittels $^{13}C$-NMR: $\delta$ = 159,59; 151,92; 143,30; 134,03; 132,03; 129,22 (Cq); 129,76; 128,38; 128,07; 127,99; 126,87; 125,84, 125,74; 124,61, 122,40, 114,10 ($C_t$); 75,85 ($C_s$); 69,46; 55,38; 44,82 ($C_p$).

Beispiel 38:

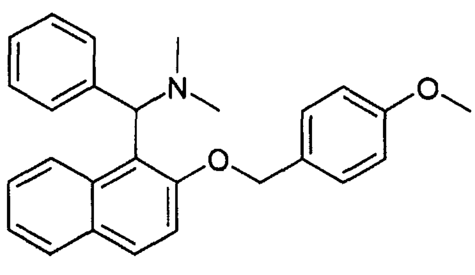

{[2-(4-Methoxybenzyloxy)-naphthalin-1-yl]-phenylmethyl}-dimethylamin

Die Herstellung erfolgte gemäß der allgemeinen Synthesevorschrift 4 und 5 aus 2-Naphthol, Benzyliden-dimethyl-ammoniumchlorid und 4-Methoxybenzylchlorid.
Der Nachweis der Struktur erfolgte mittels ESI-MS: berechnete Masse 397,52 g/mol, gefundene Masse M+H = 398,0.

Beispiel 39:

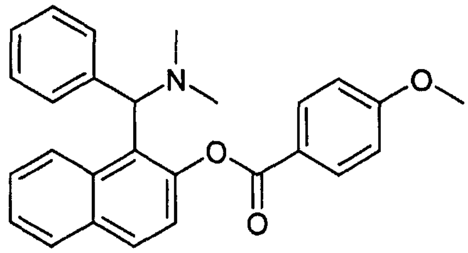

4-Methoxybenzoesäure-1-(dimethylaminophenylmethyl)-naphthalin-2-yl ester

Die Herstellung erfolgte gemäß der allgemeinen Synthesevorschriften 4 und 6 aus 2-Naphthol, Benzyliden-dimethyl-ammoniumchlorid und 4-Methoxybenzoylchlorid.
Der Nachweis der Struktur erfolgte mittels ESI-MS: berechnete Masse 411,51 g/mol, gefundene Masse M+H = 412,0.

Beispiel 40:

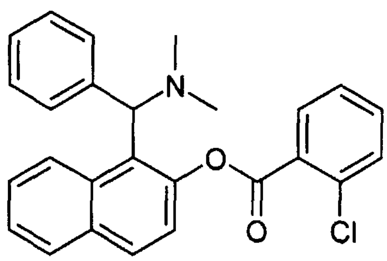

2-Chlorobenzoesäure-1-(dimethylaminophenylmethyl)-naphthalin-2-yl-ester

Die Herstellung erfolgte gemäß der allgemeinen Synthesevorschrift 4 und 6 aus 2-Naphthol, Benzyliden-dimethyl-ammoniumchlorid und 2-Chlorbenzoylchlorid.
Der Nachweis der Struktur erfolgte mittels ESI-MS: berechnete Masse 415,92 g/mol, gefundene Masse M+H = 416,0.

Beispiel 41:

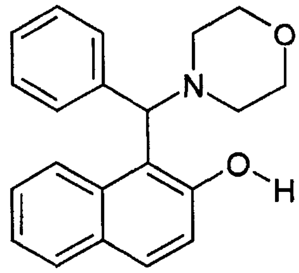

1-(Morpholin-4-yl-phenylmethyl)-naphthalin-2-ol

1. Stufe

4-Benzyliden-morpholin-4-ium-chlorid

Die Umsetzung von 17,9 ml (0,200 mol) Morpholin und 10,1 ml (0,100 mol) Benzaldehyd gemäß der allgemeinen Synthesevorschrift 2 und nachfolgende Reaktion mit 6,0 ml (0,100 mol) Acetylchlorid gemäß der allgemeinen Synthesevorschrift 3 ergaben 10,1 g (entsprechend 48 % der theoretisch berechneten Ausbeute) an 4-Benzyliden-morpholin-4-ium-chlorid.

2. Stufe

1-(Morpholin-4-yl-phenyl-methyl)-naphthalin-2-ol

Die Herstellung erfolgte gemäß der allgemeinen Synthesevorschrift 4 aus 4-Benzyliden-morpholin-4-ium-chlorid und 2-Naphthol.

Der Nachweis der Struktur erfolgte mittels ESI-MS: berechnete Masse 319,41 g/mol, gefundene Masse M+H = 320,1 g/mol.

Beispiel 42:

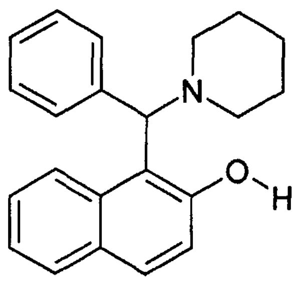

1-(Phenylpiperidin-1-yl-methyl)-naphthalin-2-ol

1. Stufe

1-Benzyliden-piperidinium-chlorid

Die Umsetzung von 19,8 ml (0,200 mol) Piperidin und 10,1 ml (0,100 mol) Benzaldehyd gemäß der allgemeinen Synthesevorschrift 2 und nachfolgende Reaktion mit 6,0 ml (0,100 mol) Acetylchlorid gemäß der allgemeinen Synthesevorschrift 3 ergaben 11,7 g (entsprechend 56 % der theoretisch berechneten Ausbeute) an 1-Benzyliden-piperidinium-chlorid.

2. Stufe

1-(Phenylpiperidin-1-yl-methyl)-naphthalin-2-ol

Die Herstellung erfolgte gemäß der allgemeinen Synthesevorschrift 4 aus 1-Benzyliden-piperidinium-chlorid und 2-Naphthol.

Der Nachweis der Struktur erfolgte mittels ESI-MS: berechnete Masse 317,43 g/mol, gefundene Masse M+H = 318,3 g/mol.

Beispiel 43:

2-[(4-Fluor-phenyl)-pyrrolidin-1-yl-methyl]-naphthalin-1-ol

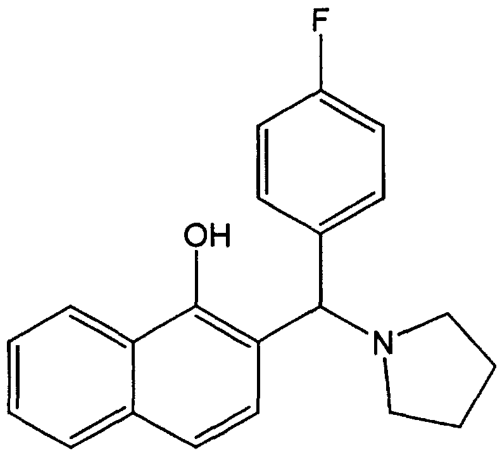

Die Herstellung erfolgte gemäß der allgemeinen Synthesevorschrift 4 aus 1-Naphthol und (4-Fluor-benzyliden)-pyrrolidiniumchlorid, welches gemäß Beispiel 41 aus 4-Fluorbenzaldehyd und Pyrrolidin hergestellt worden ist.
Der Nachweis der Struktur erfolgte mittels ESI-MS: berechnete Masse 321,4 g/mol, gefundene Masse M+H = 322,1 g/mol, M-Pyrrolidin 251,3 g/mol.

**Pharmakologische Untersuchungen:**

1.) In vitro-Tests

Die Austestung der erfindungsgemäßen 1- und 2-Naphthol-Mannichbasen auf ihre Wirksamkeit erfolgte wie obenstehend angegeben.

2.) Analgesieprüfung im Writhing-Test an der Maus

Die vertiefte Untersuchung auf analgetische Wirksamkeit wurde im Phenylchinon-induzierten Writhing an der Maus durchgeführt, wie obenstehend beschrieben.
Die untersuchten erfindungsgemäßen Verbindungen zeigten eine analgetische Wirkung.
Die Ergebnisse ausgewählter Writhing-Untersuchungen sind in der nachfolgenden Tabelle 1 zusammengefaßt.

Tabelle1:

| Analgesieprüfung im Writhing-Test an der Maus | |
|---|---|
| Beispiel Nr. | Hemmung der Writhingreaktion in % |
| 37 | 40 |
| 38 | 81 |
| 39 | 21 |
| 40 | 48 |
| 41 | 30 |
| 42 | 92 |

## Patentansprüche

**1.** Substituierte 1- und 2- Naphthol-Mannichbasen der allgemeinen Formel I,

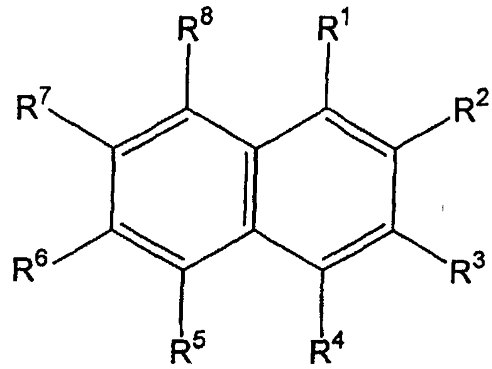

I

worin
$R^1 = CH(R^9)N(R^{10})(R^{11})$ und $R^2 = OR^{12}$ bedeuten
oder
$R^1 = OR^{12}$ und $R^2 = CH(R^9)N(R^{10})(R^{11})$ bedeuten,
und jeweils die Reste
$R^3$ bis $R^8$, gleich oder verschieden, = H, F, Cl, Br, $CF_3$, CN, $NO_2$, $SO_2NH_2$, $SO_2NHR^{13}$, $NHR^{13}$, $SR^{15}$, $OR^{16}$, CO $(OR^{20})$, $CH_2CO(OR^{21})$, $CO(R^{22})$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeuten,
$R^9$ einen Aryl-Rest, einen Heteroaryl-Rest oder einen Alkyl-Rest ohne acides Proton in $\alpha$-Stellung bedeutet,
$R^{10}$, $R^{11}$, gleich oder verschieden, einen verzweigten, unverzweigten, gesättigten, ungesättigten, unsubstituierten oder wenigstens einfach substituierten $C_{1-6}$-Alkyl-Rest oder einen unsubstituierten oder wenigstens einfach substituierten Phenyl-, Benzyl- oder Phenethyl-Rest bedeuten,
oder $R^{10}$ und $R^{11}$ zusammen $(CH_2)_2O(CH_2)_2$ oder $(CH_2)_n$ mit n = eine ganze Zahl von 3 bis 6 bedeuten,
$R^{12}$ = H, $COR^{22}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
$R^{13}$ = H, $COR^{14}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
$R^{14}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest bedeutet,
$R^{15}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest bedeutet,
$R^{16}$ = H, $CO(R^{17})$, einen $C_{1-10}$-Alkyl-, einen Aryl-, einen Heteroaryl- oder einen über eine $C_{1-6}$-Alkylengruppe gebundenen Aryl- oder Heteroaryl-Rest bedeutet,
$R^{17}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest bedeutet,
$R^{18}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest bedeutet,
$R^{20}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest bedeutet,
$R^{21}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest bedeutet,
$R^{22}$ = H, $NHNH_2$, $NHR^{18}$, einen $C_{1-10}$-Alkyl-, einen Aryl-, einen Heteroaryl- oder einen über eine $C_{1-6}$-Alkylengruppe gebundenen Aryl- oder Heteroaryl-Rest bedeutet
und/oder deren Racemate, Enantiomere, Diastereomere und/oder entsprechende Basen und/oder entsprechende Salze von physiologisch verträglichen Säuren,
wobei
die Racemate der Verbindungen, in denen die Reste $R^1 = CH(R^9)N(R^{10})(R^{11})$ und $R^2 = OR^{12}$ sind und jeweils

die Reste $R^3$ bis $R^8$ und $R^{12}$ = H, der Rest $R^9$ = Phenyl, 2-Chlorphenyl, 4-Methoxyphenyl, 3-Fluorphenyl, 3-Chlorphenyl, 3-Bromphenyl, 4-Bromphenyl, 2-Fluorphenyl, 2-Bromphenyl, Benzo-1,3-dioxol, 4-$CH_3OCO$-phenyl oder 2-Methoxyphenyl und die Reste $R^{10}$ und $R^{11}$ zusammen = $(CH_2)_5$ bedeuten
oder
die Reste $R^3$ bis $R^8$ und $R^{12}$ = H, der Rest $R^9$ = Phenyl, 4-Methoxyphenyl, 4-Dimethylaminophenyl-, 4-Hydroxy-2,3-Di-tert-Butylphenyl, 2,3-Dihydrobenzodioxan, 4-Nitrophenyl oder Benzo-1,3-dioxol und die Reste $R^{10}$ und $R^{11}$ zusammen = $(CH_2)_2O(CH_2)_2$ bedeuten
oder
die Reste $R^3$ bis $R^8$ und $R^{12}$ = H, der Rest $R^9$ = 4-Methoxyphenyl und die Reste $R^{10}$ und $R^{11}$ zusammen = $(CH_2)_4$ bedeuten
oder
der Rest $R^3$ = $CO(OR^{20})$, die Reste $R^4$ bis $R^8$ und $R^{12}$ = H, der Rest $R^9$ = Phenyl, 4-Methoxyphenyl, 4-Methylphenyl, 4-Nitrophenyl, p-Benzaldehyd, die Reste $R^{10}$ und $R^{11}$ zusammen = $(CH_2)_5$ und der Rest $R^{20}$ = $CH_3$ bedeuten
oder
die Reste $R^3$ bis $R^8$ und $R^{12}$ = H, der Rest $R^9$ = Phenyl und beide Reste $R^{10}$ und $R^{11}$ jeweils = $CH_3$, $C_2H_5$ oder n-$C_3H_7$ bedeuten
oder
die Reste $R^3$ bis $R^8$ jeweils gleich H, der Rest $R^{12}$ = $CH_3$, der Rest $R^9$ = Phenyl und beide Reste $R^{10}$ und $R^{11}$ jeweils = $CH_3$ bedeuten
oder
die Reste $R^3$ bis $R^8$ und $R^{12}$ = H, der Rest $R^9$ = 4-Methoxyphenyl oder Benzo-1,3-dioxol und die Reste $R^{10}$ und $R^{11}$ jeweils = $CH_3$ bedeuten
oder
die Reste $R^3$ bis $R^5$, $R^7$, $R^8$, $R^{12}$ = H, der Rest $R^6$ = Br, der Rest $R^9$ = Phenyl und die Reste $R^{10}$ und $R^{11}$ zusammen = $(CH_2)_5$ bedeuten
oder
die Reste $R^3$ bis $R^5$, $R^7$, $R^8$, $R^{12}$ = H, der Rest $R^6$ = Br, der Rest $R^9$ = 4-Hydroxy-3,5-Di-tert-Butylphenyl und die Reste $R^{10}$ und $R^{11}$ zusammen = $(CH_2)_2O(CH_2)_2$ bedeuten
oder
die Reste $R^3$ bis $R^8$ und $R^{12}$ = H, der Rest $R^9$ = Phenyl und die Reste $R^{10}$ und $R^{11}$ jeweils = $CH_3$ bedeuten als Hydrochlorid
oder
die Reste $R^3$ bis $R^8$ und $R^{12}$ = H, der Rest $R^9$ = Phenyl oder 4-Methoxyphenyl und die Reste $R^{10}$ und $R^{11}$ zusammen = $(CH_2)_5$ bedeuten als Hydrochlorid
oder
der Rest $R^3$ = $CO(OR^{20})$, die Reste $R^4$ bis $R^8$ und $R^{12}$ = H, der Rest $R^9$ = Phenyl, die Reste $R^{10}$ und $R^{11}$ zusammen = $(CH_2)_5$ und der Rest $R^{20}$ = $CH_3$ bedeuten als Hydrochlorid
oder
die Reste $R^3$ bis $R^8$ und $R^{12}$ = H, der Rest $R^9$ = Thiophen und die Reste $R^{10}$ und $R^{11}$ zusammen = $(CH_2)_2O(CH_2)_2$ bedeuten
oder
die Reste $R^3$ bis $R^8$ = H, der Rest $R^{12}$ = $CH_3$, Der Rest $R^9$ = Thiophen, 4-Methoxyphenyl oder 3,4-Dimethoxyphenyl und die Reste $R^{10}$ und $R^{11}$ zusammen = $(CH_2)_2O(CH_2)_2$ bedeuten
und die Enantiomere der Verbindung der allgemeinen Formel I, in der $R^1$ = $CH(R^9)N(R^{10})(R^{11})$ und $R^2$ = $OR^{12}$ ist und die Reste $R^3$ bis $R^8$, $R^{12}$ = H, $R^9$ = Phenyl, $R^{10}$ und $R^{11}$ zusammen = $(CH_2)_5$ bedeuten,
(+)-1-(α-N,N-Dimethylaminobenzyl)-2-naphtol sowie das entsprechende Tartrat,
(-)-1-(α-N,N-Dimethylaminobenzyl)-2-naphtol sowie das entsprechende Tartrat, und
(-)-[(2-Methoxynaphth-1-yl)benzyl]-dimethylamin
sowie
die Racemate der Verbindungen in denen die Reste $R^1$ = $OR^{12}$ und $R^2$ =$CH(R^9)N(R^{10})(R^{11})$ sind und jeweils die Reste
$R^3$ bis $R^8$ und $R^{12}$ = H, der Rest $R^9$ = Phenyl, 2-Bromphenyl, 3-Bromphenyl oder 4-Bromphenyl und die Reste $R^{10}$ und $R^{11}$ zusammen = $(CH_2)_5$ bedeuten
oder
$R^3$ bis $R^8$ und $R^{12}$ = H, der Rest $R^9$ = Phenyl oder 2-Nitrophenyl und die Reste $R^{10}$ und $R^{11}$ zusammen = $(CH_2)_2O(CH_2)_2$ bedeuten
oder
$R^3$, $R^4$, $R^6$, $R^8$ und $R^{12}$ = H, die Reste $R^5$, $R^7$ = $CH_3$, der Rest $R^9$ = Phenyl oder 4-Methoxyphenyl und die Reste

$R^{10}$ und $R^{11}$ zusammen = $(CH_2)_5$ bedeuten
oder
$R^3$ bis $R^6$, $R^8$, $R^{12}$ = H, der Rest $R^7$ = $CH_3$, der Rest $R^9$ = 4-Methoxyphenyl oder Phenyl und die Reste $R^{10}$, $R^{11}$ zusammen = $(CH_2)_5$ bedeuten
oder
$R^3$ bis $R^8$ und $R^{12}$ = H, der Rest $R^9$ = Phenyl, der Rest $R^{10}$ = $CH_3$ und der Rest $R^{11}$ = $C_6H_{11}$ oder die Reste $R^{10}$ und $R^{11}$ jeweils = $CH_3$ bedeuten
oder
$R^3$ bis $R^6$, $R^8$, $R^{12}$ = H, der Rest $R^7$ = $CH_3$, der Rest $R^9$ = Phenyl oder 4-Methoxyphenyl und die Reste $R^{10}$ und $R^{11}$ zusammen = $(CH_2)_2O(CH_2)_2$ bedeuten
oder
$R^3$, $R^4$, $R^6$, $R^8$, $R^{12}$ = H, die Reste $R^5$ und $R^7$ = $CH_3$, der Rest $R^8$ = 4-Methoxyphenyl und die Reste $R^{10}$ und $R^{11}$ zusammen = $(CH_2)_2O(CH_2)_2$ bedeuten
oder
$R^3$ bis $R^8$, $R^{12}$ = H, der Rest $R^9$ = Phenyl und die Reste $R^{10}$ und $R^{11}$ zusammen = $(CH_2)_2O(CH_2)_2$ bedeuten als Hydrochlorid
ausgenommen sind.

2. Substituierte 1- und 2-Naphthol-Mannichbasen gemäß Anspruch 1, **dadurch gekennzeichnet, daß** wenigstens einer der Reste $R^3$, $R^4$, $R^5$, $R^6$, $R^7$ oder $R^8$ für H steht und die übrigen Reste $R^3$, $R^4$, $R^5$, $R^6$, $R^7$ oder $R^8$, $R^9$ bis $R^{18}$ und $R^{20}$ bis $R^{22}$ die Bedeutung gemäß Anspruch 1 haben.

3. Substituierte 1- und 2-Naphthol-Mannichbasen gemäß Anspruch 1, **dadurch gekennzeichnet, daß** wenigstens einer der Reste $R^3$, $R^4$, $R^5$, $R^6$, $R^7$ oder $R^8$ für einen $C_{1-6}$-Alkyl-Rest steht und die übrigen Reste $R^3$, $R^4$, $R^5$, $R^6$, $R^7$ oder $R^8$, $R^9$ bis $R^{18}$ und $R^{20}$ bis $R^{22}$ die Bedeutung gemäß Anspruch 1 haben.

4. Substituierte 1- und 2-Naphthol-Mannichbasen gemäß Anspruch 1, **dadurch gekennzeichnet, daß** wenigstens einer der Reste $R^3$, $R^4$, $R^5$, $R^6$, $R^7$ oder $R^8$ für einen über eine $C_{1-2}$-Alkylen-Gruppe gebundenen Aryl-Rest steht und die übrigen Reste $R^3$, $R^4$, $R^5$, $R^6$, $R^7$ oder $R^8$, $R^9$ bis $R^{18}$ und $R^{20}$ bis $R^{22}$ die Bedeutung gemäß Anspruch 1 haben.

5. Substituierte 1- und 2-Naphthol-Mannichbasen gemäß Anspruch 1, **dadurch gekennzeichnet, daß** wenigstens einer der Reste $R^3$, $R^4$, $R^5$, $R^6$, $R^7$ oder $R^8$ für F, Cl oder Br steht und die übrigen Reste $R^3$, $R^4$, $R^5$, $R^6$, $R^7$ oder $R^8$, $R^9$ bis $R^{18}$ und $R^{20}$ bis $R^{22}$ die Bedeutung gemäß Anspruch 1 haben.

6. Substituierte 1- und 2-Naphthol-Mannichbasen gemäß Anspruch 1, **dadurch gekennzeichnet, daß** wenigstens einer der Reste $R^3$, $R^4$, $R^5$, $R^6$, $R^7$ oder $R^8$ für $SO_2NH_2$ steht und die übrigen Reste $R^3$, $R^4$, $R^5$, $R^6$, $R^7$ oder $R^8$, $R^9$ bis $R^{18}$ und $R^{20}$ bis $R^{22}$ die Bedeutung gemäß Anspruch 1 haben.

7. Substituierte 1- und 2-Naphthol-Mannichbasen gemäß Anspruch 1, **dadurch gekennzeichnet, daß** wenigstens einer der Reste $R^3$, $R^4$, $R^5$, $R^6$, $R^7$ oder $R^8$ für $NHR^{13}$ steht und die übrigen Reste $R^3$, $R^4$, $R^5$, $R^6$, $R^7$ oder $R^8$, $R^9$ bis $R^{18}$ und $R^{20}$ bis $R^{22}$ die Bedeutung gemäß Anspruch 1 haben.

8. Substituierte 1- und 2-Naphthol-Mannichbasen gemäß Anspruch 1, **dadurch gekennzeichnet, daß** wenigstens einer der Reste $R^3$, $R^4$, $R^5$, $R^6$, $R^7$ oder $R^8$ für $CO(R^{22})$ steht und die übrigen Reste $R^3$, $R^4$, $R^5$, $R^6$, $R^7$ oder $R^8$, $R^9$ bis $R^{18}$ und $R^{20}$ bis $R^{22}$ die Bedeutung gemäß Anspruch 1 haben.

9. Substituierte 1- und 2-Naphthol-Mannichbasen gemäß Anspruch 1, **dadurch gekennzeichnet, daß** wenigstens einer der Reste $R^3$, $R^4$, $R^5$, $R^6$, $R^7$ oder $R^8$ für $OR^{16}$ steht und die übrigen Reste $R^3$, $R^4$, $R^5$, $R^6$, $R^7$ oder $R^8$, $R^9$ bis $R^{18}$ und $R^{20}$ bis $R^{22}$ die Bedeutung gemäß Anspruch 1 haben.

10. Substituierte 1- und 2-Naphthol-Mannichbasen gemäß Anspruch 1, **dadurch gekennzeichnet, daß** wenigstens einer der Reste $R^3$, $R^4$, $R^5$, $R^6$, $R^7$ oder $R^8$ für $CO(OR^{20})$ steht und die übrigen Reste $R^3$, $R^4$, $R^5$, $R^6$, $R^7$ oder $R^8$, $R^9$ bis $R^{18}$ und $R^{20}$ bis $R^{22}$ die Bedeutung gemäß Anspruch 1 haben.

11. Substituierte 1- und 2-Naphthol-Mannichbasen gemäß einem der Ansprüche 1 bis 10, **dadurch gekennzeichnet, daß** der Rest $R^9$ einen unsubstituierten Phenyl-Rest oder einen wenigstens einfach mit $C_{1-4}$-Alkyl-, $C_{1-3}$-Alkoxy-, Halogen-, $CF_3$-, CN-, O-Phenyl- oder OH substituierten Phenyl-Rest, bevorzugt einen unsubstituierten Phenyl-Rest oder einen 2-Methoxy-phenyl-, 3-Methoxy-phenyl-, 4-Methoxy-phenyl-, 2-Methyl-phenyl-, 3-Methyl-phenyl-, 4-Methyl-phenyl-, 2-tert-Butyl-phenyl-, 3-tert-Butyl-phenyl-, 4-tert-Butyl-phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 5-Brom-2-Fluor-phenyl-, 2-Chlor-4-Fluor-phenyl-, 2-Chlor-5-Fluor-phenyl-, 2-Chlor-6-Fluor-phenyl-, 4-Brom-2-Fluor-phenyl-, 3-Brom-4-Fluor-phenyl-, 3-Brom-2-Fluor-phenyl-, 2,3-Dichlor-phenyl-, 2,4-Dichlor-phenyl-, 2,5-Dichlorphenyl-, 3,4-Dichlor-phenyl-, 2,3-Dimethyl-phenyl-, 2,4-Dimethyl-phenyl-, 2,5-Dimethylphenyl-, 2,3-Dimethoxy-phenyl-, 2,4-Dimethoxy-phenyl-, 2,5-Dimethoxy-phenyl-, 3,4-Dimethoxy-phenyl-, 3,4,5-Trimethoxy-phenyl-, 2-Trifluormethyl-phenyl, 3-Trifluormethyl-phenyl oder einen 4-Trifluormethyl-phenyl-Rest, besonders bevorzugt einen unsubstituierten Phenyl-Rest, bedeutet und die Reste $R^{10}$ bis $R^{18}$ und $R^{20}$ bis $R^{22}$ die Bedeutung gemäß Anspruch 1 haben.

**12.** Substituierte 1- und 2-Naphthol-Mannichbasen gemäß einem der Ansprüche 1 bis 11, **dadurch gekennzeichnet, daß** wenigstens einer der Reste $R^{10}$ oder $R^{11}$ für einen gesättigten, unsubstituierten oder wenigstens einfach substituierten $C_{1-6}$-Alkyl-Rest, vorzugsweise für einen $CH_3$-Rest, steht und der jeweils andere Rest $R^{10}$ oder $R^{11}$ sowie die Reste $R^{12}$ bis $R^{18}$ und $R^{20}$ bis $R^{22}$ die Bedeutung gemäß Anspruch 1 haben.

**13.** Substituierte 1- und 2-Naphthol-Mannichbasen gemäß einem der Ansprüche 1 bis 11, **dadurch gekennzeichnet, daß** die Reste $R^{10}$ und $R^{11}$ zusammen $(CH_2)_n$ mit n = 4 oder 5 bedeuten und die Reste $R^{12}$ bis $R^{18}$ und $R^{20}$ bis $R^{22}$ die Bedeutung gemäß Anspruch 1 haben.

**14.** Substituierte 1- und 2-Naphthol-Mannichbasen gemäß einem der Ansprüche 1 bis 13, **dadurch gekennzeichnet, daß** der Rest $R^{12}$ für H steht und die Reste $R^{13}$ bis $R^{18}$ und $R^{20}$ bis $R^{22}$ die Bedeutung gemäß Anspruch 1 haben.

**15.** Substituierte 1- und 2-Naphthol-Mannichbasen gemäß einem der Ansprüche 1 bis 13, **dadurch gekennzeichnet, daß** der Rest $R^{12}$ für einen $C_{1-6}$-Alkyl-Rest steht und die Reste $R^{13}$ bis $R^{18}$ und $R^{20}$ bis $R^{22}$ die Bedeutung gemäß Anspruch 1 haben.

**16.** Substituierte 1- und 2-Naphthol-Mannichbasen gemäß einem der Ansprüche 1 bis 13, **dadurch gekennzeichnet, daß** der Rest $R^{12}$ für einen über eine $C_{1-2}$-Alkylen-Gruppe gebundenen Aryl-Rest steht und die Reste $R^{13}$ bis $R^{18}$ und $R^{20}$ bis $R^{22}$ die Bedeutung gemäß Anspruch 1 haben.

**17.** Substituierte 1- und 2-Naphthol-Mannichbasen gemäß einem der Ansprüche 1 bis 16, **dadurch gekennzeichnet, daß** der Rest $R^{13}$ für H steht und die Reste $R^{14}$ bis $R^{18}$ und $R^{20}$ bis $R^{22}$ die Bedeutung gemäß Anspruch 1 haben.

**18.** Substituierte 1- und 2-Naphthol-Mannichbasen gemäß einem der Ansprüche 1 bis 16, **dadurch gekennzeichnet, daß** der Rest $R^{13}$ für einen $C_{1-6}$-Alkyl-Rest steht und die Reste $R^{14}$ bis $R^{18}$ und $R^{20}$ bis $R^{22}$ die Bedeutung gemäß Anspruch 1 haben.

**19.** Substituierte 1- und 2-Naphthol-Mannichbasen gemäß einem der Ansprüche 1 bis 16, **dadurch gekennzeichnet, daß** der Rest $R^{13}$ für einen über eine $C_{1-2}$-Alkylen-Gruppe gebundenen Aryl-Rest steht und die Reste $R^{14}$ bis $R^{18}$ und $R^{20}$ bis $R^{22}$ die Bedeutung gemäß Anspruch 1 haben.

**20.** Substituierte 1- und 2-Naphthol-Mannichbasen gemäß einem der Ansprüche 1 bis 19, **dadurch gekennzeichnet, daß** der Rest $R^{14}$ für einen $C_{1-6}$-Alkyl-Rest steht und die Reste $R^{15}$ bis $R^{18}$ und $R^{20}$ bis $R^{22}$ die Bedeutung gemäß Anspruch 1 haben.

**21.** Substituierte 1- und 2-Naphthol-Mannichbasen gemäß einem der Ansprüche 1 bis 19, **dadurch gekennzeichnet, daß** der Rest $R^{14}$ für einen über eine $C_{1-2}$-Alkylen-Gruppe gebundenen Aryl-Rest steht und die Reste $R^{15}$ bis $R^{18}$ und $R^{20}$ bis $R^{22}$ die Bedeutung gemäß Anspruch 1 haben.

**22.** Substituierte 1- und 2-Naphthol-Mannichbasen gemäß einem der Ansprüche 1 bis 21, **dadurch gekennzeichnet, daß** der Rest $R^{15}$ für einen $C_{1-6}$-Alkyl-Rest steht und die Reste $R^{16}$ bis $R^{18}$ und $R^{20}$ bis $R^{22}$ die Bedeutung gemäß Anspruch 1 haben.

**23.** Substituierte 1- und 2-Naphthol-Mannichbasen gemäß einem der Ansprüche 1 bis 21, **dadurch gekennzeichnet, daß** der Rest $R^{15}$ für einen über eine $C_{1-2}$-Alkylen-Gruppe gebundenen Aryl-Rest steht und die Reste $R^{16}$ bis $R^{18}$ und $R^{20}$ bis $R^{22}$ die Bedeutung gemäß Anspruch 1 haben.

**24.** Substituierte 1- und 2-Naphthol-Mannichbasen gemäß einem der Ansprüche 1 bis 23, **dadurch gekennzeichnet, daß** der Rest $R^{16}$ für einen $C_{1-6}$-Alkyl-Rest steht und die Reste $R^{17}$, $R^{18}$ und $R^{20}$ bis $R^{22}$ die Bedeutung gemäß Anspruch 1 haben.

**25.** Substituierte 1- und 2-Naphthol-Mannichbasen gemäß einem der Ansprüche 1 bis 23, **dadurch gekennzeichnet, daß** der Rest $R^{16}$ für einen über eine $C_{1-2}$-Alkylen-Gruppe gebundenen Aryl-Rest steht und die Reste $R^{17}$, $R^{18}$ und $R^{20}$ bis $R^{22}$ die Bedeutung gemäß Anspruch 1 haben.

**26.** Substituierte 1- und 2-Naphthol-Mannichbasen gemäß einem der Ansprüche 1 bis 23, **dadurch gekennzeichnet, daß** der Rest $R^{16}$ für H steht und die Reste $R^{17}$, $R^{18}$ und $R^{20}$ bis $R^{22}$ die Bedeutung gemäß Anspruch 1 haben.

**27.** Substituierte 1- und 2-Naphthol-Mannichbasen gemäß einem der Ansprüche 1 bis 23, **dadurch gekennzeichnet, daß** der Rest $R^{16}$ für $CO(R^{17})$ steht und die Reste $R^{17}$, $R^{18}$ und $R^{20}$ bis $R^{22}$ die Bedeutung gemäß Anspruch 1 haben.

**28.** Substituierte 1- und 2-Naphthol-Mannichbasen gemäß einem der Ansprüche 1 bis 27, **dadurch gekennzeichnet, daß** der Rest $R^{17}$ für einen $C_{1-6}$-Alkyl-Rest steht und die Reste $R^{18}$ und $R^{20}$ bis $R^{22}$ die Bedeutung gemäß Anspruch 1 haben.

**29.** Substituierte 1- und 2-Naphthol-Mannichbasen gemäß einem der Ansprüche 1 bis 27, **dadurch gekennzeichnet, daß** der Rest $R^{17}$ für einen über eine $C_{1-2}$-Alkylen-Gruppe gebundenen Aryl-Rest steht und die Reste $R^{18}$ und $R^{20}$ bis $R^{22}$ die Bedeutung gemäß Anspruch 1 haben.

**30.** Substituierte 1- und 2-Naphthol-Mannichbasen gemäß einem der Ansprüche 1 bis 27, **dadurch gekennzeichnet, daß** der Rest $R^{17}$ für einen gegebenenfalls mit F-, Cl-, Br-, $C_{1-4}$-Alkyl-, $C_{1-3}$-Alkoxy- substituierten Phenyl-Rest steht und die Reste $R^{18}$ und $R^{20}$ bis $R^{22}$ die Bedeutung gemäß Anspruch 1 haben.

**31.** Substituierte 1- und 2-Naphthol-Mannichbasen gemäß einem der Ansprüche 1 bis 30, **dadurch gekennzeichnet, daß** der Rest $R^{18}$ einen $C_{1-6}$-Alkyl-Rest steht und die Reste $R^{20}$ bis $R^{22}$ die Bedeutung gemäß Anspruch 1 haben.

**32.** Substituierte 1- und 2-Naphthol-Mannichbasen gemäß einem der Ansprüche 1 bis 30, **dadurch gekennzeichnet, daß** der Rest $R^{18}$ für einen über eine $C_{1-2}$-Alkylen-Gruppe gebundenen Aryl-Rest steht und die Reste $R^{20}$ bis $R^{22}$ die Bedeutung gemäß Anspruch 1 haben.

**33.** Substituierte 1- und 2-Naphthol-Mannichbasen gemäß einem der Ansprüche 1 bis 30, **dadurch gekennzeichnet, daß** der Rest $R^{18}$ für einen gegebenenfalls mit F-, Cl-, Br-, $C_{1-4}$-Alkyl-, oder $C_{1-3}$-Alkoxy- substituierten Phenyl- oder Naphthyl-Rest, bevorzugt für einen gegebenenfalls mit F-, Cl-, Br-, $C_{1-4}$-Alkyl-, $C_{1-3}$-Alkoxy- substituierten Phenyl-Rest, steht und die Reste $R^{20}$ bis $R^{22}$ die Bedeutung gemäß Anspruch 1 haben.

**34.** Substituierte 1- und 2-Naphthol-Mannichbasen gemäß einem der Ansprüche 1 bis 33, **dadurch gekennzeichnet, daß** der Rest $R^{20}$ für einen $C_{1-6}$-Alkyl-Rest steht und die Reste $R^{21}$ und $R^{22}$ die Bedeutung gemäß Anspruch 1 haben.

**35.** Substituierte 1- und 2-Naphthol-Mannichbasen gemäß einem der Ansprüche 1 bis 33, **dadurch gekennzeichnet, daß** der Rest $R^{20}$ für einen über eine $C_{1-2}$-Alkylen-Gruppe gebundenen Aryl-Rest steht und die Reste $R^{21}$ und $R^{22}$ die Bedeutung gemäß Anspruch 1 haben.

**36.** Substituierte 1- und 2-Naphthol-Mannichbasen gemäß einem der Ansprüche 1 bis 33, **dadurch gekennzeichnet, daß** der Rest $R^{20}$ für H steht und die Reste $R^{21}$ und $R^{22}$ die Bedeutung gemäß Anspruch 1 haben.

**37.** Substituierte 1- und 2-Naphthol-Mannichbasen gemäß einem der Ansprüche 1 bis 33, **dadurch gekennzeichnet, daß** der Rest $R^{20}$ für einen gegebenenfalls mit F-, Cl-, Br-, $C_{1-4}$-Alkyl-, oder $C_{1-3}$-Alkoxy- substituierten Phenyl-Rest steht und die Reste $R^{21}$ und $R^{22}$ die Bedeutung gemäß Anspruch 1 haben.

**38.** Substituierte 1- und 2-Naphthol-Mannichbasen gemäß einem der Ansprüche 1 bis 37, **dadurch gekennzeichnet, daß** der Rest $R^{21}$ für H steht und der Rest $R^{22}$ die Bedeutung gemäß Anspruch 1 hat.

**39.** Substituierte 1- und 2-Naphthol-Mannichbasen gemäß einem der Ansprüche 1 bis 37, **dadurch gekennzeichnet,**

**daß** der Rest $R^{21}$ für einen $C_{1-6}$-Alkyl-Rest steht und der Rest $R^{22}$ die Bedeutung gemäß Anspruch 1 hat.

**40.** Substituierte 1- und 2-Naphthol-Mannichbasen gemäß einem der Ansprüche 1 bis 37, **dadurch gekennzeichnet, daß** der Rest $R^{21}$ für einen über eine $C_{1-2}$-Alkylen-Gruppe gebundenen Aryl-Rest steht und der Rest $R^{22}$ die Bedeutung gemäß Anspruch 1 hat.

**41.** Substituierte 1- und 2-Naphthol-Mannichbasen gemäß einem der Ansprüche 1 bis 40, **dadurch gekennzeichnet, daß** der Rest $R^{22}$ für H steht.

**42.** Substituierte 1- und 2-Naphthol-Mannichbasen gemäß einem der Ansprüche 1 bis 40, **dadurch gekennzeichnet, daß** der Rest $R^{22}$ für einen $C_{1-6}$-Alkyl-Rest steht.

**43.** Substituierte 1- und 2-Naphthol-Mannichbasen gemäß einem der Ansprüche 1 bis 40, **dadurch gekennzeichnet, daß** der Rest $R^{22}$ für einen über eine $C_{1-2}$-Alkylen-Gruppe gebundenen Aryl-Rest steht.

**44.** Substituierte 1- und 2-Naphthol-Mannichbasen gemäß einem der Ansprüche 1 bis 40, **dadurch gekennzeichnet, daß** der Rest $R^{22}$ für $NHNH_2$, $NHR^{18}$ oder einen gegebenenfalls mit F-, Cl-, Br-, $C_{1-4}$-Alkyl-, oder $C_{1-3}$-Alkoxy-substituierten Phenyl-Rest, bevorzugt für $NHNH_2$ oder $NHR^{18}$, steht.

**45.** Substituierte 1- und 2-Naphthol-Mannichbasen gemäß Anspruch 1:

6-(Dimethylaminophenylmethyl)-5-hydroxy-naphthalin-1-sulfonsäureamid

4-Amino-2-(dimethylaminophenylmethyl)-naphthalin-1-ol

4-(Dimethylaminophenylmethyl)-3-hydroxy-naphthalin-2-carbonsäurehydrazid

4-(Dimethylaminophenylmethyl)-3-hydroxy-naphthalin-2-carbonsäuremethylester

4-(Dimethylamino-phenyl-methyl)-3-hydroxynaphthalin-2-carbonsäure

4-(Dimethylaminophenylmethyl)-3-hydroxynaphthalin-2-carbonsäurephenylester

[5-(Dimethylaminophenylmethyl)-6-hydroxynaphthalin-2-yl]-phenylmethanon

3-Amino-1-(dimethylaminophenylmethyl)-naphthalin-2-ol

4-(Dimethylaminophenylmethyl)-3-hydroxynaphthalin-2-carbonsäure-(2-methoxy-phenyl)-amid

4-(Dimethylaminophenylmethyl)-3-hydroxy-naphthalin-2-carbonsäure-*o*tolylamid

4-(Dimethylaminophenylmethyl)-3-hydroxynaphthalin-2-carbonsäure naphthalin-1-ylamid

4-(Dimethylaminophenylmethyl)-3-hydroxy-7-methoxynaphthalin-2-carbonsäure

5-(Dimethylaminophenylmethyl)-6-hydroxynaphthalin-2-carbonsäure

1-(Dimethylaminophenylmethyl)-7-methoxynaphthalin-2-ol

1-(Dimethylaminophenylmethyl)-6-methoxynaphthalin-2-ol

5-(Dimethylaminophenylmethyl)-6-hydroxynaphthalin-1-carbonsäure

4-(Dimethylaminophenylmethyl)-3-hydroxy-7-methoxynaphthalin-2-carboxylat-Natriumsalz

4-Chlor-2-(morpholin-4-yl-*o*-tolylmethyl)-naphthalin-1-ol

4-Chlor-2-(piperidin-1-yl-*o*-tolylmethyl)-naphthalin-1-ol

4-Chlor-2-[(2-chlorophenyl)-piperidin-1-yl-methyl]-naphthalin-1-ol

4-Chlor-2-[(2,3-dimethoxyphenyl)-morpholin-4-yl-methyl]-naphthalin-1-ol

5-Amino-2-[(2-chlorophenyl)-piperidin-1-yl-methyl]-naphthalin-1-ol

5-Amino-2-[(2,3-dimethoxyphenyl)-morpholin-4-yl-methyl]-naphthalin-1-ol

3-Hydroxy-4-(piperidin-1-yl-*o*-tolylmethyl)-naphthalin-2-carbonsäurehydrazid

7-Methoxy-1-(morpholin-4-yl-*o*-tolylmethyl)-naphthalin-2-ol

1-[(2-Chlorophenyl)-piperidin-1-yl-methyl]-7-methoxynaphthalin-2-ol

1-[(2,3-Dimethoxyphenyl)-morpholin-4-yl-methyl]-7-methoxynaphthalin-2-ol

6-Brom-1-[(2-methoxyphenyl)-morpholin-4-yl-methyl]-naphthalin-2-ol

6-Hydroxy-5-[(2-methoxyphenyl)-morpholin-4-yl-methyl]-naphthalin-1-carbonsäure

7-Methoxy-1-[(2-methoxyphenyl)-morpholin-4-yl-methyl]-naphthalin-2-ol

6-Methoxy-1-[(2-methoxyphenyl)-morpholin-4-yl-methyl]-naphthalin-2-ol

4-Chlor-2-[(2-methoxyphenyl)-piperidin-1-yl-methyl]-naphthalin-1-ol

6-Brom-1-[(2-methoxyphenyl)-piperidin-1-yl-methyl]-naphthalin-2-ol

6-Methoxy-1-[(2-methoxyphenyl)-piperidin-1-yl-methyl]-naphthalin-2-ol

7-Methoxy-1-[(2-methoxyphenyl)-piperidin-1-yl-methyl]-naphthalin-2-ol

5-Chlor-2-[dimethylamino-(2-methoxy-phenyl)-methyl]-naphthalin-1-ol

{[1-(4-Methoxybenzyloxy)-naphthalin-2-yl]-phenylmethyl}-dimethylamin

{[2-(4-Methoxybenzyloxy)-naphthalin-1-yl]-phenylmethyl}-dimethylamin

4-Methoxybenzoesäure-1-(dimethylaminophenymethyl)-naphthalin-2-yl-ester

2-Chlorobenzoesäure-1-(dimethylaminophenylmethyl)-naphthalin-2-yl-ester

1-(Morpholin-4-yl-phenylmethyl)-naphthalin-2-ol

1-(Phenylpiperidin-1-yl-methyl)-naphthalin-2-ol

2-[(4-Fluor-phenyl)-pyrrolidin-1-yl-methyl]-naphthalin-1-ol.

**46.** Verfahren zur Herstellung von substituierten 1- und 2-Naphthol-Mannichbasen der allgemeinen Formel I gemäß einem der Ansprüche 1 bis 45, in denen der Rest $R^{12}$ für H steht und die Reste $R^1$ bis $R^{11}$, $R^{13}$ bis $R^{18}$ und $R^{20}$ bis $R^{22}$ die Bedeutung gemäß der allgemeinen Formel I haben, **dadurch gekennzeichnet, daß** man aromatische Aldehydverbindungen und/oder heteroaromatische Aldehydverbindungen und/oder aliphatische Aldehydverbindungen der allgemeinen Formel II,

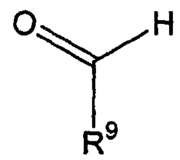

II

in denen $R^9$ die Bedeutung gemäß der allgemeinen Formel I hat, in Lösung in Gegenwart einer Base vorzugsweise bei einer Temperatur von -10 °C bis + 110°C mit sekundären Aminen der allgemeinen Formel III,

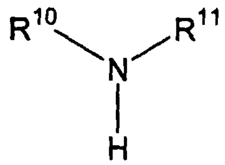

III

in denen $R^{10}$ und $R^{11}$ die Bedeutung gemäß der allgemeinen Formel I haben,
zu Aminalverbindungen der allgemeinen Formel IV

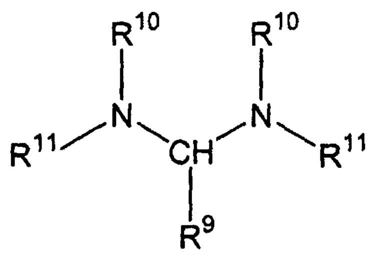

IV

umsetzt, und diese Aminalverbindungen der allgemeinen Formel IV ohne weitere Aufreinigung mit einem Säurechlorid in absolutem Lösungsmittel zu Iminiumsalzen der allgemeinen Formel V

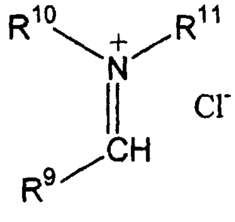

V

umsetzt, und diese Iminiumsalze der allgemeinen Formel V ohne weitere Aufreinigung in Lösung, vorzugsweise

in Acetonitril mit substituierten und/oder unsubstituierten 1- und 2-Naphtholverbindungen der allgemeinen Formel VI,

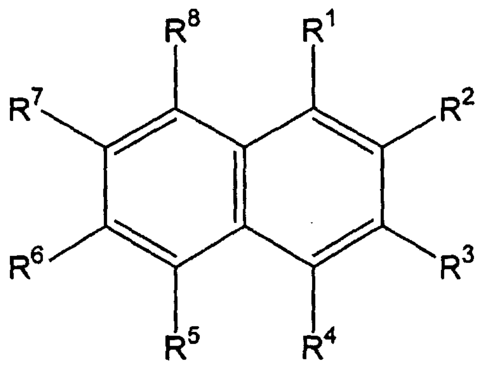

VI

worin $R^1$ = H und $R^2$ = OH oder $R^1$ = OH und $R^2$ = H bedeuten und jeweils die Reste $R^3$ bis $R^8$, $R^{13}$ bis $R^{18}$ und $R^{20}$ bis $R^{22}$ die Bedeutung gemäß der allgemeinen Formel I haben, umsetzt, und die so erhaltenen 1- und 2-Naphthol-Mannichbasen der allgemeinen Formel I, in der der Rest $R^{12}$ für H steht und die Reste $R^1$ bis $R^{11}$, $R^{13}$ bis $R^{18}$ und $R^{20}$ bis $R^{22}$ die Bedeutung gemäß der allgemeinen Formel I haben durch Extraktion reinigt und nach den üblichen Methoden isoliert.

**47.** Verfahren zur Herstellung von substituierten 1- und 2-Naphthol-Mannichbasen der allgemeinen Formel I gemäß einem der Ansprüche 1 bis 45, in denen der Rest $R^{12}$ = $COR^{22}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet und die Reste $R^1$ bis $R^{11}$, $R^{13}$ bis $R^{18}$ und $R^{20}$ bis $R^{22}$ die Bedeutung gemäß der allgemeinen Formel I haben, **dadurch gekennzeichnet, daß** man aromatische Aldehydverbindungen und/oder heteroaromatische Aldehydverbindungen und/oder aliphatische Aldehydverbindungen der allgemeinen Formel II,

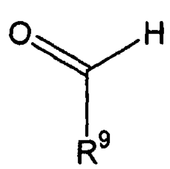

II

in denen $R^9$ die Bedeutung gemäß der allgemeinen Formel I hat, in Lösung in Gegenwart einer Base, vorzugsweise bei einer Temperatur von -10 bis +110 °C mit sekundären Aminen der allgemeinen Formel III,

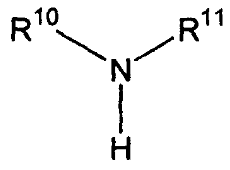

III

in denen $R^{10}$ und $R^{11}$ die Bedeutung gemäß der allgemeinen Formel I haben, zu Aminalverbindungen der allgemeinen Formel IV

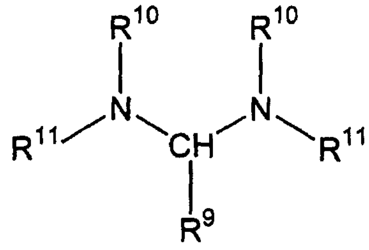

IV

umsetzt, und diese Aminalverbindungen der allgemeinen Formel IV ohne weitere Aufreinigung mit einem Säurechlorid in absolutem Lösungsmittel zu Iminiumsalzen der allgemeinen Formel V

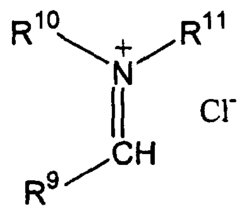

V

umsetzt, und diese Iminiumsalze der allgemeinen Formel V ohne weitere Aufreinigung in Lösung, vorzugsweise in Acetonitril mit substituierten und/oder unsubstituierten 1- und 2-Naphtholverbindungen der allgemeinen Formel VI,

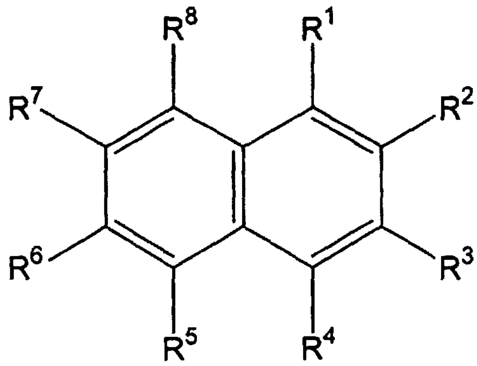

VI

worin $R^1$ = H und $R^2$ = OH oder $R^1$ = OH und $R^2$ = H bedeuten und jeweils die übrigen Reste $R^3$ bis $R^8$, $R^{13}$ bis $R^{18}$ und $R^{20}$ bis $R^{22}$ jeweils die Bedeutung gemäß der allgemeinen Formel I haben, umsetzt, und die so erhaltenen Verbindungen der allgemeinen Formel VI, worin $R^1$ = $CH(R^9)N(R^{10})(R^{11})$ und $R^2$ = OH oder $R^1$ = OH und $R^2$ = $CH(R^9)N(R^{10})(R^{11})$ bedeuten und die Reste $R^3$ bis $R^{11}$, $R^{13}$ bis $R^{18}$ und $R^{20}$ bis $R^{22}$ die Bedeutung gemäß der allgemeinen Formel I haben, in Lösung mit Verbindungen der allgemeinen Formel $XR^{12'}$, worin X = Cl, Br oder I bedeutet und $R^{12'}$ = $COR^{22}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet, in Gegenwart einer Base bei einer Temperatur von vorzugsweise 10 bis 150 °C umsetzt, und die so erhaltenen 1- und 2-Naphthol-Mannichbasen der allgemeinen Formel I, in denen der Rest $R^{12}$ für $COR^{22}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest steht und die Reste $R^1$ bis $R^{11}$, $R^{13}$ bis $R^{18}$ und $R^{20}$ bis $R^{22}$ die Bedeutung gemäß der allgemeinen Formel I haben durch Filtration reinigt und nach den üblichen Methoden isoliert.

48. Verfahren gemäß Anspruch 47, **dadurch gekennzeichnet, daß** die Umsetzung mit den Verbindungen der allgemeinen Formel $XR^{12'}$ in Dimethylformamid erfolgt.

49. Verfahren gemäß Anspruch 47 oder 48, **dadurch gekennzeichnet, daß** X = Cl bedeutet.

50. Verfahren gemäß einem der Ansprüche 47 bis 49, **dadurch gekennzeichnet, daß** die Umsetzung mit den Verbindungen der allgemeinen Formel $XR^{12'}$ in Gegenwart von Triethylamin oder Kalium-tert-Butylat als Base durchgeführt wird.

51. Verfahren gemäß einem der Ansprüche 47 bis 50, **dadurch gekennzeichnet, daß** die Verbindungen der allgemeinen Formel I, in denen $R^{12} \neq$ H ist, durch Filtration über ein Scavenger-Harz, vorzugsweise durch Filtration über polymergebundenes Tris(2-aminoethyl)amin und/oder 3-(3-Mercaptophenyl)propanamidomethylpolystyrol gereinigt werden.

52. Verfahren gemäß einem der Ansprüche 46 bis 51, **dadurch gekennzeichnet, daß** die aromatischen Aldehydverbindungen und/oder heteroaromatischen Aldehydverbindungen und/oder aliphatischen Aldehydverbindungen der allgemeinen Formel II in einem organischen Lösungsmittel, vorzugsweise in Toluol mit sekundären Aminen der allgemeinen Formel III umgesetzt werden.

53. Verfahren gemäß einem der Ansprüche 46 bis 52, **dadurch gekennzeichnet, daß** die aromatischen Aldehydverbindungen und/oder heteroaromatischen Aldehydverbindungen und/oder aliphatischen Aldehydverbindungen der allgemeinen Formel II in Gegenwart von Kaliumcarbonat oder Borsäureanhydrid als Base umgesetzt werden.

54. Verfahren gemäß einem der Ansprüche 46 bis 53, **dadurch gekennzeichnet, daß** die Aminalverbindungen der allgemeinen Formel IV mit Acetylchlorid zu Iminiumsalzen der allgemeinen Formel V umgesetzt werden.

55. Verfahren gemäß einem der Ansprüche 46 bis 54, **dadurch gekennzeichnet, daß** die Aminalverbindungen der allgemeinen Formel IV in absolutem Diethylether zu Iminiumsalzen der allgemeinen Formel V umgesetzt werden.

**56.** Arzneimittel enthaltend als Wirkstoff wenigstens eine substituierte 1- und/oder 2- Naphthol-Mannichbase der allgemeinen Formel I,

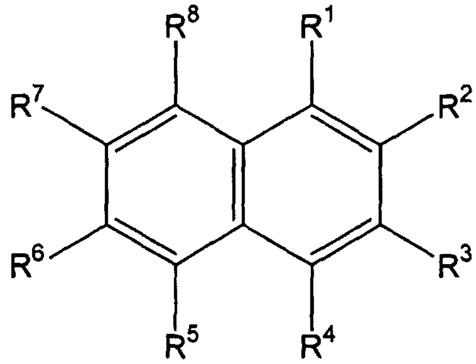

I

worin
$R^1 = CH(R^9)N(R^{10})(R^{11})$ und $R^2 = OR^{12}$ bedeuten
oder
$R^1 = OR^{12}$ und $R^2 = CH(R^9)N(R^{10})(R^{11})$ bedeuten,
und jeweils die Reste
$R^3$ bis $R^8$, gleich oder verschieden, = H, F, Cl, Br, $CF_3$, CN, $NO_2$, $SO_2NH_2$, $SO_2NHR^{13}$, $NHR^{13}$, $SR^{15}$, $OR^{16}$, CO($OR^{20}$), $CH_2CO(OR^{21})$, $CO(R^{22})$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise = H, F, Cl, Br, $SO_2NH_2$, $NHR^{13}$, $CO(R^{22})$, $OR^{16}$, $CO(OR^{20})$, einen $C_{1-6}$-Alkyl- oder einen über eine $C_{1-2}$-Alkylen-Gruppe gebundenen Aryl-Rest, besonders bevorzugt H, $NHR^{13}$, $CO(R^{22})$, $OR^{16}$, $CO(OR^{20})$, bedeuten,
$R^9$ einen Aryl-Rest, einen Heteroaryl-Rest oder einen Alkyl-Rest ohne
acides Proton in $\alpha$-Stellung, vorzugsweise einen unsubstituierten Phenyl-Rest oder einen wenigstens einfach mit $C_{1-4}$-Alkyl-, $C_{1-3}$-Alkoxy-, Halogen-, $CF_3$-, CN-, O-Phenyl- oder OH substituierten Phenyl-Rest, besonders bevorzugt einen unsubstituierten Phenyl-Rest oder einen 2-Methoxy-phenyl-, 3-Methoxy-phenyl-, 4-Methoxy-phenyl-, 2-Methyl-phenyl-, 3-Methyl-phenyl-, 4-Methyl-phenyl-, 2-tert-Butyl-phenyl-, 3-tert-Butyl-phenyl-, 4-tert-Butyl-phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 5-Brom-2-Fluor-phenyl-, 2-Chlor-4-Fluor-phenyl-, 2-Chlor-5-Fluor-phenyl-, 2-Chlor-6-Fluor-phenyl-, 4-Brom-2-Fluor-phenyl-, 3-Brom-4-Fluor-phenyl-, 3-Brom-2-Fluor-phenyl-, 2,3-Dichlor-phenyl-, 2,4-Dichlor-phenyl-, 2,5-Dichlorphenyl-, 3,4-Dichlor-phenyl-, 2,3-Dimethyl-phenyl-, 2,4-Dimethyl-phenyl-, 2,5-Dimethylphenyl-, 2,3-Dimethoxy-phenyl-, 2,4-Dimethoxy-phenyl-, 2,5-Dimethoxy-phenyl-, 3,4-Dimethoxy-phenyl-, 3,4,5-Trimethoxy-phenyl-, 2-Trifluormethyl-phenyl, 3-Trifluormethyl-phenyl oder einen 4-Trifluormethyl-phenyl-Rest, ganz besonders bevorzugt einen unsubstituierten Phenyl-Rest, bedeutet,
$R^{10}$, $R^{11}$, gleich oder verschieden, einen verzweigten, unverzweigten, gesättigten, ungesättigten, unsubstituierten oder wenigstens einfach substituierten $C_{1-6}$-Alkyl-Rest oder einen unsubstituierten oder wenigstens einfach substituierten Phenyl-, Benzyl- oder Phenethyl-Rest, vorzugsweise einen gesättigten, unsubstituierten oder wenigstens einfach substituierten $C_{1-6}$-Alkyl-Rest, besonders bevorzugt einen $CH_3$-Rest, bedeuten,
oder $R^{10}$ und $R^{11}$ zusammen $(CH_2)_n$ mit n = eine ganze Zahl von 3 bis 6 oder $(CH_2)_2O(CH_2)_2$, vorzugsweise $(CH_2)_n$ mit n = 4 oder 5, bedeuten,
$R^{12}$ = H, $COR^{22}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise = H, einen $C_{1-6}$-Alkyl- oder einen über eine $C_{1-2}$-Alkylen-Gruppe gebundenen Aryl-Rest bedeutet,
$R^{13}$ = H, $COR^{14}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise = H, einen $C_{1-6}$-Alkyl- oder einen über eine $C_{1-2}$-Alkylen-Gruppe gebundenen Aryl-Rest, besonders bevorzugt = H, bedeutet,
$R^{14}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,
$R^{15}$ = H, einen $C_{1-10}$-Alkyl-, einen Aryl-, einen Heteroaryl- oder einen über eine $C_{1-6}$-Alkylengruppe gebundenen

Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,
$R^{16}$ = H, CO($R^{17}$), einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise H, einen $C_{1-6}$-Alkyl-, einen über eine $C_{1-2}$-Alkylen-Gruppe gebundenen Aryl-Rest oder CO($R^{17}$), besonders bevorzugt H oder CO($R^{17}$), bedeutet,
$R^{17}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise einen $C_{1-6}$-Alkyl-, einen über eine $C_{1-2}$-Alkylen-Gruppe gebundenen Aryl-Rest oder einen gegebenenfalls mit F-, Cl-, Br-, $C_{1-4}$-Alkyl-, $C_{1-3}$-Alkoxy- substituierten Phenyl-Rest, besonders bevorzugt einen gegebenenfalls mit F-, Cl-, Br-, $C_{1-4}$-Alkyl-, $C_{1-3}$-Alkoxy- substituierten Phenyl-Rest, bedeutet,
$R^{18}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise einen $C_{1-6}$-Alkyl-, einen über eine $C_{1-2}$-Alkylen-Gruppe gebundenen Aryl-Rest oder einen gegebenenfalls mit F-, Cl-, Br-, $C_{1-4}$-Alkyl-, oder $C_{1-3}$-Alkoxy- substituierten Phenyl- oder Naphthyl-Rest, besonders bevorzugt einen gegebenenfalls mit F-, Cl-, Br-, $C_{1-4}$-Alkyl-, $C_{1-3}$-Alkoxy- substituierten Phenyl-Rest, bedeutet,
$R^{20}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise H, einen $C_{1-6}$-Alkyl-, einen über eine $C_{1-2}$-Alkylen-Gruppe gebundenen Aryl-Rest oder einen gegebenenfalls mit F-, Cl-, Br-, $C_{1-4}$-Alkyl-, oder $C_{1-3}$-Alkoxy- substituierten Phenyl-Rest, besonders bevorzugt H oder einen gegebenenfalls mit F-, Cl-, Br-, $C_{1-4}$-Alkyl-, $C_{1-3}$-Alkoxy- substituierten Phenyl-Rest, bedeutet,
$R^{21}$ = H, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise = H, einen $C_{1-6}$-Alkyl- oder einen über eine $C_{1-2}$-Alkylen-Gruppe gebundenen Aryl-Rest bedeutet,
$R^{22}$ = H, $NHNH_2$, $NHR^{18}$, einen $C_{1-10}$-Alkyl-, einen Aryl-, einen Heteroaryl- oder einen über eine $C_{1-6}$-Alkylengruppe gebundenen Aryl- oder Heteroaryl-Rest, vorzugsweise H, einen $C_{1-6}$-Alkyl-, einen über eine $C_{1-2}$-Alkylen-Gruppe gebundenen Aryl-Rest, $NHNH_2$, $NHR^{18}$ oder einen gegebenenfalls mit F-, Cl-, Br-, $C_{1-4}$-Alkyl-, oder $C_{1-3}$-Alkoxy-substituierten Phenyl-Rest, besonders bevorzugt $NHNH_2$, $NHR^{18}$ oder einen gegebenenfalls mit F-, Cl-, Br-, $C_{1-4}$-Alkyl-, oder $C_{1-3}$-Alkoxy- substituierten Phenyl-Rest, ganz besonders bevorzugt $NHNH_2$ oder $NHR^{18}$, bedeutet und/oder deren Racemate, Enantiomere, Diastereomere und/oder entsprechende Basen und/oder entsprechende Salze von physiologisch verträglichen Säuren und gegebenfalls weitere Wirkstoffe und/oder Hilfsstoffe.

**57.** Arzneimittel gemäß Anspruch 56, **dadurch gekennzeichnet, daß** es als Wirkstoff ein Gemisch aus Enantiomeren wenigstens einer substituierten 1-Naphthol-Mannichbase und/oder 2-Naphthol-Mannichbase der allgemeinen Formel I enthält, wobei das Gemisch die Enantiomere in nicht äquimolaren Mengen aufweist.

**58.** Arzneimittel gemäß Anspruch 57, **dadurch gekennzeichnet, daß** der relative Anteil eines der Enantiomere an dem Gemisch 5 bis 45 Mol-%, vorzugsweise 10 bis 40 Mol-%, bezogen auf das Gemisch der Enantiomere beträgt.

**59.** Arzneimittel gemäß einem der Ansprüche 56 bis 58 zur Behandlung/Bekämpfung von Schmerzen und/oder inflammatorischen Reaktionen und/oder allergischen Reaktionen und/oder Drogenmißbrauch und/oder Alkoholmißbrauch und/oder Diarrhoe und/oder Gastritis und/oder Ulcera und/oder cardiovaskulären Erkrankungen und/oder Harninkontinenz und/oder Depressionen und/oder Schockzuständen und/oder Migräne und/oder Narkolepsie und/oder Übergewicht und/oder Asthma und/oder Glaukom und/oder hyperkinetischem Syndrom.

**60.** Verwendung wenigstens einer Verbindung der allgemeinen Formel I gemäß den Ansprüchen 1 bis 45 zur Herstellung eines Arzneimittels zur Bekämpfung von Schmerzen.

**61.** Verwendung wenigstens einer Verbindung der allgemeinen Formel I gemäß den Ansprüchen 1 bis 45 zur Herstellung eines Arzneimittels zur Behandlung von inflammatorischen Reaktionen.

**62.** Verwendung wenigstens einer Verbindung der allgemeinen Formel I gemäß den Ansprüchen 1 bis 45 zur Herstellung eines Arzneimittels zur Behandlung von allergischen Reaktionen.

**63.** Verwendung wenigstens einer Verbindung der allgemeinen Formel I gemäß den Ansprüchen 1 bis 45 zur Herstellung eines Arzneimittels zur Behandlung von Drogen- und/oder Alkoholmißbrauch.

**64.** Verwendung wenigstens einer Verbindung der allgemeinen Formel I gemäß den Ansprüchen 1 bis 45 zur Herstellung eines Arzneimittels zur Behandlung von Diarrhoe.

**65.** Verwendung wenigstens einer Verbindung der allgemeinen Formel I gemäß den Ansprüchen 1 bis 45 zur Herstellung eines Arzneimittels zur Behandlung von Gastritis.

**66.** Verwendung wenigstens einer Verbindung der allgemeinen Formel I gemäß den Ansprüchen 1 bis 45 zur Herstellung eines Arzneimittels zur Behandlung von Ulcera.

**67.** Verwendung wenigstens einer Verbindung der allgemeinen Formel I gemäß den Ansprüchen 1 bis 45 zur Herstellung eines Arzneimittels zur Behandlung von cardiovaskulären Erkrankungen.

**68.** Verwendung wenigstens einer Verbindung der allgemeinen Formel I gemäß den Ansprüchen 1 bis 45 zur Herstellung eines Arzneimittels zur Behandlung von Harninkontinenz.

**69.** Verwendung wenigstens einer Verbindung der allgemeinen Formel I gemäß den Ansprüchen 1 bis 45 zur Herstellung eines Arzneimittels zur Behandlung von Depressionen.

**70.** Verwendung wenigstens einer Verbindung der allgemeinen Formel I gemäß den Ansprüchen 1 bis 45 zur Herstellung eines Arzneimittels zur Behandlung von Schockzuständen.

**71.** Verwendung wenigstens einer Verbindung der allgemeinen Formel I gemäß den Ansprüchen 1 bis 45 zur Herstellung eines Arzneimittels zur Behandlung von Migräne.

**72.** Verwendung wenigstens einer Verbindung der allgemeinen Formel I gemäß den Ansprüchen 1 bis 45 zur Herstellung eines Arzneimittels zur Behandlung von Narkolepsie.

**73.** Verwendung wenigstens einer Verbindung der allgemeinen Formel I gemäß den Ansprüchen 1 bis 45 zur Herstellung eines Arzneimittels zur Behandlung von Übergewicht.

**74.** Verwendung wenigstens einer Verbindung der allgemeinen Formel I gemäß den Ansprüchen 1 bis 45 zur Herstellung eines Arzneimittels zur Behandlung von Asthma.

**75.** Verwendung wenigstens einer Verbindung der allgemeinen Formel I gemäß den Ansprüchen 1 bis 45 zur Herstellung eines Arzneimittels zur Behandlung von Glaukomen.

**76.** Verwendung wenigstens einer Verbindung der allgemeinen Formel I gemäß den Ansprüchen 1 bis 45 zur Herstellung eines Arzneimittels zur Behandlung bei hyperkinetischem Syndrom.

## Claims

**1.** Substituted 1- and 2-naphthol Mannich bases of the general formula I

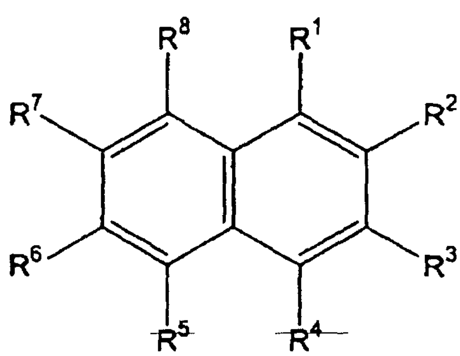

I

wherein

$R^1 = CH(R^9)N(R^{10})(R^{11})$ and $R^2 = OR^{12}$
or
$R^1 = OR^{12}$ and $R^2 = CH(R^9)N(R^{10}) (R^{11})$,
and in each case the radicals
$R^3$ to $R^8$ are identical or different and = H, F, Cl, Br, $CF_3$, CN, $NO_2$, $SO_2NH_2$, $SO_2NHR^{13}$, $NHR^{13}$, $SR^{15}$, $OR^{16}$, CO ($OR^{20}$), $CH_2CO$ ($OR^{21}$), CO($R^{22}$), a $C_{1-10}$-alkyl, an aryl or a heteroaryl radical or an aryl or heteroaryl radical bonded via a $C_{1-6}$-alkylene group,
$R^9$ denotes an aryl radical, a heteroaryl radical or an alkyl radical without an acid proton in the $\alpha$-position,
$R^{10}$, $R^{11}$ are identical or different and denote a branched or unbranched, saturated or unsaturated, unsubstituted or at least monosubstituted $C_{1-6}$-alkyl radical or an unsubstituted or at least monosubstituted phenyl, benzyl or phenethyl radical,
or $R^{10}$ and $R^{11}$ together denote $(CH_2)_2O(CH_2)_2$ or $(CH_2)_n$, where n = an integer from 3 to 6
$R^{12}$ = H, $COR^{22}$, a $C_{1-10}$-alkyl, an aryl or a heteroaryl radical or an aryl or heteroaryl radical bonded via a $C_{1-6}$-alkylene group,
$R^{13}$ = H, $COR^{14}$, a $C_{1-10}$-alkyl, an aryl or a heteroaryl radical or an aryl or heteroaryl radical bonded via a $C_{1-6}$-alkylene group,
$R^{14}$ = H, a $C_{1-10}$-alkyl, an aryl or a heteroaryl radical or an aryl or heteroaryl radical bonded via a $C_{1-6}$-alkylene group,
$R^{15}$ = H, a $C_{1-10}$-alkyl, an aryl or a heteroaryl radical or an aryl or heteroaryl radical bonded via a $C_{1-6}$-alkylene group,
$R^{16}$ = H, CO($R^{17}$), a $C_{1-10}$-alkyl, an aryl or a heteroaryl radical or an aryl or heteroaryl radical bonded via a $C_{1-6}$-alkylene group,
$R^{17}$ = H, a $C_{1-10}$-alkyl, an aryl or a heteroaryl radical or an aryl or heteroaryl radical bonded via a $C_{1-6}$-alkylene group,
$R^{18}$ = H, a $C_{1-10}$-alkyl, an aryl or a heteroaryl radical or an aryl or heteroaryl radical bonded via a $C_{1-6}$-alkylene group,
$R^{20}$ = H, a $C_{1-10}$-alkyl, an aryl or a heteroaryl radical or an aryl or heteroaryl radical bonded via a $C_{1-6}$-alkylene group,
$R^{21}$ = H, a $C_{1-10}$-alkyl, an aryl or a heteroaryl radical or an aryl or heteroaryl radical bonded via a $C_{1-6}$-alkylene group,
$R^{22}$ = H, $NHNH_2$, $NHR^{18}$, a $C_{1-10}$-alkyl, an aryl or a heteroaryl radical or an aryl or heteroaryl radical bonded via a $C_{1-6}$-alkylene group,
and/or their racemates, enantiomers, diastereomers and/or corresponding bases and/or corresponding salts of physiologically tolerated acids,
excluding
the racemates of the compounds in which the radical $R^1 = CH(R^9)N(R^{10})(R^{11})$ and $R^2 = OR^{12}$ and in each case the radicals $R^3$ to $R^8$ and $R^{12}$ = H, the radical $R^9$ = phenyl, 2-chlorophenyl, 4-methoxyphenyl, 3-fluorophenyl, 3-chlorophenyl, 3-bromophenyl, 4-bromophenyl, 2-fluorophenyl, 2-bromophenyl, benzo-1,3-dioxole, 4-$CH_3OCO$-phenyl or 2-methoxyphenyl and the radicals $R^{10}$ and $R^{11}$ together = $(CH_2)_5$
or
the radicals $R^3$ to $R^8$ and $R^{12}$ = H, the radical $R^9$ = phenyl, 4-methoxyphenyl, 4-dimethylaminophenyl, 4-hydroxy-2,3-di-tert-butylphenyl, 2,3-dihydrobenzodioxane, 4-nitrophenyl or benzo-1,3-dioxole and the radicals $R^{10}$ and $R^{11}$ together = $(CH_2)_2O(CH_2)_2$
or
the radicals $R^3$ to $R^8$ and $R^{12}$ = H, the radical $R^9$ = 4-methoxyphenyl and the radicals $R^{10}$ and $R^{11}$ together = $(CH_2)_4$
or
the radical $R^3$ = CO($OR^{20}$), the radicals $R^4$ to $R^8$ and $R^{12}$ = H, the radical $R^9$ = phenyl, 4-methoxyphenyl, 4-methylphenyl, 4-nitrophenyl or p-benzaldehyde, the radicals $R^{10}$ and $R^{11}$ together = $(CH_2)_5$ and the radical $R^{20}$ = $CH_3$
or
the radicals $R^3$ to $R^8$ and $R^{12}$ = H, the radical $R^9$ = phenyl and the two radicals $R^{10}$ and $R^{11}$ each = $CH_3$, $C_2H_5$ or n-$C_3H_7$
or
the radicals $R^3$ to $R^8$ each denote H, the radical $R^{12}$ = $CH_3$, the radical $R^9$ = phenyl and the two radicals $R^{10}$ and $R^{11}$ each = $CH_3$
or
the radicals $R^3$ to $R^8$ and $R^{12}$ = H, the radical $R^9$ = 4-methoxyphenyl or benzo-1,3-dioxole and the radicals $R^{10}$ and $R^{11}$ each = $CH_3$
or
the radicals $R^3$ to $R^5$, $R^7$, $R^8$, $R^{12}$ = H, the radical $R^6$ = Br, the radical $R^9$ = phenyl and the radicals $R^{10}$ and $R^{11}$ together = $(CH_2)_5$
or
the radicals $R^3$ to $R^5$, $R^7$, $R^8$, $R^{12}$ = H, the radical $R^6$ = Br, the radical $R^9$ = 4-hydroxy-3,5-di-tert-butylphenyl and the radicals $R^{10}$ and $R^{11}$ together = $(CH_2)_2O(CH_2)_2$
or

the radicals $R^3$ to $R^8$ and $R^{12}$ = H, the radical $R^9$ = phenyl and the radicals $R^{10}$ and $R^{11}$ each = $CH_3$ as the hydrochloride
or
the radicals $R^3$ to $R^8$ and $R^{12}$ = H, the radical $R^9$ = phenyl or 4-methoxyphenyl and the radicals $R^{10}$ and $R^{11}$ together = $(CH_2)_5$ as the hydrochloride
or
the radical $R^3$ = $CO(OR^{20})$, the radicals $R^4$ to $R^8$ and $R^{12}$ = H, the radical $R^9$ = phenyl, the radicals $R^{10}$ and $R^{11}$ together = $(CH_2)_5$ and the radical $R^{20}$ = $CH_3$ as the hydrochloride
or
the radicals $R^3$ to $R^8$ and $R^{12}$ = H, the radical $R^9$ = thiophene and the radicals $R^{10}$ and $R^{11}$ together = $(CH_2)_2O(CH_2)_2$
or
the radicals $R^3$ to $R^8$ = H, the radical $R^{12}$ = $CH_3$, the radical $R^9$ = thiophene, 4-methoxyphenyl or 3,4-dimethoxyphenyl and the radicals $R^{10}$ and $R^{11}$ together = $(CH_2)_2O(CH_2)_2$
and the enantiomers of the compound of the general formula I in which $R^1$ = $CH(R^9)N(R^{10})(R^{11})$ and $R^2$ = $OR^{12}$ and the radicals $R^3$ to $R^8$, $R^{12}$ = H, $R^9$ = phenyl and $R^{10}$ and $R^{11}$ together = $(CH_2)_5$,
(+)-1-(α-N,N-dimethylaminobenzyl)-2-naphthol and the corresponding tartrate,
(-)-1-(α-N,N-dimethylaminobenzyl)-2-naphthol and the corresponding tartrate, and
(-)-[(2-methoxynaphth-1-yl)benzyl]-dimethylamine
and
the racemates of the compounds in which the radicals $R^1$ = $OR^{12}$ and $R^2$ = $CH(R^9)N(R^{10})(R^{11})$ and in each case the radicals
$R^3$ to $R^8$ and $R^{12}$ = H, the radical $R^9$ = phenyl, 2-bromophenyl, 3-bromophenyl or 4-bromophenyl and the radicals $R^{10}$ and $R^{11}$ together = $(CH_2)_5$
or
$R^3$ to $R^8$ and $R^{12}$ = H, the radical $R^9$ = phenyl or 2-nitrophenyl and the radicals $R^{10}$ and $R^{11}$ together = $(CH_2)_2O(CH_2)_2$
or
$R^3$, $R^4$, $R^6$, $R^8$ and $R^{12}$ = H, the radicals $R^5$, $R^7$ = $CH_3$, the radical $R^9$ = phenyl or 4-methoxyphenyl and the radicals $R^{10}$ and $R^{11}$ together = $(CH_2)_5$
or
$R^3$ to $R^6$, $R^8$, $R^{12}$ = H, the radical $R^7$ = $CH_3$, the radical $R^9$ = 4-methoxyphenyl or phenyl and the radicals $R^{10}$, $R^{11}$ together = $(CH_2)_5$
or
$R^3$ to $R^8$ and $R^{12}$ = H, the radical $R^9$ = phenyl, the radical $R^{10}$ = $CH_3$ and the radical $R^{11}$ = $C_6H_{11}$ or the radicals $R^{10}$ and $R^{11}$ each = $CH_3$
or
$R^3$ to $R^6$, $R^8$, $R^{12}$ = H, the radical $R^7$ = $CH_3$, the radical $R^9$ = phenyl or 4-methoxyphenyl and the radicals $R^{10}$ and $R^{11}$ together = $(CH_2)_2O(CH_2)_2$
or
$R^3$, $R^4$, $R^6$, $R^8$, $R^{12}$ = H, the radicals $R^5$ and $R^7$ = $CH_3$, the radical $R^9$ = 4-methoxyphenyl and the radicals $R^{10}$ and $R^{11}$ together = $(CH_2)_2O(CH_2)_2$
or
$R^3$ to $R^8$, $R^{12}$ = H, the radical $R^9$ = phenyl and the radicals $R^{10}$ and $R^{11}$ together = $(CH_2)_2O(CH_2)_2$ as the hydrochloride.

2. Substituted 1- and 2-naphthol Mannich bases according to claim 1, **characterized in that** at least one of the radicals $R^3$, $R^4$, $R^5$, $R^6$, $R^7$ or $R^8$ represents H and the other radicals $R^3$, $R^4$, $R^5$, $R^6$ $R^7$ or $R^8$, $R^9$ to $R^{18}$ and $R^{20}$ to $R^{22}$ have the meaning according to claim 1.

3. Substituted 1- and 2-naphthol Mannich bases according to claim 1, **characterized in that** at least one of the radicals $R^3$, $R^4$, $R^5$, $R^6$, $R^7$ or $R^8$ represents a $C_{1-6}$-alkyl radical and the other radicals $R^3$, $R^4$, $R^5$, $R^6$, $R^7$ or $R^8$, $R^9$ to $R^{18}$ and $R^{20}$ to $R^{22}$ have the meaning according to claim 1.

4. Substituted 1- and 2-naphthol Mannich bases according to claim 1, **characterized in that** at least one of the radicals $R^3$, $R^4$, $R^5$, $R^6$, $R^7$ or $R^8$ represents an aryl radical bonded via a $C_{1-2}$-alkylene group and the other radicals $R^3$, $R^4$, $R^5$, $R^6$, $R^7$ or $R^8$, $R^9$ to $R^{18}$ and $R^{20}$ to $R^{22}$ have the meaning according to claim 1.

5. Substituted 1- and 2-naphthol Mannich bases according to claim 1, **characterized in that** at least one of the

radicals $R^3$, $R^4$, $R^5$, $R^6$, $R^7$ or $R^8$ represents F, Cl or Br and the other radicals $R^3$, $R^4$, $R^5$, $R^6$, $R^7$ or $R^8$, $R^9$ to $R^{18}$ and $R^{20}$ to $R^{22}$ have the meaning according to claim 1.

**6.** Substituted 1- and 2-naphthol Mannich bases according to claim 1, **characterized in that** at least one of the radicals $R^3$, $R^4$, $R^5$, $R^6$, $R^7$ or $R^8$ represents $SO_2NH_2$ and the other radicals $R^3$, $R^4$, $R^5$, $R^6$, $R^7$ or $R^8$, $R^9$ to $R^{18}$ and $R^{20}$ to $R^{22}$ have the meaning according to claim 1.

**7.** Substituted 1- and 2-naphthol Mannich bases according to claim 1, **characterized in that** at least one of the radicals $R^3$, $R^4$, $R^5$, $R^6$, $R^7$ or $R^8$ represents $NHR^{13}$ and the other radicals $R^3$, $R^4$, $R^5$, $R^6$, $R^7$ or $R^8$, $R^9$ to $R^{18}$ and $R^{20}$ to $R^{22}$ have the meaning according to claim 1.

**8.** Substituted 1- and 2-naphthol Mannich bases according to claim 1, **characterized in that** at least one of the radicals $R^3$, $R^4$, $R^5$, $R^6$, $R^7$ or $R^8$ represents $CO(R^{22})$ and the other radicals $R^3$, $R^4$, $R^5$, $R^6$, $R^7$ or $R^8$, $R^9$ to $R^{18}$ and $R^{20}$ to $R^{22}$ have the meaning according to claim 1.

**9.** Substituted 1- and 2-naphthol Mannich bases according to claim 1, **characterized in that** at least one of the radicals $R^3$, $R^4$, $R^5$, $R^6$, $R^7$ or $R^8$ represents $OR^{16}$ and the other radicals $R^3$, $R^9$, $R^5$, $R^6$, $R^7$ or $R^8$, $R^9$ to $R^{18}$ and $R^{20}$ to $R^{22}$ have the meaning according to claim 1.

**10.** Substituted 1- and 2-naphthol Mannich bases according to claim 1, **characterized in that** at least one of the radicals $R^3$, $R^4$, $R^5$, $R^6$, $R^7$ or $R^8$ represents $CO(OR^{20})$ and the other radicals $R^3$, $R^4$, $R^5$, $R^6$, $R^7$ or $R^8$, $R^9$ to $R^{18}$ and $R^{20}$ to $R^{22}$ have the meaning according to claim 1.

**11.** Substituted 1- and 2-naphthol Mannich bases according to one of claims 1 to 10, **characterized in that** the radical $R^9$ denotes an unsubstituted phenyl radical or a phenyl radical which is at least monosubstituted by $C_{1-4}$-alkyl, $C_{1-3}$-alkoxy, halogen, $CF_3$, CN, O-phenyl or OH, preferably an unsubstituted phenyl radical or a 2-methoxy-phenyl, 3-methoxy-phenyl, 4-methoxy-phenyl, 2-methyl-phenyl, 3-methyl-phenyl, 4-methyl-phenyl, 2-tert-butyl-phenyl, 3-tert-butyl-phenyl, 4-tert-butyl-phenyl, 2-fluoro-phenyl, 3-fluoro-phenyl, 4-fluoro-phenyl, 2-chloro-phenyl, 3-chloro-phenyl, 4-chloro-phenyl, 2-bromo-phenyl, 3-bromo-phenyl, 4-bromo-phenyl, 5-bromo-2-fluoro-phenyl, 2-chloro-4-fluoro-phenyl, 2-chloro-5-fluoro-phenyl, 2-chloro-6-fluoro-phenyl, 4-bromo-2-fluoro-phenyl, 3-bromo-4-fluoro-phenyl, 3-bromo-2-fluoro-phenyl, 2,3-dichloro-phenyl, 2,4-dichloro-phenyl, 2,5-dichlorophenyl, 3,4-dichloro-phenyl, 2,3-dimethyl-phenyl, 2,4-dimethyl-phenyl, 2,5-dimethylphenyl, 2,3-dimethoxy-phenyl, 2,4-dimethoxy-phenyl, 2,5-dimethoxy-phenyl, 3,4-dimethoxy-phenyl, 3,4,5-trimethoxy-phenyl, 2-trifluoromethyl-phenyl, 3-trifluoromethyl-phenyl or 4-trifluoromethyl-phenyl radical, particularly preferably an unsubstituted phenyl radical, and the radicals $R^{10}$ to $R^{18}$ and $R^{20}$ to $R^{22}$ have the meaning according to claim 1.

**12.** Substituted 1- and 2-naphthol Mannich bases according to one of claims 1 to 11, **characterized in that** at least one of the radicals $R^{10}$ or $R^{11}$ represents a saturated, unsubstituted or at least monosubstituted $C_{1-6}$-alkyl radical, preferably a $CH_3$ radical, and the other particular radical $R^{10}$ or $R^{11}$ and the radicals $R^{12}$ to $R^{18}$ and $R^{20}$ to $R^{22}$ have the meaning according to claim 1.

**13.** Substituted 1- and 2-naphthol Mannich bases according to one of claims 1 to 11, **characterized in that** the radicals $R^{10}$ and $R^{11}$ together denote $(CH_2)_n$, where n = 4 or 5, and the radicals $R^{12}$ to $R^{18}$ and $R^{20}$ to $R^{22}$ have the meaning according to claim 1.

**14.** Substituted 1- and 2-naphthol Mannich bases according to one of claims 1 to 13, **characterized in that** the radical $R^{12}$ represents H and the radicals $R^{13}$ to $R^{18}$ and $R^{20}$ to $R^{22}$ have the meaning according to claim 1.

**15.** Substituted 1- and 2-naphthol Mannich bases according to one of claims 1 to 13, **characterized in that** the radical $R^{12}$ represents a $C_{1-6}$-alkyl radical and the radicals $R^{13}$ to $R^{18}$ and $R^{20}$ to $R^{22}$ have the meaning according to claim 1.

**16.** Substituted 1- and 2-naphthol Mannich bases according to one of claims 1 to 13, **characterized in that** the radical $R^{12}$ represents an aryl radical bonded via a $C_{1-2}$-alkylene group and the radicals $R^{13}$ to $R^{18}$ and $R^{20}$ to $R^{22}$ have the meaning according to claim 1.

**17.** Substituted 1- and 2-naphthol Mannich bases according to one of claims 1 to 16, **characterized in that** the radical $R^{13}$ represents H and the radicals $R^{14}$ to $R^{18}$ and $R^{20}$ to $R^{22}$ have the meaning according to claim 1.

**18.** Substituted 1- and 2-naphthol Mannich bases according to one of claims 1 to 16, **characterized in that** the radical $R^{13}$ represents a $C_{1-6}$-alkyl radical and the radicals $R^{14}$ to $R^{18}$ and $R^{20}$ to $R^{22}$ have the meaning according to claim 1.

**19.** Substituted 1- and 2-naphthol Mannich bases according to one of claims 1 to 16, **characterized in that** the radical $R^{13}$ represents an aryl radical bonded via a $C_{1-2}$-alkylene group and the radicals $R^{14}$ to $R^{18}$ and $R^{20}$ to $R^{22}$ have the meaning according to claim 1.

**20.** Substituted 1- and 2-naphthol Mannich bases according to one of claims 1 to 19, **characterized in that** the radical $R^{14}$ represents a $C_{1-6}$-alkyl radical and the radicals $R^{15}$ to $R^{18}$ and $R^{20}$ to $R^{22}$ have the meaning according to claim 1.

**21.** Substituted 1- and 2-naphthol Mannich bases according to one of claims 1 to 19, **characterized in that** the radical $R^{14}$ represents an aryl radical bonded via a $C_{1-2}$-alkylene group and the radicals $R^{15}$ to $R^{18}$ and $R^{20}$ to $R^{22}$ have the meaning according to claim 1.

**22.** Substituted 1- and 2-naphthol Mannich bases according to one of claims 1 to 21, **characterized in that** the radical $R^{15}$ represents a $C_{1-6}$-alkyl radical and the radicals $R^{16}$ to $R^{18}$ and $R^{20}$ to $R^{22}$ have the meaning according to claim 1.

**23.** Substituted 1- and 2-naphthol Mannich bases according to one of claims 1 to 21, **characterized in that** the radical $R^{15}$ represents an aryl radical bonded via a $C_{1-2}$-alkylene group and the radicals $R^{16}$ to $R^{18}$ and $R^{20}$ to $R^{22}$ have the meaning according to claim 1.

**24.** Substituted 1- and 2-naphthol Mannich bases according to one of claims 1 to 23, **characterized in that** the radical $R^{16}$ represents a $C_{1-6}$-alkyl radical and the radicals $R^{17}$, $R^{18}$ and $R^{20}$ to $R^{22}$ have the meaning according to claim 1.

**25.** Substituted 1- and 2-naphthol Mannich bases according to one of claims 1 to 23, **characterized in that** the radical $R^{16}$ represents an aryl radical bonded via a $C_{1-2}$-alkylene group and the radicals $R^{17}$, $R^{18}$ and $R^{20}$ to $R^{22}$ have the meaning according to claim 1.

**26.** Substituted 1- and 2-naphthol Mannich bases according to one of claims 1 to 23, **characterized in that** the radical $R^{16}$ represents H and the radicals $R^{17}$, $R^{18}$ and $R^{20}$ to $R^{22}$ have the meaning according to claim 1.

**27.** Substituted 1- and 2-naphthol Mannich bases according to one of claims 1 to 23, **characterized in that** the radical $R^{16}$ represents $CO(R^{17})$ and the radicals $R^{17}$, $R^{18}$ and $R^{20}$ to $R^{22}$ have the meaning according to claim 1.

**28.** Substituted 1- and 2-naphthol Mannich bases according to one of claims 1 to 27, **characterized in that** the radical $R^{17}$ represents a $C_{1-6}$-alkyl radical and the radicals $R^{18}$ and $R^{20}$ to $R^{22}$ have the meaning according to claim 1.

**29.** Substituted 1- and 2-naphthol Mannich bases according to one of claims 1 to 27, **characterized in that** the radical $R^{17}$ represents an aryl radical bonded via a $C_{1-2}$-alkylene group and the radicals $R^{18}$ and $R^{20}$ to $R^{22}$ have the meaning according to claim 1.

**30.** Substituted 1- and 2-naphthol Mannich bases according to one of claims 1 to 27, **characterized in that** the radical $R^{17}$ represents a phenyl radical which is optionally substituted by F, Cl, Br, $C_{1-4}$-alkyl or $C_{1-3}$-alkoxy and the radicals $R^{18}$ and $R^{20}$ to $R^{22}$ have the meaning according to claim 1.

**31.** Substituted 1- and 2-naphthol Mannich bases according to one of claims 1 to 30, **characterized in that** the radical $R^{18}$ represents a $C_{1-6}$-alkyl radical and the radicals $R^{20}$ to $R^{22}$ have the meaning according to claim 1.

**32.** Substituted 1- and 2-naphthol Mannich bases according to one of claims 1 to 30, **characterized in that** the radical $R^{18}$ represents an aryl radical bonded via a $C_{1-2}$-alkylene group and the radicals $R^{20}$ to $R^{22}$ have the meaning according to claim 1.

**33.** Substituted 1- and 2-naphthol Mannich bases according to one of claims 1 to 30, **characterized in that** the radical $R^{18}$ represents a phenyl or naphthyl radical which is optionally substituted by F, Cl, Br, $C_{1-4}$-alkyl or $C_{1-3}$-alkoxy, preferably a phenyl radical which is optionally substituted by F, Cl, Br, $C_{1-4}$-alkyl or $C_{1-3}$-alkoxy, and the radicals $R^{20}$ to $R^{22}$ have the meaning according to claim 1.

**34.** Substituted 1- and 2-naphthol Mannich bases according to one of claims 1 to 33, **characterized in that** the radical

$R^{20}$ represents a $C_{1-6}$-alkyl radical and the radicals $R^{21}$ and $R^{22}$ have the meaning according to claim 1.

**35.** Substituted 1- and 2-naphthol Mannich bases according to one of claims 1 to 33, **characterized in that** the radical $R^{20}$ represents an aryl radical bonded via a $C_{1-2}$-alkylene group and the radicals $R^{21}$ and $R^{22}$ have the meaning according to claim 1.

**36.** Substituted 1- and 2-naphthol Mannich bases according to one of claims 1 to 33, **characterized in that** the radical $R^{20}$ represents H and the radicals $R^{21}$ and $R^{22}$ have the meaning according to claim 1.

**37.** Substituted 1- and 2-naphthol Mannich bases according to one of claims 1 to 33, **characterized in that** the radical $R^{20}$ represents a phenyl radical which is optionally substituted by F, Cl, Br, $C_{1-4}$-alkyl or $C_{1-3}$-alkoxy and the radicals $R^{21}$ and $R^{22}$ have the meaning according to claim 1.

**38.** Substituted 1- and 2-naphthol Mannich bases according to one of claims 1 to 37, **characterized in that** the radical $R^{21}$ represents H and the radical $R^{22}$ has the meaning according to claim 1.

**39.** Substituted 1- and 2-naphthol Mannich bases according to one of claims 1 to 37, **characterized in that** the radical $R^{21}$ represents a $C_{1-6}$-alkyl radical and the radical $R^{22}$ has the meaning according to claim 1.

**40.** Substituted 1- and 2-naphthol Mannich bases according to one of claims 1 to 37, **characterized in that** the radical $R^{21}$ represents an aryl radical bonded via a $C_{1-2}$-alkylene group and the radical $R^{22}$ has the meaning according to claim 1.

**41.** Substituted 1- and 2-naphthol Mannich bases according to one of claims 1 to 40, **characterized in that** the radical $R^{22}$ represents H.

**42.** Substituted 1- and 2-naphthol Mannich bases according to one of claims 1 to 40, **characterized in that** the radical $R^{22}$ represents a $C_{1-6}$-alkyl radical.

**43.** Substituted 1- and 2-naphthol Mannich bases according to one of claims 1 to 40, **characterized in that** the radical $R^{22}$ represents an aryl radical bonded via a $C_{1-2}$-alkylene group.

**44.** Substituted 1- and 2-naphthol Mannich bases according to one of claims 1 to 40, **characterized in that** the radical $R^{22}$ represents $NHNH_2$, $NHR^{18}$ or a phenyl radical which is optionally substituted by F, Cl, Br, $C_{1-4}$-alkyl or $C_{1-3}$-alkoxy, preferably $NHNH_2$ or $NHR^{18}$.

**45.** Substituted 1- and 2-naphthol Mannich bases according to claim 1:

6-(dimethylaminophenylmethyl)-5-hydroxy-naphthalene-1-sulfonic acid amide

4-amino-2-(dimethylaminophenylmethyl)-naphthalen-1-ol

4-(dimethylaminophenylmethyl)-3-hydroxy-naphthalene-2-carboxylic acid hydrazide

4-(dimethylaminophenylmethyl)-3-hydroxy-naphthalene-2-carboxylic acid methyl ester

4-(dimethylamino-phenyl-methyl)-3-hydroxy-naphthalene-2-carboxylic acid

4-(dimethylaminophenylmethyl)-3-hydroxy-naphthalene-2-carboxylic acid phenyl ester

[5-(dimethylaminophenylmethyl)-6-hydroxynaphthalen-2-yl]-phenylmethanone

3-amino-1-(dimethylaminophenylmethyl)-naphthalen-2-ol

4-(dimethylaminophenylmethyl)-3-hydroxynaphthalene-2-carboxylic acid (2-methoxy-phenyl)-amide

4-(dimethylaminophenylmethyl)-3-hydroxy-naphthalene-2-carboxylic acid *o*-tolylamide

4-(dimethylaminophenylmethyl)-3-hydroxynaphthalene-2-carboxylic acid naphthalen-1-ylamide

4-(dimethylaminophenylmethyl)-3-hydroxy-7-methoxynaphthalene-2-carboxylic acid

5-(dimethylaminophenylmethyl)-6-hydroxynaphthalene-2-carboxylic acid

1-(dimethylaminophenylmethyl)-7-methoxynaphthalen-2-ol

1-(dimethylaminophenylmethyl)-6-methoxynaphthalen-2-ol

5-(dimethylaminophenylmethyl)-6-hydroxynaphthalene-1-carboxylic acid

4-(dimethylaminophenylmethyl)-3-hydroxy-7-methoxynaphthalene-2-carboxylate sodium salt

4-chloro-2-(morpholin-4-yl-*o*-tolylmethyl)-naphthalen-1-ol

4-chloro-2-(piperidin-1-yl-*o*-tolylmethyl)-naphthalen-1-ol

4-chloro-2-[(2-chlorophenyl)-piperidin-1-yl-methyl]-naphthalen-1-ol

4-chloro-2-[(2,3-dimethoxyphenyl)-morpholin-4-yl-methyl]-naphthalen-1-ol

5-amino-2-[(2-chlorophenyl)-piperidin-1-yl-methyl]-naphthalen-1-ol

5-amino-2-[(2,3-dimethoxyphenyl)-morpholin-4-yl-methyl]-naphthalen-1-ol

3-hydroxy-4-(piperidin-1-yl-*o*-tolylmethyl)-naphthalene-2-carboxylic acid hydrazide

7-methoxy-1-(morpholin-4-yl-*o*-tolylmethyl)-naphthalen-2-ol

1-[(2-chlorophenyl)-piperidin-1-yl-methyl]-7-methoxynaphthalen-2-ol

1-[(2,3-dimethoxyphenyl)-morpholin-4-yl-methyl]-7-methoxynaphthalen-2-ol

6-bromo-1-[(2-methoxyphenyl)-morpholin-4-yl-methyl]-naphthalen-2-ol

6-hydroxy-5-[(2-methoxyphenyl)-morpholin-4-yl-methyl]-naphthalene-1-carboxylic acid

7-methoxy-1-[(2-methoxyphenyl)-morpholin-4-yl-mehtyl]-naphthalen-2-ol

6-methoxy-1-[(2-methoxyphenyl)-morpholin-4-yl-methyl]-naphthalen-2-ol

4-chloro-2-[(2-methoxyphenyl)-piperidin-1-yl-methyl]-naphthalen-1-ol

6-bromo-1-[(2-methoxyphenyl)-piperidin-1-yl-methyl]-naphthalen-1-ol

6-methoxy-1-[(2-methoxyphenyl)-piperidin-1-yl-methyl]-naphthalen-2-ol

7-methoxy-1-[(2-methoxyphenyl)-piperidin-1-yl-methyl]-naphthalen-2-ol

5-chloro-2-[dimethylamino-(2-methoxyphenyl)-methyl]-naphthalen-1-ol

{[1-(4-methoxybenzyloxy)-naphthalen-2-yl]-phenylmethyl}-dimethylamine

{[2-(4-methoxybenzyloxy)-naphthalen-1-yl]-phenylmethyl}-dimethylamine

4-methoxybenzoic acid 1-(dimethylaminophenylmethyl)-naphthalen-2-yl ester

2-chlorobenzoic acid 1-(dimethylaminophenylmethyl)-naphthalen-2-yl ester

1-(morpholin-4-yl-phenylmethyl)-naphthalen-2-ol

1-(phenylpiperidin-1-yl-methyl)-naphthalen-2-ol

2-[(4-fluoro-phenyl)-pyrrolidin-1-yl-methyl]-naphthalen-1-ol.

**46.** Process for the preparation of substituted 1- and 2-naphthol Mannich bases of the general formula I according to one of claims 1 to 45, in which the radical $R^{12}$ represents H and the radicals $R^1$ to $R^{11}$, $R^{13}$ to $R^{18}$ and $R^{20}$ to $R^{22}$ have the meaning according to the general formula I, **characterized in that** aromatic aldehyde compounds and/or heteroaromatic aldehyde compounds and/or aliphatic aldehyde compounds of the general formula II

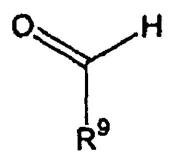

II

in which $R^9$ has the meaning according to the general formula I, are reacted in solution in the presence of a base, preferably at a temperature of -10°C to +110°C, with secondary amines of the general formula III

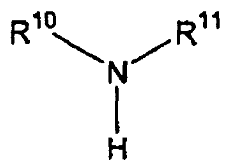

III

in which $R^{10}$ and $R^{11}$ have the meaning according to the general formula I, to give aminal compounds of the general formula IV

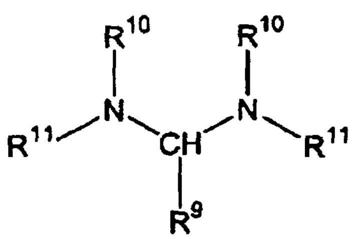

IV

and these aminal compounds of the general formula IV are reacted, without further purification, with an acid chloride in an absolute solvent to give iminium salts of the general formula V

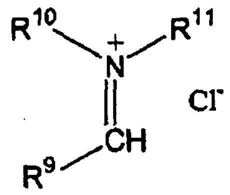

V

and these iminium salts of the general formula V are reacted, without further purification and in solution, preferably in acetonitrile, with substituted and/or unsubstituted 1- and 2-naphthol compounds of the general formula VI

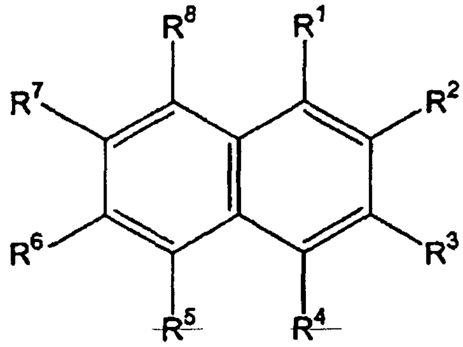

VI

wherein $R^1$ = H and $R^2$ = OH or $R^1$ = OH and $R^2$ = H and in each case the radicals $R^3$ to $R^8$, $R^{13}$ to $R^{18}$ and $R^{20}$ to $R^{22}$ have the meaning according to the general formula I, and the 1- and 2-naphthol Mannich bases of the general formula I obtained in this way in which the radical $R^{12}$ represents H and the radicals $R^1$ to $R^{11}$, $R^{13}$ to $R^{18}$ and $R^{20}$ to $R^{22}$ have the meaning according to the general formula I are purified by extraction and are isolated by conventional methods.

**47.** Process for the preparation of substituted 1- and 2-naphthol Mannich bases of the general formula I according to one of claims 1 to 45 in which the radical $R^{12}$ = $COR^{22}$, a $C_{1-10}$-alkyl, an aryl or a heteroaryl radical or an aryl or heteroaryl radical bonded via a $C_{1-6}$-alkylene group and the radicals $R^1$ to $R^{11}$, $R^{23}$ to $R^{18}$ and $R^{20}$ to $R^{22}$ have the meaning according to the general formula I, **characterized in that** aromatic aldehyde compounds and/or heteroaromatic aldehyde compounds and/or aliphatic aldehyde compounds of the general formula II

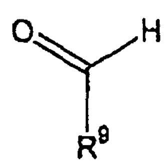

II

in which $R^9$ has the meaning according to the general formula I, are reacted in solution in the presence of a base, preferably at a temperature of 10 to +110°C, with secondary amines of the general formula III

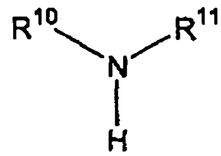

III

in which $R^{10}$ and $R^{11}$ have the meaning according to the general formula I, to give aminal compounds of the general formula IV

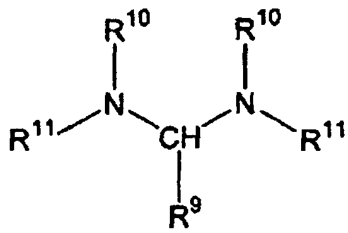

IV

and these aminal compounds of the general formula IV are reacted, without further purification, with an acid chloride in an absolute solvent to give iminium salts of the general formula V

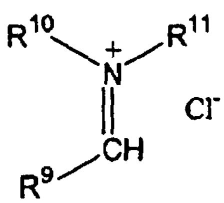

V

and these iminium salts of the general formula V are reacted, without further purification and in solution, preferably in acetonitrile, with substituted and/or unsubstituted 1- and 2-naphthol compounds of the general formula VI

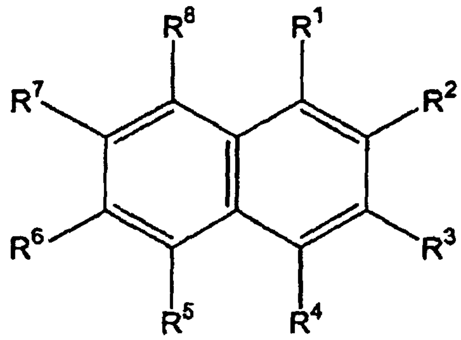

VI

wherein $R^1$ = H and $R^2$ = OH or $R^1$ = OH and $R^2$ = H and in each case the other radicals $R^3$ to $R^8$, $R^{13}$ to $R^{18}$ and $R^{20}$ to $R^{22}$ in each case have the meaning according to the general formula I, and the compounds of the general formula VI obtained in this way, wherein $R^1$ = $CH(R^9)N(R^{10})$ $(R^{11})$ and $R^2$ = OH or $R^1$ = OH and $R^2$ = $CH(R^9)N$ $(R^{10})$ $(R^{11})$ and the radicals $R^3$ to $R^{11}$, $R^{13}$ to $R^{18}$ and $R^{20}$ to $R^{22}$ have the meaning according to the general formula I, are reacted in solution with compounds of the general formula $XR^{12'}$, wherein X= Cl, Br or I and $R^{12'}$ $COR^{22}$, a $C_{1-10}$-alkyl, an aryl or a heteroaryl radical or an aryl or heteroaryl radical bonded via a $C_{1-6}$-alkylene group, in the presence of a base at a temperature of preferably 10 to 150°C and the 1- and 2-naphthol Mannich bases of the general formula I obtained in this way, in which the radical $R^{12}$ represents $COR^{22}$, a $C_{1-10}$-alkyl, an aryl or a heteroaryl radical or an aryl or heteroaryl radical bonded via a $C_{1-6}$-alkylene group and the radicals $R^1$ to $R^{11}$, $R^{13}$ to $R^{18}$ and $R^{20}$ to $R^{22}$ have the meaning according to the general formula I, are purified by filtration and are isolated by conventional methods.

**48.** Process according to claim 47, **characterized in that** the reaction with the compounds of the general formula $XR^{12'}$ is carried out in dimethylformamide.

**49.** Process according to claim 47 or 48, **characterized in that** X = Cl.

**50.** Process according to one of claims 47 to 49, **characterized in that** the reaction with the compounds of the general formula $XR^{12'}$ is carried out in the presence of triethylamine or potassium tert-butylate as the base.

**51.** Process according to one of claims 47 to 50, **characterized in that** the compounds of the general formula I in which $R^{12} \neq H$, are purified by filtration over a scavenger resin, preferably by filtration over polymer-bonded tris (2-aminoethyl)amine and/or 3-(3-mercaptophenyl)propane-amidomethylpolystyrene.

**52.** Process according to one of claims 46 to 51, **characterized in that** the aromatic aldehyde compounds and/or heteroaromatic aldehyde compounds and/or aliphatic aldehyde compounds of the general formula II are reacted in an organic solvent, preferably in toluene, with secondary amines of the general formula III.

**53.** Process according to one of claims 46 to 52, **characterized in that** the aromatic aldehyde compounds and/or heteroaromatic aldehyde compounds and/or aliphatic aldehyde compounds of the general formula II are reacted in the presence of potassium carbonate or boric acid anhydride as the base.

**54.** Process according to one of claims 46 to 53, **characterized in that** the aminal compounds of the general formula IV are reacted with acetyl chloride to give iminium salts of the general formula V.

**55.** Process according to one of claims 46 to 54, **characterized in that** the aminal compounds of the general formula IV are reacted in absolute diethyl ether to give iminium salts of the general formula V.

**56.** Medicaments comprising, as the active compound, at least one substituted 1- and/or 2-naphthol Mannich base of the general formula I

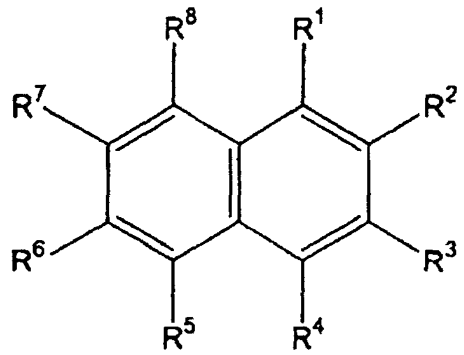

I

wherein

$R^1 = CH(R^9)N(R^{10})(R^{11})$ and $R^2 = OR^{12}$

or

$R^1 = OR^{12}$ and $R^2 = CH(R^9)N(R^{10})(R^{11})$,

and in each case the radicals

$R^3$ to $R^8$ are identical or different and = H, F, Cl, Br, $CF_3$, CN, $NO_2$, $SO_2NH_2$, $SO_2NHR^{13}$, $NHR^{13}$, $SR^{15}$, $OR^{16}$, CO $(OR^{20})$, $CH_2CO(OR^{21})$, $CO(R^{22})$, a $C_{1-10}$-alkyl, an aryl or a heteroaryl radical or an aryl or heteroaryl radical bonded via a $C_{1-6}$-alkylene group, preferably = H, F, Cl, Br, $SO_2NH_2$, $NHR^{13}$, $CO(R^{22})$, $OR^{16}$, $CO(OR^{20})$, a $C_{1-6}$-alkyl radical or an aryl radical bonded via a $C_{1-2}$-alkylene group, particularly preferably H, $NHR^{13}$, $CO(R^{22})$, $OR^{16}$ or $CO(OR^{20})$,

$R^9$ denotes an aryl radical, a heteroaryl radical or an alkyl radical without an acid proton in the $\alpha$-position, preferably an unsubstituted phenyl radical or a phenyl radical which is at least monosubstituted by $C_{1-4}$-alkyl, $C_{1-3}$-alkoxy, halogen, $CF_3$, CN, O-phenyl or OH, particularly preferably an unsubstituted phenyl radical or a 2-methoxy-phenyl, 3-methoxy-phenyl, 4-methoxy-phenyl, 2-methyl-phenyl, 3-methyl-phenyl, 4-methyl-phenyl, 2-tert-butyl-phenyl, 3-tert-butyl-phenyl, 4-tert-butyl-phenyl, 2-fluoro-phenyl, 3-fluoro-phenyl, 4-fluoro-phenyl, 2-chloro-phenyl, 3-chloro-phenyl, 4-chloro-phenyl, 2-bromo-phenyl, 3-bromo-phenyl, 4-bromo-phenyl, 5-bromo-2-fluoro-phenyl, 2-chloro-4-fluoro-phenyl, 2-chloro-5-fluoro-phenyl, 2-chloro-6-fluoro-phenyl, 4-bromo-2-fluoro-phenyl, 3-bromo-4-fluoro-phenyl, 3-bromo-2-fluoro-phenyl, 2,3-dichloro-phenyl, 2,4-dichloro-phenyl, 2,5-dichlorophenyl, 3,4-dichlorophenyl, 2,3-dimethyl-phenyl, 2,4-dimethyl-phenyl, 2,5-dimethylphenyl, 2,3-dimethoxy-phenyl, 2,4-dimethoxy-phenyl, 2,5-dimethoxy-phenyl, 3,4-dimethoxy-phenyl, 3,4,5-trimethoxy-phenyl, 2-trifluoromethyl-phenyl, 3-trifluoromethyl-phenyl or 4-trifluoromethyl-phenyl radical, very particularly preferably an unsubstituted phenyl radical,

$R^{10}$, $R^{11}$ are identical or different and denote a branched or unbranched, saturated or unsaturated, unsubstituted or at least monosubstituted $C_{1-6}$-alkyl radical or an unsubstituted or at least monosubstituted phenyl, benzyl or phenethyl radical, preferably a saturated, unsubstituted or at least monosubstituted $C_{1-6}$-alkyl radical, particularly preferably a $CH_3$ radical,

or $R^{10}$ and $R^{11}$ together denote $(CH_2)_n$, where n = an integer from 3 to 6, or $(CH_2)_2O(CH_2)_2$, preferably $(CH_2)_n$, where n = 4 or 5,

$R^{12}$ = H, $COR^{22}$, a $C_{1-10}$-alkyl, an aryl or a heteroaryl radical or an aryl or heteroaryl radical bonded via a $C_{1-6}$-alkylene group, preferably = H, a $C_{1-6}$-alkyl radical or an aryl radical bonded via a $C_{1-2}$-alkylene group,

$R^{13}$ = H, $COR^{14}$, a $C_{1-10}$-alkyl, an aryl or a heteroaryl radical or an aryl or heteroaryl radical bonded via a $C_{1-6}$-alkylene group, preferably = H, a $C_{1-6}$-alkyl radical or an aryl radical bonded via a $C_{1-2}$-alkylene group, particularly preferably = H,

$R^{14}$ = H, a $C_{1-10}$-alkyl, an aryl or a heteroaryl radical or an aryl or heteroaryl radical bonded via a $C_{1-6}$-alkylene group, preferably a $C_{1-6}$-alkyl radical or an aryl radical bonded via a $C_{1-2}$-alkylene group,

$R^{15}$ = H, a $C_{1-10}$-alkyl, an aryl or a heteroaryl radical or an aryl or heteroaryl radical bonded via a $C_{1-6}$-alkylene group, preferably a $C_{1-6}$-alkyl radical or an aryl radical bonded via a $C_{1-2}$-alkylene group,

$R^{16}$ = H, $CO(R^{17})$, a $C_{1-10}$-alkyl, an aryl or a heteroaryl radical or an aryl or heteroaryl radical bonded via a $C_{1-6}$-alkylene group, preferably H, a $C_{1-6}$-alkyl radical, an aryl radical bonded via a $C_{1-2}$-alkylene group or $CO(R^{17})$, particularly preferably H or $CO(R^{17})$,

$R^{17}$ = H, a $C_{1-10}$-alkyl, an aryl or a heteroaryl radical or an aryl or heteroaryl radical bonded via a $C_{1-6}$-alkylene

group, preferably a $C_{1-6}$-alkyl radical, an aryl radical bonded via a $C_{1-2}$-alkylene group or a phenyl radical which is optionally substituted by F, Cl, Br, $C_{1-4}$-alkyl or $C_{1-3}$-alkoxy, particularly preferably a phenyl radical which is optionally substituted by F, Cl, Br, $C_{1-4}$-alkyl or $C_{1-3}$-alkoxy,

$R^{18}$ = H, a $C_{1-10}$-alkyl, an aryl or a heteroaryl radical or an aryl or heteroaryl radical bonded via a $C_{1-6}$-alkylene group, preferably a $C_{1-6}$-alkyl radical, an aryl radical bonded via a $C_{1-2}$-alkylene group or a phenyl or naphthyl radical which is optionally substituted by F, Cl, Br, $C_{1-4}$-alkyl or $C_{1-3}$-alkoxy, particularly preferably a phenyl radical which is optionally substituted by F, Cl, Br, $C_{1-4}$-alkyl or $C_{1-3}$-alkoxy,

$R^{20}$ = H, a $C_{1-10}$-alkyl, an aryl or a heteroaryl radical or an aryl or heteroaryl radical bonded via a $C_{1-6}$-alkylene group, preferably H, a $C_{1-6}$-alkyl radical, an aryl radical bonded via a $C_{1-2}$-alkylene group or a phenyl radical which is optionally substituted by F, Cl, Br, $C_{1-4}$-alkyl or $C_{1-3}$-alkoxy, particularly preferably H or a phenyl radical which is optionally substituted by F, Cl, Br, $C_{1-4}$-alkyl or $C_{1-3}$-alkoxy,

$R^{21}$ = H, a $C_{1-10}$-alkyl, an aryl or a heteroaryl radical or an aryl or heteroaryl radical bonded via a $C_{1-6}$-alkylene group, preferably = H, a $C_{1-6}$-alkyl radical or an aryl radical bonded via a $C_{1-2}$-alkylene group,

$R^{22}$ = H, $NHNH_2$, $NHR^{18}$, a $C_{1-10}$-alkyl, an aryl or a heteroaryl radical or an aryl or heteroaryl radical bonded via a $C_{1-6}$-alkylene group, preferably H, a $C_{1-6}$-alkyl radical, an aryl radical bonded via a $C_{1-2}$-alkylene group, $NHNH_2$, $NHR^{18}$ or a phenyl radical which is optionally substituted by F, Cl, Br, $C_{1-4}$-alkyl or $C_{1-3}$-alkoxy, particularly preferably $NHNH_2$, $NHR^{18}$ or a phenyl radical which is optionally substituted by F, Cl, Br, $C_{1-4}$-alkyl or $C_{1-3}$-alkoxy, very particularly preferably $NHNH_2$ or $NHR^{18}$,

and/or their racemates, enantiomers, diastereomers and/or corresponding bases and/or corresponding salts of physiologically tolerated acids and optionally further active compounds and/or auxiliary substances.

**57.** Medicament according to claim 56, **characterized in that** it comprises as the active compound a mixture of enantiomers of at least one substituted 1-naphthol Mannich base and/or 2-naphthol Mannich base of the general formula I, the mixture containing the enantiomers in non-equimolar amounts.

**58.** Medicament according to claim 57, **characterized in that** the relative proportion of one of the enantiomers of the mixture is 5 to 45 mol%, preferably 10 to 40 mol%, based on the mixture of enantiomers.

**59.** Medicament according to one of claims 56 to 58 for treatment of/combating pain and/or inflammatory reactions and/or allergic reactions and/or drug abuse and/or alcohol abuse and/or diarrhoea and/or gastritis and/or ulcers and/or cardiovascular diseases and/or urinary incontinence and/or depression and/or states of shock and/or migraines and/or narcolepsy and/or excess weight and/or asthma and/or glaucoma and/or hyperkinetic syndrome.

**60.** Use of at least one compound of the general formula I according to claims 1 to 45 for the preparation of a medicament for combating pain.

**61.** Use of at least one compound of the general formula I according to claims 1 to 45 for the preparation of a medicament for treatment of inflammatory reactions.

**62.** Use of at least one compound of the general formula I according to claims 1 to 45 for the preparation of a medicament for treatment of allergic reactions.

**63.** Use of at least one compound of the general formula I according to claims 1 to 45 for the preparation of a medicament for treatment of drug and/or alcohol abuse.

**64.** Use of at least one compound of the general formula I according to claims 1 to 45 for the preparation of a medicament for treatment of diarrhoea.

**65.** Use of at least one compound of the general formula I according to claims 1 to 45 for the preparation of a medicament for treatment of gastritis.

**66.** Use of at least one compound of the general formula I according to claims 1 to 45 for the preparation of a medicament for treatment of ulcers.

**67.** Use of at least one compound of the general formula I according to claims 1 to 45 for the preparation of a medicament for treatment of cardiovascular diseases.

**68.** Use of at least one compound of the general formula I according to claims 1 to 45 for the preparation of a medicament for treatment of urinary incontinence.

**69.** Use of at least one compound of the general formula I according to claims 1 to 45 for the preparation of a medicament for treatment of depression.

**70.** Use of at least one compound of the general formula I according to claims 1 to 45 for the preparation of a medicament for treatment of states of shock.

**71.** Use of at least one compound of the general formula I according to claims 1 to 45 for the preparation of a medicament for treatment of migraines.

**72.** Use of at least one compound of the general formula I according to claims 1 to 45 for the preparation of a medicament for treatment of narcolepsy.

**73.** Use of at least one compound of the general formula I according to claims 1 to 45 for the preparation of a medicament for treatment of excess weight.

**74.** Use of at least one compound of the general formula I according to claims 1 to 45 for the preparation of a medicament for treatment of asthma.

**75.** Use of at least one compound of the general formula I according to claims 1 to 45 for the preparation of a medicament for treatment of glaucoma.

**76.** Use of at least one compound of the general formula I according to claims 1 to 45 for the preparation of a medicament for treatment in cases of hyperkinetic syndrome.

**Revendications**

**1.** Bases de Mannich de type 1- et 2-naphtol substituées de formule général 1

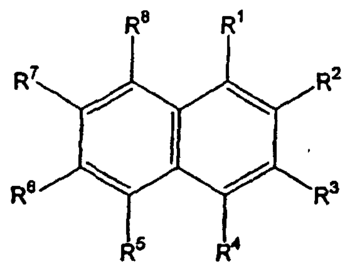

I

dans laquelle
$R^1$ représente $CH(R^9)N(R^{10})$ $(R^{11})$ et $R^2$ représente $OR^{12}$,
ou
$R^1$ représente $OR^{12}$ et $R^2$ représente $CH(R^9)N(R^{10})$ $(R^{11})$,
et les radicaux
$R^3$ à $R^8$, identiques ou différents, représentent chacun H, F, Cl, Br, $CF_3$, CN, $NO_2$, $SO_2NH_2$, $SO_2NHR^{13}$, $NHR^{13}$, $SR^{15}$, $OR^{16}$, $CO(OR^{20})$, $CH_2CO(OR^{21})$, $CO(R^{22})$, un radical alkyle en $C_{1-10}$, un radical aryle, un radical hétéroaryle ou un radical aryle ou hétéroaryle relié par un groupe alkylène en $C_{1-6}$,
$R^9$ représente un radical aryle, un radical hétéroaryle ou un radical alkyle sans proton acide en position $\alpha$,
$R^{10}$, $R^{11}$, identiques ou différents, représentent chacun un radical alkyle en $C_{1-6}$ ramifié, non ramifié, saturé, insaturé, non substitué ou au moins monosubstitué, ou un radical phényle, benzyle ou phénéthyle non substitué ou au moins monosubstitué,
ou $R^{10}$ et $R^{11}$ représentent ensemble $(CH_2)_2O(CH_2)_2$ ou $(CH_2)_n$ où n représente un nombre entier allant de 3 à 6,
$R^{12}$ représente H, $COR^{22}$, un radical alkyle en $C_{1-10}$, un radical aryle, un radical hétéroaryle ou un radical aryle

ou hétéroaryle relié par un groupe alkylène en $C_{1-6}$,
$R^{13}$ représente H, $COR^{14}$, un radical alkyle en $C_{1-10}$, un radical aryle, un radical hétéroaryle ou un radical aryle ou hétéroaryle relié par un groupe alkylène en $C_{1-6}$,
$R^{14}$ représente H, un radical alkyle en $C_{1-10}$, un radical aryle, un radical hétéroaryle ou un radical aryle ou hétéroaryle relié par un groupe alkylène en $C_{1-6}$,
$R^{15}$ représente H, un radical alkyle en $C_{1-10}$, un radical aryle, un radical hétéroaryle ou un radical aryle ou hétéroaryle relié par un groupe alkylène en $C_{1-6}$,
$R^{16}$ représente H, $CO(R^{17})$, un radical alkyle en $C_{1-10}$, un radical aryle, un radical hétéroaryle ou un radical aryle ou hétéroaryle relié par un groupe alkylène en $C_{1-6}$,
$R^{17}$ représente H, un radical alkyle en $C_{1-10}$, un radical aryle, un radical hétéroaryle ou un radical aryle ou hétéroaryle relié par un groupe alkylène en $C_{1-6}$,
$R^{18}$ représente H, un radical alkyle en $C_{1-10}$, un radical aryle, un radical hétéroaryle ou un radical aryle ou hétéroaryle relié par un groupe alkylène en $C_{1-6}$,
$R^{20}$ représente H, un radical alkyle en $C_{1-10}$, un radical aryle, un radical hétéroaryle ou un radical aryle ou hétéroaryle relié par un groupe alkylène en $C_{1-6}$,
$R^{21}$ représente H, un radical alkyle en $C_{1-10}$, un radical aryle, un radical hétéroaryle ou un radical aryle ou hétéroaryle relié par un groupe alkylène en $C_{1-6}$,
$R^{22}$ représente H, $NHNH_2$, $NHR^{18}$, un radical alkyle en $C_{1-10}$, un radical aryle, un radical hétéroaryle ou un radical aryle ou hétéroaryle relié par un groupe alkylène en $C_{1-6}$,
et/ou racémates, énantiomères, diastéréoisomères de tels composés et/ou bases correspondantes et/ou sels correspondants avec des acides physiologiquement acceptables,
à l'exclusion
des racémates des composés dans lesquels les radicaux $R^1$ représentent $CH(R^9)N(R^{10})(R^{11})$ et $R^2$ représente $OR^{12}$, et
les radicaux $R^3$ à $R^8$ et $R^{12}$ représentent chacun H, le radical $R^9$ représente le groupe phényle, 2-chlorophényle, 4-méthoxyphényle, 3-fluorophényle, 3-chlorophényle, 3-bromophényle, 4-bromophényle, 2-fluorophényle, 2-bromophényle, benzo-1,3-dioxole, 4-$CH_3OCO$-phényle ou 2-méthoxyphényle et les radicaux $R^{10}$ et $R^{11}$ représentent ensemble $(CH_2)_5$
ou
les radicaux $R^3$ à $R^8$ et $R^{12}$ représentent H, le radical $R^9$ représente le groupe phényle, 4-méthoxyphényle, 4-diméthylaminophényle, 4-hydroxy-2,3-di-tert-butylphényle, 2,3-dihydrobenzodioxanne, 4-nitrophényle ou benzo-1,3-dioxole et les radicaux $R^{10}$ et $R^{11}$ représentent ensemble $(CH_2)_2O(CH_2)_2$
ou
les radicaux $R^3$ à $R^8$ et $R^{12}$ représentent H, le radical $R^9$ représente le groupe 4-méthoxyphényle et les radicaux $R^{10}$ et $R^{11}$ représentent ensemble $(CH_2)_4$
ou
le radical $R^3$ représente $CO(OR^{20})$, les radicaux $R^4$ à $R^8$ et $R^{12}$ représentent H, le radical $R^9$ représente le groupe phényle, 4-méthoxyphényle, 4-méthylphényle, 4-nitrophényle, p-benzaldéhyde, les radicaux $R^{10}$ et $R^{11}$ représentent ensemble $(CH_2)_5$ et le radical $R^{20}$ représente $CH_3$
ou
les radicaux $R^3$ à $R^8$ et $R^{12}$ représentent H, le radical $R^9$ représente le groupe phényle et les deux radicaux $R^{10}$ et $R^{11}$ représentent chacun $CH_3$, $C_2H_5$ ou n-$C_3H_7$
ou
les radicaux $R^3$ à $R^8$ représentent chacun H, le radical $R^{12}$ représente $CH_3$, le radical $R^9$ représente le groupe phényle et les deux radicaux $R^{10}$ et $R^{11}$ représentent chacun $CH_3$
ou
les radicaux $R^3$ à $R^8$ et $R^{12}$ représentent H, le radical $R^9$ représente le groupe 4-méthoxyphényle ou benzo-1,3-dioxole et les radicaux $R^{10}$ et $R^{11}$ représentent chacun $CH_3$
ou
les radicaux $R^3$ à $R^5$, $R^7$, $R^8$, $R^{12}$ représentent H, le radical $R^6$ représente Br, le radical $R^9$ représente le groupe phényle et les radicaux $R^{10}$ et $R^{11}$ représentent ensemble $(CH_2)_5$
ou
les radicaux $R^3$ à $R^5$, $R^7$, $R^8$, $R^{12}$ représentent H, le radical $R^6$ représente Br, le radical $R^9$ représente le groupe 4-hydroxy-3,5-di-tert-butylphényle et les radicaux $R^{10}$ et $R^{11}$ représentent ensemble $(CH_2)_2O(CH_2)_2$
ou
les radicaux $R^3$ à $R^8$ et $R^{12}$ représentent H, le radical $R^9$ représente le groupe phényle et les radicaux $R^{10}$ et $R^{11}$ représentent chacun $CH_3$, sous forme de chlorhydrate
ou

les radicaux $R^3$ à $R^8$ et $R^{12}$ représentent H, le radical $R^9$ représente le groupe phényle ou 4-méthoxyphényle et les radicaux $R^{10}$ et $R^{11}$ représentent ensemble $(CH_2)_5$, sous forme de chlorhydrate
ou
le radical $R^3$ représente $CO(OR^{20})$, les radicaux $R^4$ à $R^8$ et $R^{12}$ représentent H, le radical $R^9$ représente le groupe phényle, les radicaux $R^{10}$ et $R^{11}$ représentent ensemble $(CH_2)_5$ et le radical $R^{20}$ représente $CH_3$, sous forme de chlorhydrate
ou
les radicaux $R^3$ à $R^8$ et $R^{12}$ représentent H, le radical $R^9$ représente le groupe thiophène et les radicaux $R^{10}$ et $R^{11}$ représentent ensemble $(CH_2)_2O(CH_2)_2$
ou
les radicaux $R^3$ à $R^8$ représentent H, le radical $R^{12}$ représente $CH_3$, le radical $R^9$ représente le groupe thiophène, 4-méthoxyphényle ou 3,4-diméthoxyphényle et les radicaux $R^{10}$ et $R^{11}$ représentent ensemble $(CH_2)_2O(CH_2)_2$
et des énantiomères du composé de formule générale I dans lequel $R^1$ est $CH(R^9)N(R^{10})(R^{11})$ et $R^2$ est $OR^{12}$ et les radicaux $R^3$ à $R^8$ et $R^{12}$ représentent H, $R^9$ représente le groupe phényle, $R^{10}$ et $R^{11}$ représentent ensemble $(CH_2)_5$,
du (+)-1-(α-N,N-diméthylaminobenzyl)-2-naphtol ainsi que du tartrate correspondant,
du (-)-1-(α-N,N-diméthylaminobenzyl)-2-naphtol ainsi que du tartrate correspondant, et
de la (-)-[(2-méthoxynapht-1-yl)benzyl]diméthylamine
ainsi que
des racémates des composés dans lesquels $R^1$ est $OR^{12}$ et $R^2$ est $CH(R^9)N(R^{10})$ $(R^{11})$ et les radicaux $R^3$ à $R^8$ et $R^{12}$ représentent H, le radical $R^9$ représente le groupe phényle, 2-bromophényle, 3-bromophényle ou 4-bromophényle et les radicaux $R^{10}$ et $R^{11}$ représentent ensemble $(CH_2)_5$
ou
$R^3$ à $R^8$ et $R^{12}$ représentent H, le radical $R^9$ représente le groupe phényle ou 2-nitrophényle et les radicaux $R^{10}$ et $R^{11}$ représentent ensemble $(CH_2)_2O$ $(CH_2)_2$
ou
$R^3$, $R^4$, $R^6$, $R^8$ et $R^{12}$ représentent H, les radicaux $R^5$, $R^7$ représentent $CH_3$, le radical $R^9$ représente le groupe phényle ou 4-méthoxyphényle et les radicaux $R^{10}$ et $R^{11}$ représentent ensemble $(CH_2)_5$
ou
$R^3$ à $R^6$, $R^8$, $R^{12}$ représentent H, le radical $R^7$ représente $CH_3$, le radical $R^9$ représente le groupe 4-méthoxyphényle ou phényle et les radicaux $R^{10}$, $R^{11}$ représentent ensemble $(CH_2)_5$
ou
$R^3$ à $R^8$ et $R^{12}$ représentent H, le radical $R^9$ représente le groupe phényle, le radical $R^{10}$ représente $CH_3$ et le radical $R^{11}$ représente $C_6H_{11}$ ou les radicaux $R^{10}$ et $R^{11}$ représentent chacun $CH_3$
ou
$R^3$ à $R^6$, $R^8$, $R^{12}$ représentent H, le radical $R^7$ représente $CH_3$, le radical $R^9$ représente le groupe phényle ou 4-méthoxyphényle et les radicaux $R^{10}$ et $R^{11}$ représentent ensemble $(CH_2)O(CH_2)_2$
ou
$R^3$, $R^4$, $R^6$, $R^8$, $R^{12}$ représentent H, les radicaux $R^5$ et $R^7$ représentent $CH_3$, le radical $R^9$ représente le groupe 4-méthoxyphényle et les radicaux $R^{10}$ et $R^{11}$ représentent ensemble $(CH_2)_2O(CH_2)_2$
ou
$R^3$ à $R^8$ et $R^{12}$ représentent H, le radical $R^9$ représente le groupe phényle et les radicaux $R^{10}$ et $R^{11}$ représentent ensemble $(CH_2)_2O(CH_2)_2$, sous forme de chlorhydrate.

2. Bases de Mannich de type 1- et 2-naphtol substituées selon la revendication 1, **caractérisées en ce qu'**au moins l'un des radicaux $R^3$, $R^4$, $R^5$, $R^6$, $R^7$ ou $R^8$ représente H et les autres radicaux $R^3$, $R^4$, $R^5$, $R^6$, $R^7$ ou $R^8$, $R^9$ à $R^{18}$ et $R^{20}$ à $R^{22}$ ont les significations selon la revendication 1.

3. Bases de Mannich de type 1- et 2-naphtol substituées selon la revendication 1, **caractérisées en ce qu'**au moins l'un des radicaux $R^3$, $R^4$, $R^5$, $R^6$, $R^7$ ou $R^8$ représente un radical alkyle en $C_{1-6}$ et les autres radicaux $R^3$, $R^4$, $R^5$, $R^6$, $R^7$ ou $R^8$, $R^9$ à $R^{18}$ et $R^{20}$ à $R^{22}$ ont les significations selon la revendication 1.

4. Bases de Mannich de type 1- et 2-naphtol substituées selon la revendication 1, **caractérisées en ce qu'**au moins l'un des radicaux $R^3$, $R^4$, $R^5$, $R^6$, $R^7$ ou $R^8$ représente un radical aryle relié par un groupe alkylène en $C_{1-2}$ et les autres radicaux $R^3$, $R^4$, $R^5$, $R^6$, $R^7$ ou $R^8$, $R^9$ à $R^{18}$ et $R^{20}$ à $R^{22}$ ont les significations selon la revendication 1.

5. Bases de Mannich de type 1- et 2-naphtol substituées selon la revendication 1, **caractérisées en ce qu'**au moins l'un des radicaux $R^3$, $R^4$, $R^5$, $R^6$, $R^7$ ou $R^8$ représente F, Cl ou Br et les autres radicaux $R^3$, $R^4$, $R^5$, $R^6$, $R^7$ ou $R^8$,

$R^9$ à $R^{18}$ et $R^{20}$ à $R^{22}$ ont les significations selon la revendication 1.

6. Bases de Mannich de type 1- et 2-naphtol substituées selon la revendication 1, **caractérisées en ce qu'**au moins l'un des radicaux $R^3$, $R^4$, $R^5$, $R^6$, $R^7$ ou $R^8$ représente $SO_2NH_2$ et les autres radicaux $R^3$, $R^4$, $R^5$, $R^6$, $R^7$ ou $R^8$, $R^9$ à $R^{18}$ et $R^{20}$ à $R^{22}$ ont les significations selon la revendication 1.

7. Bases de Mannich de type 1- et 2-naphtol substituées selon la revendication 1, **caractérisées en ce qu'**au moins l'un des radicaux $R^3$, $R^4$, $R^5$, $R^6$, $R^7$ ou $R^8$ représente $NHR^{13}$ et les autres radicaux $R^3$, $R^4$, $R^5$, $R^6$, $R^7$ ou $R^8$, $R^9$ à $R^{18}$ et $R^{20}$ à $R^{22}$ ont les significations selon la revendication 1.

8. Bases de Mannich de type 1- et 2-naphtol substituées selon la revendication 1, **caractérisées en ce qu'**au moins l'un des radicaux $R^3$, $R^4$, $R^5$, $R^6$, $R^7$ ou $R^8$ représente $CO(R^{22})$ et les autres radicaux $R^3$, $R^4$, $R^5$, $R^6$, $R^7$ ou $R^8$, $R^9$ à $R^{18}$ et $R^{20}$ à $R^{22}$ ont les significations selon la revendication 1.

9. Bases de Mannich de type 1- et 2-naphtol substituées selon la revendication 1, **caractérisées en ce qu'**au moins l'un des radicaux $R^3$, $R^4$, $R^5$, $R^6$, $R^7$ ou $R^8$ représente $OR^{16}$ et les autres radicaux $R^3$, $R^4$, $R^5$, $R^6$, $R^7$ ou $R^8$, $R^9$ à $R^{18}$ et $R^{20}$ à $R^{22}$ ont les significations selon la revendication 1.

10. Bases de Mannich de type 1- et 2-naphtol substituées selon la revendication 1, **caractérisées en ce qu'**au moins l'un des radicaux R3, $R^4$, $R^5$, $R^6$, $R^7$ ou $R^8$ représente $CO(OR^{20})$ et les autres radicaux $R^3$, $R^4$, $R^5$, $R^6$, $R^7$ ou $R^8$, $R^9$ à $R^{18}$ et $R^{20}$ à $R^{22}$ ont les significations selon la revendication 1.

11. Bases de Mannich de type 1- et 2-naphtol substituées selon l'une quelconque des revendications 1 à 10, **caractérisées en ce que** le radical $R^9$ représente un radical phényle non substitué ou un radical phényle au moins monosubstitué par un atome d'halogène ou par un groupe alkyle en $C_{1-4}$, alcoxy en $C_{1-3}$, $CF_3$, CN, O-phényle ou OH, de préférence un radical phényle non substitué ou un radical 2-méthoxyphényle, 3-méthoxyphényle, 4-méthoxyphényle, 2-méthylphényle, 3-méthylphényle, 4-méthylphényle, 2-tert-butylphényle, 3-tert-butylphényle, 4-tert-butylphényle, 2-fluorophényle, 3-fluorophényle, 4-fluorophényle, 2-chlorophényle, 3-chlorophényle, 4-chlorophényle, 2-bromophényle, 3-bromophényle, 4-bromophényle, 5-bromo-2-fluorophényle, 2-chloro-4-fluorophényle, 2-chloro-5-fluorophényle, 2-chloro-6-fluorophényle, 4-bromo-2-fluorophényle, 3-bromo-4-fluorophényle, 3-bromo-2-fluorophényle, 2,3-dichlorophényle, 2,4-dichlorophényle, 2,5-dichlorophényle, 3,4-dichlorophényle, 2,3-diméthylphényle, 2,4-diméthylphényle, 2,5-diméthylphényle, 2,3-diméthoxyphényle, 2,4-diméthoxyphényle, 2,5-diméthoxyphényle, 3,4-diméthoxyphényle, 3,4,5-triméthoxyphényle, 2-trifluorométhylphényle, 3-trifluorométhylphényle ou 4-trifluorométhylphényle, de façon particulièrement préférée un radical phényle non substitué, et les radicaux $R^{10}$ à $R^{18}$ et $R^{20}$ à $R^{22}$ ont les significations selon la revendication 1.

12. Bases de Mannich de type 1- et 2-naphtol substituées selon l'une quelconque des revendications 1 à 11, **caractérisées en ce qu'**au moins l'un des radicaux $R^{10}$ et $R^{11}$ représente un radical alkyle en $C_{1-6}$ saturé, non substitué ou au moins monosubstitué, de préférence un radical $CH_3$, et l'autre radical $R^{10}$ ou $R^{11}$ ainsi que les radicaux $R^{12}$ à $R^{18}$ et $R^{20}$ à $R^{22}$ ont chacun la signification selon la revendication 1.

13. Bases de Mannich de type 1- et 2-naphtol substituées selon l'une quelconque des revendications 1 à 11, **caractérisées en ce que** les radicaux $R^{10}$ et $R^{11}$ représentent ensemble $(CH_2)_n$ où n = 4 ou 5 et les radicaux $R^{12}$ à $R^{18}$ et $R^{20}$ à $R^{22}$ ont les significations selon la revendication 1.

14. Bases de Mannich de type 1- et 2-naphtol substituées selon l'une quelconque des revendications 1 à 13, **caractérisées en ce que** le radical $R^{12}$ représente H et les radicaux $R^{13}$ à $R^{18}$ et $R^{20}$ à $R^{22}$ ont les significations selon la revendication 1.

15. Bases de Mannich de type 1- et 2-naphtol substituées selon l'une quelconque des revendications 1 à 13, **caractérisées en ce que** le radical $R^{12}$ représente un radical alkyle en $C_{1-6}$ et les radicaux $R^{13}$ à $R^{18}$ et $R^{20}$ à $R^{22}$ ont les significations selon la revendication 1.

16. Bases de Mannich de type 1- et 2-naphtol substituées selon l'une quelconque des revendications 1 à 13, **caractérisées en ce que** le radical $R^{12}$ représente un radical aryle relié par un groupe alkylène en $C_{1-2}$ et les radicaux $R^{13}$ à $R^{18}$ et $R^{20}$ à $R^{22}$ ont les significations selon la revendication 1.

17. Bases de Mannich de type 1- et 2-naphtol substituées selon l'une quelconque des revendications 1 à 16, **carac-**

**térisées en ce que** le radical $R^{13}$ représente H et les radicaux $R^{14}$ à $R^{18}$ et $R^{20}$ à $R^{22}$ ont les significations selon la revendication 1.

**18.** Bases de Mannich de type 1- et 2-naphtol substituées selon l'une quelconque des revendications 1 à 16, **caractérisées en ce que** le radical $R^{13}$ représente un radical alkyle en $C_{1-6}$ et les radicaux $R^{14}$ à $R^{18}$ et $R^{20}$ à $R^{22}$ ont les significations selon la revendication 1.

**19.** Bases de Mannich de type 1- et 2-naphtol substituées selon l'une quelconque des revendications 1 à 16, **caractérisées en ce que** le radical $R^{13}$ représente un radical aryle relié par un groupe alkylène en $C_{1-2}$ et les radicaux $R^{14}$ à $R^{18}$ et $R^{20}$ à $R^{22}$ ont les significations selon la revendication 1.

**20.** Bases de Mannich de type 1- et 2-naphtol substituées selon l'une quelconque des revendications 1 à 19, **caractérisées en ce que** le radical $R^{14}$ représente un radical alkyle en $C_{1-6}$ et les radicaux $R^{15}$ à $R^{18}$ et $R^{20}$ à $R^{22}$ ont les significations selon la revendication 1.

**21.** Bases de Mannich de type 1- et 2-naphtol substituées selon l'une quelconque des revendications 1 à 19, **caractérisées en ce que** le radical $R^{14}$ représente un radical aryle relié par un groupe alkylène en $C_{1-2}$ et les radicaux $R^{15}$ à $R^{18}$ et $R^{20}$ à $R^{22}$ ont les significations selon la revendication 1.

**22.** Bases de Mannich de type 1- et 2-naphtol substituées selon l'une quelconque des revendications 1 à 21, **caractérisées en ce que** le radical $R^{15}$ représente un radical alkyle en $C_{1-6}$ et les radicaux $R^{16}$ à $R^{18}$ et $R^{20}$ à $R^{22}$ ont les significations selon la revendication 1.

**23.** Bases de Mannich de type 1- et 2-naphtol substituées selon l'une quelconque des revendications 1 à 21, **caractérisées en ce que** le radical $R^{15}$ représente un radical aryle relié par un groupe alkylène en $C_{1-2}$ et les radicaux $R^{16}$ à $R^{18}$ et $R^{20}$ à $R^{22}$ ont les significations selon la revendication 1.

**24.** Bases de Mannich de type 1- et 2-naphtol substituées selon l'une quelconque des revendications 1 à 23, **caractérisées en ce que** le radical $R^{16}$ représente un radical alkyle en $C_{1-6}$ et les radicaux $R^{17}$, $R^{18}$ et $R^{20}$ à $R^{22}$ ont les significations selon la revendication 1.

**25.** Bases de Mannich de type 1- et 2-naphtol substituées selon l'une quelconque des revendications 1 à 23, **caractérisées en ce que** le radical $R^{16}$ représente un radical aryle relié par un groupe alkylène en $C_{1-2}$ et les radicaux $R^{17}$, $R^{18}$ et $R^{20}$ à $R^{22}$ ont les significations selon la revendication 1.

**26.** Bases de Mannich de type 1- et 2-naphtol substituées selon l'une quelconque des revendications 1 à 23, **caractérisées en ce que** le radical $R^{16}$ représente H et les radicaux $R^{17}$, $R^{18}$ et $R^{20}$ à $R^{22}$ ont les significations selon la revendication 1.

**27.** Bases de Mannich de type 1- et 2-naphtol substituées selon l'une quelconque des revendications 1 à 23, **caractérisées en ce que** le radical $R^{16}$ représente $CO(R^{17})$ et les radicaux $R^{17}$, $R^{18}$ et $R^{20}$ à $R^{22}$ ont les significations selon la revendication 1.

**28.** Bases de Mannich de type 1- et 2-naphtol substituées selon l'une quelconque des revendications 1 à 27, **caractérisées en ce que** le radical $R^{17}$ représente un radical alkyle en $C_{1-6}$ et les radicaux $R^{18}$ et $R^{20}$ à $R^{22}$ ont les significations selon la revendication 1.

**29.** Bases de Mannich de type 1- et 2-naphtol substituées selon l'une quelconque des revendications 1 à 27, **caractérisées en ce que** le radical $R^{17}$ représente un radical aryle relié par un groupe alkylène en $C_{1-2}$ et les radicaux $R^{18}$ et $R^{20}$ à $R^{22}$ ont les significations selon la revendication 1.

**30.** Bases de Mannich de type 1- et 2-naphtol substituées selon l'une quelconque des revendications 1 à 27, **caractérisées en ce que** le radical $R^{17}$ représente un radical phényle éventuellement substitué par F, Cl, Br, alkyle en $C_{1-4}$, alcoxy en $C_{1-3}$ et les radicaux $R^{18}$ et $R^{20}$ à $R^{22}$ ont les significations selon la revendication 1.

**31.** Bases de Mannich de type 1- et 2-naphtol substituées selon l'une quelconque des revendications 1 à 30, **caractérisées en ce que** le radical $R^{18}$ représente un radical alkyle en $C_{1-6}$ et les radicaux $R^{20}$ à $R^{22}$ ont les significations selon la revendication 1.

**32.** Bases de Mannich de type 1- et 2-naphtol substituées selon l'une quelconque des revendications 1 à 30, **caractérisées en ce que** le radical $R^{18}$ représente un radical aryle relié par un groupe alkylène en $C_{1-2}$ et les radicaux $R^{20}$ à $R^{22}$ ont les significations selon la revendication 1.

**33.** Bases de Mannich de type 1- et 2-naphtol substituées selon l'une quelconque des revendications 1 à 30, **caractérisées en ce que** le radical $R^{18}$ représente un radical phényle ou naphtyle éventuellement substitué par F, Cl, Br, alkyle en $C_{1-4}$ ou alcoxy en $C_{1-3}$, de préférence un radical phényle éventuellement substitué par F, Cl, Br, alkyle en $C_{1-4}$, alcoxy en $C_{1-3}$ et les radicaux $R^{20}$ à $R^{22}$ ont les significations selon la revendication 1.

**34.** Bases de Mannich de type 1- et 2-naphtol substituées selon l'une quelconque des revendications 1 à 33, **caractérisées en ce que** le radical $R^{20}$ représente un radical alkyle en $C_{1-6}$ et les radicaux $R^{21}$ et $R^{22}$ ont les significations selon la revendication 1.

**35.** Bases de Mannich de type 1- et 2-naphtol substituées selon l'une quelconque des revendications 1 à 33, **caractérisées en ce que** le radical $R^{20}$ représente un radical aryle relié par un groupe alkylène en $C_{1-2}$ et les radicaux $R^{21}$ et $R^{22}$ ont les significations selon la revendication 1.

**36.** Bases de Mannich de type 1- et 2-naphtol substituées selon l'une quelconque des revendications 1 à 33, **caractérisées en ce que** le radical $R^{20}$ représente H et les radicaux $R^{21}$ et $R^{22}$ ont les significations selon la revendication 1.

**37.** Bases de Mannich de type 1- et 2-naphtol substituées selon l'une quelconque des revendications 1 à 33, **caractérisées en ce que** le radical $R^{20}$ représente un radical phényle éventuellement substitué par F, Cl, Br, alkyle en $C_{1-4}$ ou alcoxy en $C_{1-3}$ et les radicaux $R^{21}$ et $R^{22}$ ont les significations selon la revendication 1.

**38.** Bases de Mannich de type 1- et 2-naphtol substituées selon l'une quelconque des revendications 1 à 37, **caractérisées en ce que** le radical $R^{21}$ représente H et le radical $R^{22}$ a la signification selon la revendication 1.

**39.** Bases de Mannich de type 1- et 2-naphtol substituées selon l'une quelconque des revendications 1 à 37, **caractérisées en ce que** le radical $R^{21}$ représente alkyle en $C_{1-6}$ et le radical $R^{22}$ a la signification selon la revendication 1.

**40.** Bases de Mannich de type 1- et 2-naphtol substituées selon l'une quelconque des revendications 1 à 37, **caractérisées en ce que** le radical $R^{21}$ représente un radical aryle relié par un groupe alkylène en $C_{1-2}$ et le radical $R^{22}$ a la signification selon la revendication 1.

**41.** Bases de Mannich de type 1- et 2-naphtol substituées selon l'une quelconque des revendications 1 à 40, **caractérisées en ce que** le radical $R^{22}$ représente H.

**42.** Bases de Mannich de type 1- et 2-naphtol substituées selon l'une quelconque des revendications 1 à 40, **caractérisées en ce que** le radical $R^{22}$ représente un radical alkyle en $C_{1-6}$.

**43.** Bases de Mannich de type 1- et 2-naphtol substituées selon l'une quelconque des revendications 1 à 40, **caractérisées en ce que** le radical $R^{22}$ représente un radical aryle relié par un groupe alkylène en $C_{1-2}$.

**44.** Bases de Mannich de type 1- et 2-naphtol substituées selon l'une quelconque des revendications 1 à 40, **caractérisées en ce que** le radical $R^{22}$ représente $NHNH_2$, $NHR^{18}$ ou un radical phényle éventuellement substitué par F, Cl, Br, alkyle en $C_{1-4}$ ou alcoxy en $C_{1-3}$, de préférence représente $NHNH_2$ ou $NHR^{18}$.

**45.** Bases de Mannich de type 1- et 2-naphtol substituées selon l'une quelconque des revendications 1 :
6-(diméthylaminophénylméthyl)-5-hydroxynaphtalène-1-sulfonamide
4-amino-2-(diméthylaminophénylméthyl)naphtalén-1-ol
4-(diméthylaminophénylméthyl)-3-hydroxynaphtalène-2-carbohydrazide
4-(diméthylaminophénylméthyl)-3-hydroxynaphtalène-2-carboxylate de méthyle
acide 4-(diméthylaminophénylméthyl)-3-hydroxynaphtalène-2-carboxylique
4-(diméthylaminophénylméthyl)-3-hydroxynaphtalène-2-carboxylate de phényle
[5-(diméthylaminophénylméthyl)-6-hydroxynaphtalén-2-yl]phénylméthanone
3-amino-1-(diméthylaminophénylméthyl)naphtalén-2-ol

4-(diméthylaminophénylméthyl)-3-hydroxynaphtalène-2-(2-méthoxyphényl)carboxamide
4-(diméthylaminophénylméthyl)-3-hydroxynaphtalène-2-*o*-tolylcarboxamide
4-(diméthylaminophénylméthyl)-3-hydroxynaphtalène-2-naphtalén-1-ylcarboxamide
acide 4-(diméthylaminophénylméthyl)-3-hydroxy-7-méthoxynaphtalène-2-carboxylique
acide 5-(diméthylaminophénylméthyl)-6-hydroxynaphtalène-2-carboxylique
1-(diméthylaminophénylméthyl)-7-méthoxynaphtalén-2-ol
1-(diméthylaminophénylméthyl)-6-méthoxynaphtalén-2-ol
acide 5-(diméthylaminophénylméthyl)-6-hydroxynaphtalène-1-carboxylique
4-(diméthylaminophénylméthyl)-3-hydroxy-7-méthoxynaphtalène-2-carboxylate de sodium
4-chloro-2-(morpholin-4-yl-*o*-tolylméthyl)naphtalén-1-ol
4-chloro-2-(pipéridin-1-yl-*o*-tolylméthyl)naphtalén1-ol
4-chloro-2-[(2-chlorophényl)pipéridin-1-ylméthyl]naphtalén-1-ol
4-chloro-2-[(2,3-diméthoxyphényl)morpholin-4-yl-méthyl]naphtalén-1-ol
5-amino-2-[(2-chlorophényl)pipéridin-1-ylméthyl]naphtalén-1-ol
5-amino-2-[(2,3-diméthoxyphényl)morpholin-4-yl-méthyl]naphtalén-1-ol
3-hydroxy-4-(pipéridin-1-yl-*o*-tolylméthyl)naphtalène-2-carbohydrazide
7-méthoxy-1-(morpholin-4-yl-*o*-tolylméthyl)naphtalén-2-ol
1-[(2-chlorophényl)pipéridin-1-ylméthyl]-7-méthoxynaphtalén-2-ol
1-[(2,3-diméthoxyphényl)morpholin-4-ylméthyl]-7-méthoxynaphtalén-2-ol
6-bromo-1-[(2-méthoxyphényl)morpholin-4-ylméthyl]naphtalén-2-ol
acide 6-hydroxy-5-[(2-méthoxyphényl)morpholin-4-yl-méthyl]naphtalène-1-carboxylique
7-méthoxy-1-[(2-méthoxyphényl)morpholin-4-yl-méthyl]naphtalén-2-ol
6-méthoxy-1-[(2-méthoxyphényl)morpholin-4-yl-méthyl)naphtalén-2-ol
4-chloro-2-[(2-méthoxyphényl)pipéridin-1-ylméthyl]naphtalén-1-ol
6-bromo-1-[(2-méthoxyphényl)pipéridin-1-ylméthyl]naphtalén-2-ol
6-méthoxy-1-[(2-méthoxyphényl)pipéridin-1-yl-méthyl]naphtalén-2-ol
7-méthoxy-1-[(2-méthoxyphényl)pipéridin-1-yl-méthyl]naphtalén-2-ol
5-chloro-2-[diméthylamino-(2-méthoxyphényl)méthyl]naphtalén-1-ol
{[1-(4-méthoxybenzyloxy)naphtalén-2-yl]phénylméthyl}diméthylamine
{[2-(4-méthoxybenzyloxy)naphtalén-1-yl]phénylméthyl}diméthylamine
4-méthoxybenzoate de 1-(diméthylaminophénylméthyl)naphtalén-2-yle
2-chlorobenzoate de 1-(diméthylaminophénylméthyl)naphtalén-2-yle
1-(morpholin-4-ylphénylméthyl)naphtalén-2-ol
1-(phénylpipéridin-1-ylméthyl)naphtalén-2-ol
2-[(4-fluorophényl)pyrrolidin-1-ylméthyl]naphtalén-1-ol.

**46.** Procédé pour la préparation de bases de Mannich de type 1- et 2-naphtol substituées de formule générale I selon l'une quelconque des revendications 1 à 45, dans lesquelles le radical $R^{12}$ représente H et les radicaux $R^1$ à $R^{11}$, $R^{13}$ à $R^{18}$ et $R^{20}$ à $R^{22}$ ont les significations selon la formule générale I, **caractérisé en ce qu'**on fait réagir des aldéhydes aromatiques et/ou des aldéhydes hétéroaromatiques et/ou des aldéhydes aliphatiques de formule générale II

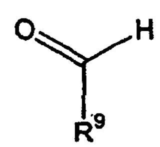

II

dans laquelle $R^9$ a la signification selon la formule générale I, en solution, en présence d'une base, de préférence à une température de -10°C à +110°C, avec des amines secondaires de formule générale III

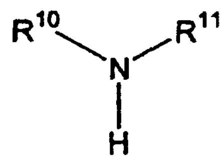

III

dans laquelle $R^{10}$ et $R^{11}$ ont les significations selon la formule générale I, pour obtenir des aminals de formule générale IV

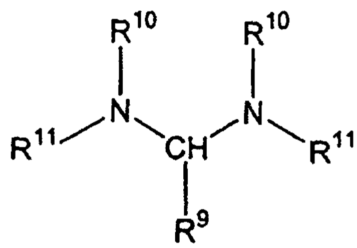

IV

et on fait réagir ces aminals de formule générale IV, sans les purifier davantage, avec un chlorure d'acide dans un solvant anhydre, pour obtenir des sels d'iminium de formule générale V

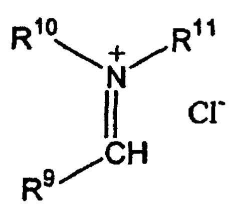

V

et on fait réagir ces sels d'iminium de formule générale V, sans les purifier davantage, en solution, de préférence dans de l'acétonitrile, avec des composés 1- et 2-naphtol substitués et/ou non substitués de formule générale VI,

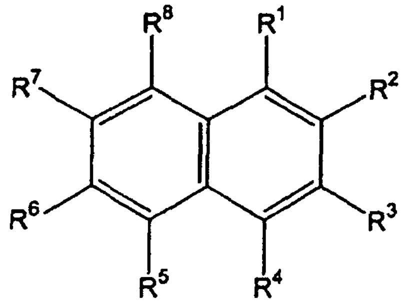

VI

dans laquelle $R^1$ = H et $R^2$ = OH ou $R^1$ = OH et $R^2$ = H, et les autres radicaux $R^3$ à $R^8$, $R^{13}$ à $R^{18}$ et $R^{20}$ à $R^{22}$ ont chacun la signification selon la formule générale I, et les bases de Mannich de type 1-et 2-naphtol de formule générale I ainsi obtenues, dans lesquelles le radical $R^{12}$ représente H et les radicaux $R^1$ à $R^{11}$, $R^{13}$ à $R^{18}$ et $R^{20}$ à $R^{22}$ ont les significations selon la formule générale I, sont purifiées par extraction et isolées selon les méthodes usuelles.

**47.** Procédé pour la préparation de bases de Mannich de type 1- et 2-naphtol substituées de formule générale I selon l'une quelconque des revendications 1 à 45, dans lesquelles le radical $R^{12}$ représente $COR^{22}$, un radical alkyle en $C_{1-10}$, un radical aryle, un radical hétéroaryle ou un radical aryle ou hétéroaryle relié par un groupe alkylène en $C_{1-6}$ et les radicaux $R^1$ à $R^{11}$, $R^{13}$ à $R^{18}$ et $R^{20}$ à $R^{22}$ ont les significations selon la formule générale I, **caractérisé en ce qu'**on fait réagir des aldéhydes aromatiques et/ou des aldéhydes hétéroaromatiques et/ou des aldéhydes aliphatiques de formule générale II

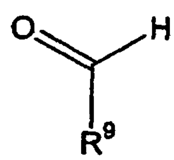

II

dans laquelle $R^9$ a la signification selon la formule générale I, en solution en présence d'une base, de préférence à une température de -10°C à +110°C, avec des amines secondaires de formule générale III

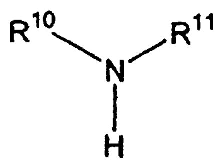

III

dans laquelle $R^{10}$ et $R^{11}$ ont les significations selon la formule générale I,
pour obtenir des aminals de formule générale IV

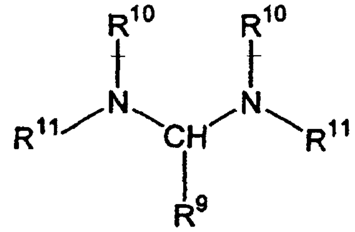

IV

et on fait réagir ces aminals de formule générale IV, sans les purifier davantage, avec un chlorure d'acide dans un solvant anhydre, pour obtenir des sels d'iminium de formule générale V

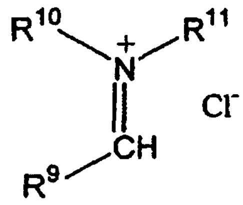

V

et on fait réagir ces sels d'iminium de formule générale V, sans les purifier davantage, en solution, de préférence dans de l'acétonitrile, avec des composés 1- et 2-naphtol substitués et/ou non substitués de formule générale VI,

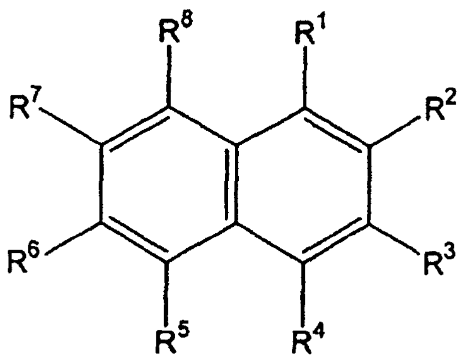

VI

dans laquelle $R^1$ = H et $R^2$ = OH ou $R^1$ = OH et $R^2$ = H, et les autres radicaux $R^3$ à $R^8$, $R^{13}$ à $R^{18}$ et $R^{20}$ à $R^{22}$ ont chacun la signification selon la formule générale I, et on fait réagir les composés ainsi obtenus de formule générale VI, dans laquelle $R^1$ représente $CH(R^9)N(R^{10})$ $(R^{11})$ et $R^2$ = OH ou $R^1$ = OH et $R^2$ représente $CH(R^9)N(R^{10})$ $(R^{11})$ et les radicaux $R^3$ à $R^{11}$, $R^{13}$ à $R^{18}$ et $R^{20}$ à $R^{22}$ ont les significations selon la formule générale I, en solution, avec des composés de formule générale $XR^{12'}$, dans laquelle X = Cl, Br ou I et $R^{12'}$ représente $COR^{22}$, un radical alkyle en $C_{1-10}$, un radical aryle, un radical hétéroaryle ou un radical aryle ou hétéroaryle relié par un groupe alkylène en $C_{1-6}$, en présence d'une base, à une température de préférence de 10 à 150°C, et on purifie par filtration les bases de Mannich de type 1- et 2-naphtol de formule générale I, ainsi obtenues, dans lesquelles le radical $R^{12}$ représente $COR^{22}$, un radical alkyle en $C_{1-10}$, un radical aryle, un radical hétéroaryle ou un radical aryle ou hétéroaryle relié par un groupe alkylène en $C_{1-6}$ et les radicaux $R^1$ à $R^{11}$, $R^{13}$ à $R^{18}$ et $R^{20}$ à $R^{22}$ ont les significations selon la formule générale I, et on les isole selon les méthodes usuelles.

**48.** Procédé selon la revendication 47, **caractérisé en ce que** la réaction avec les composés de formule générale $XR^{12'}$ s'effectue dans du diméthylformamide.

**49.** Procédé selon la revendication 47 ou 48, **caractérisé en ce que** X = Cl.

**50.** Procédé selon l'une quelconque des revendications 47 à 49, **caractérisé en ce que** la réaction avec les composés de formule générale $XR^{12'}$ est effectuée en présence de triéthylamine ou de tert-butylate de potassium en tant que base.

**51.** Procédé selon l'une quelconque des revendications 47 à 50, **caractérisé en ce que** les composés de formule générale I, dans lesquels $R^{12}$ est $\neq$ H, sont purifiés par filtration sur une résine de fixation, de préférence par filtration sur tris(2-aminoéthyl)amine fixée sur polymère et/ou sur 3-(3-mercaptophényl)propionamidométhylpolystyrène.

**52.** Procédé selon l'une quelconque des revendications 46 à 51, **caractérisé en ce que** les aldéhydes aromatiques et/ou les aldéhydes hétéroaromatiques et/ou les aldéhydes aliphatiques de formule générale II sont mis en réaction, dans un solvant organique, de préférence dans du toluène, avec des amines secondaires de formule générale III.

**53.** Procédé selon l'une quelconque des revendications 46 à 52, **caractérisé en ce que** les aldéhydes aromatiques et/ou les aldéhydes hétéroaromatiques et/ou les aldéhydes aliphatiques de formule générale II sont mis en réaction en présence de carbonate de potassium ou d'anhydride borique en tant que base.

**54.** Procédé selon l'une quelconque des revendications 46 à 53, **caractérisé en ce qu'**on fait réagir les aminals de formule générale IV avec du chlorure d'acétyle, pour aboutir aux sels d'iminium de formule générale V.

**55.** Procédé selon l'une quelconque des revendications 46 à 54, **caractérisé en ce qu'**on fait réagir les aminals de formule générale IV dans de l'oxyde d'éthyle anhydre, pour aboutir aux sels d'iminium de formule générale V.

**56.** Médicament contenant en tant que substance active au moins une base de Mannich de type 1- et/ou 2-naphtol substituée de formule générale I

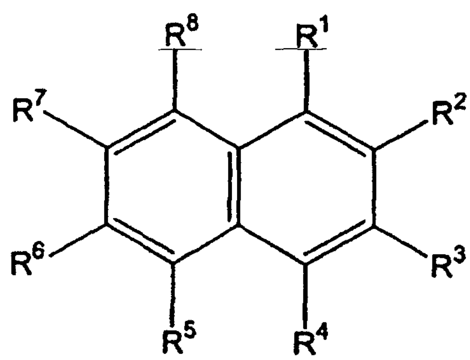

I

dans laquelle
$R^1$ représente $CH(R^9)N(R^{10})(R^{11})$ et $R^2$ représente $OR^{12}$,
ou
$R^1$ représente $OR^{12}$ et $R^2$ représente $CH(R^9)N(R^{10})(R^{11})$,
et les radicaux
$R^3$ à $R^8$, identiques ou différents, représentent chacun H, F, Cl, Br, $CF_3$, CN, $NO_2$, $SO_2NH_2$, $SO_2NHR^{13}$, $NHR^{13}$, $SR^{15}$, $OR^{16}$, $CO(OR^{20})$, $CH_2CO(OR^{21})$, $CO(R^{22})$, un radical alkyle en $C_{1-10}$, un radical aryle, un radical hétéroaryle

ou un radical aryle ou hétéroaryle relié par un groupe alkylène en $C_{1-6}$, de préférence H, F, Cl, Br, $SO_2NH_2$, $NHR^{13}$, $CO(R^{22})$, $OR^{16}$, $CO(OR^{20})$, un radical alkyle en $C_{1-6}$ ou un radical aryle relié par un groupe alkylène en $C_{1-2}$, de façon particulièrement préférée H, $NHR^{13}$, $CO(R^{22})$, $OR^{16}$, $CO(OR^{20})$,

$R^9$ représente un radical aryle, un radical hétéroaryle ou un radical alkyle sans proton acide en position $\alpha$, de préférence un radical phényle non substitué ou un radical phényle au moins monosubstitué par un atome d'halogène ou par un groupe alkyle en $C_{1-4}$, alcoxy en $C_{1-3}$, $CF_3$, CN, O-phényle ou OH, de façon particulièrement préférée un radical phényle non substitué ou un radical 2-méthoxyphényle, 3-méthoxyphényle, 4-méthoxyphényle, 2-méthylphényle, 3-méthylphényle, 4-méthylphényle, 2-tert-butylphényle, 3-tert-butylphényle, 4-tert-butylphényle, 2-fluorophényle, 3-fluorophényle, 4-fluorophényle, 2-chlorophényle, 3-chlorophényle, 4-chlorophényle, 2-bromophényle, 3-bromophényle, 4-bromophényle, 5-bromo-2-fluorophényle, 2-chloro-4-fluorophényle, 2-chloro-5-fluorophényle, 2-chloro-6-fluorophényle, 4-bromo-2-fluorophényle, 3-bromo-4-fluorophényle, 3-bromo-2-fluorophényle, 2,3-dichlorophényle, 2,4-dichlorophényle, 2,5-dichlorophényle, 3,4-dichlorophényle, 2,3-diméthylphényle, 2,4-diméthylphényle, 2,5-diméthylphényle, 2,3-diméthoxyphényle, 2,4-diméthoxyphényle, 2,5-diméthoxyphényle, 3,4-diméthoxyphényle, 3,4,5-triméthoxyphényle, 2-trifluorométhylphényle, 3-trifluorométhylphényle ou 4-trifluorométhylphényle, de façon tout particulièrement préférée un radical phényle non substitué,

$R^{10}$, $R^{11}$, identiques ou différents, représentent un radical alkyle en $C_{1-6}$ ramifié, non ramifié, saturé insaturé, non substitué ou au moins monosubstitué ou un radical phényle, benzyle ou phénéthyle non substitué ou au moins monosubstitué, de préférence un radical alkyle en $C_{1-6}$ saturé, non substitué ou au moins monosubstitué, de façon particulièrement préférée un radical $CH_3$,

ou $R^{10}$ et $R^{11}$ représentent ensemble $(CH_2)_n$ où n représente un nombre entier allant de 3 à 6, ou $(CH_2)_2O(CH_2)_2$, de préférence $(CH_2)_n$ où n = 4 ou 5,

$R^{12}$ représente H, $COR^{22}$, un radical alkyle en $C_{1-10}$, un radical aryle, un radical hétéroaryle ou un radical aryle ou hétéroaryle relié par un groupe alkylène en $C_{1-6}$, de préférence H, un radical alkyle en $C_{1-6}$ ou un radical aryle relié par un groupe alkylène en $C_{1-2}$,

$R^{13}$ représente H, $COR^{14}$, un radical alkyle en $C_{1-10}$, un radical aryle, un radical hétéroaryle ou un radical aryle ou hétéroaryle relié par un groupe alkylène en $C_{1-6}$, de préférence H, un radical alkyle en $C_{1-6}$ ou un radical aryle relié par un groupe alkylène en $C_{1-2}$, de façon particulièrement préférée H, $R^{14}$ représente H, un radical alkyle en $C_{1-10}$, un radical aryle, un radical hétéroaryle ou un radical aryle ou hétéroaryle relié par un groupe alkylène en $C_{1-6}$, de préférence un radical alkyle en $C_{1-6}$ ou un radical aryle relié par un groupe alkylène en $C_{1-2}$,

$R^{15}$ représente H, un radical alkyle en $C_{1-10}$, un radical aryle, un radical hétéroaryle ou un radical aryle ou hétéroaryle relié par un groupe alkylène en $C_{1-6}$, de préférence un radical alkyle en $C_{1-6}$ ou un radical aryle relié par un groupe alkylène en $C_{1-2}$,

$R^{16}$ représente H, $CO(R^{17})$, un radical alkyle en $C_{1-10}$, un radical aryle, un radical hétéroaryle ou un radical aryle ou hétéroaryle relié par un groupe alkylène en $C_{1-6}$, de préférence H, un radical alkyle en $C_{1-6}$, un radical aryle relié par un groupe alkylène en $C_{1-2}$, ou $CO(R^{17})$, de façon particulièrement préférée H ou $CO(R^{17})$,

$R^{17}$ représente H, un radical alkyle en $C_{1-10}$, un radical aryle, un radical hétéroaryle ou un radical aryle ou hétéroaryle relié par un groupe alkylène en $C_{1-6}$, de préférence un radical alkyle en $C_{1-6}$, un radical aryle relié par un groupe alkylène en $C_{1-2}$ ou un radical phényle éventuellement substitué par F, Cl, Br, alkyle en $C_{1-4}$, alcoxy en $C_{1-3}$, de façon particulièrement préférée un radical phényle éventuellement substitué par F, Cl, Br, alkyle en $C_{1-4}$, alcoxy en $C_{1-3}$,

$R^{18}$ représente H, un radical alkyle en $C_{1-10}$, un radical aryle, un radical hétéroaryle ou un radical aryle ou hétéroaryle relié par un groupe alkylène en $C_{1-6}$, de préférence un radical alkyle en $C_{1-6}$, un radical aryle relié par un groupe alkylène en $C_{1-2}$ ou un radical phényle ou naphtyle éventuellement substitué par F, Cl, Br, alkyle en $C_{1-4}$ ou alcoxy en $C_{1-3}$, de façon particulièrement préférée un radical phényle éventuellement substitué par F, Cl, Br, alkyle en $C_{1-4}$, alcoxy en $C_{1-3}$,

$R^{20}$ représente H, un radical alkyle en $C_{1-10}$, un radical aryle, un radical hétéroaryle ou un radical aryle ou hétéroaryle relié par un groupe alkylène en $C_{1-6}$, de préférence H, un radical alkyle en $C_{1-6}$, un radical aryle relié par un groupe alkylène en $C_{1-2}$ ou un radical phényle éventuellement substitué par F, Cl, Br, alkyle en $C_{1-4}$ ou alcoxy en $C_{1-3}$, de façon particulièrement préférée H ou un radical phényle éventuellement substitué par F, Cl, Br, alkyle en $C_{1-4}$, alcoxy en $C_{1-3}$,

$R^{21}$ représente H, un radical alkyle en $C_{1-10}$, un radical aryle, un radical hétéroaryle ou un radical aryle ou hétéroaryle relié par un groupe alkylène en $C_{1-6}$, de préférence H, un radical alkyle en $C_{1-6}$ ou un radical aryle relié par un groupe alkylène en $C_{1-2}$,

$R^{22}$ représente H, $NHNH_2$, $NHR^{18}$, un radical alkyle en $C_{1-10}$, un radical aryle, un radical hétéroaryle ou un radical aryle ou hétéroaryle relié par un groupe alkylène en $C_{1-6}$, de préférence H, un radical alkyle en $C_{1-6}$, un radical aryle relié par un groupe alkylène en $C_{1-2}$, $NHNH_2$, $NHR^{18}$ ou un radical phényle éventuellement substitué par F, Cl, Br, alkyle en $C_{1-4}$ ou alcoxy en $C_{1-3}$, de façon particulièrement préférée $NHNH_2$, $NHR^{18}$ ou un radical phényle éventuellement substitué par F, Cl, Br, alkyle en $C_{1-4}$ ou alcoxy en $C_{1-3}$, de façon tout particulièrement préférée

$NHNH_2$ ou $NHR^{18}$,
et/ou des racémates, énantiomères, diastéréoisomères et/ou bases correspondantes et/ou sels correspondants avec des acides physiologiquement acceptables de tels composés, et éventuellement d'autres substances actives et/ou adjuvants.

**57.** Médicament selon la revendication 56, **caractérisé en ce qu'**il contient en tant que substance active un mélange d'énantiomères d'au moins une base de Mannich de type 1-naphtol substituée et/ou une base de Mannich de type 2-naphtol substituée, de formule générale I, le mélange comportant les énantiomères en quantités non équimolaires.

**58.** Médicament selon la revendication 57, **caractérisé en ce que** la proportion relative de l'un des énantiomères dans le mélange va de 5 à 45 % en moles, de préférence de 10 à 40 % en moles, par rapport au mélange des énantiomères.

**59.** Médicament selon l'une quelconque des revendications 56 à 58, pour traiter/lutter contre des maladies et/ou des réactions inflammatoires et/ou des réactions allergiques et/ou l'abus de drogue et/ou l'abus d'alcool et/ou la diarrhée et/ou la gastrite et/ou des ulcères et/ou des maladies cardiovasculaires et/ou l'incontinence urinaire et/ou des dépressions et/ou des états de choc et/ou la migraine et/ou la narcolepsie et/ou l'excès de poids et/ou l'asthme et/ou le glaucome et/ou le syndrome hyperkinétique.

**60.** Utilisation d'au moins un composé de formule générale I selon les revendications 1 à 45, pour la fabrication d'un médicament destiné à la lutte contre des douleurs.

**61.** Utilisation d'au moins un composé de formule générale I selon les revendications 1 à 45, pour la fabrication d'un médicament destiné au traitement de réactions inflammatoires.

**62.** Utilisation d'au moins un composé de formule générale I selon les revendications 1 à 45, pour la fabrication d'un médicament destiné au traitement de réactions allergiques.

**63.** Utilisation d'au moins un composé de formule générale I selon les revendications 1 à 45, pour la fabrication d'un médicament destiné au traitement de l'abus de drogue et/ou d'alcool.

**64.** Utilisation d'au moins un composé de formule générale I selon les revendications 1 à 45, pour la fabrication d'un médicament destiné au traitement de la diarrhée.

**65.** Utilisation d'au moins un composé de formule générale I selon les revendications 1 à 45, pour la fabrication d'un médicament destiné au traitement de la gastrite.

**66.** Utilisation d'au moins un composé de formule générale I selon les revendications 1 à 45, pour la fabrication d'un médicament destiné au traitement d'ulcères.

**67.** Utilisation d'au moins un composé de formule générale I selon les revendications 1 à 45, pour la fabrication d'un médicament destiné au traitement de maladies cardiovasculaires.

**68.** Utilisation d'au moins un composé de formule générale I selon les revendications 1 à 45, pour la fabrication d'un médicament destiné au traitement de l'incontinence urinaire.

**69.** Utilisation d'au moins un composé de formule générale I selon les revendications 1 à 45, pour la fabrication d'un médicament destiné au traitement de dépressions.

**70.** Utilisation d'au moins un composé de formule générale I selon les revendications 1 à 45, pour la fabrication d'un médicament destiné au traitement d'états de choc.

**71.** Utilisation d'au moins un composé de formule générale I selon les revendications 1 à 45, pour la fabrication d'un médicament destiné au traitement de la migraine.

**72.** Utilisation d'au moins un composé de formule générale I selon les revendications 1 à 45, pour la fabrication d'un médicament destiné au traitement de la narcolepsie.

**73.** Utilisation d'au moins un composé de formule générale I selon les revendications 1 à 45, pour la fabrication d'un médicament destiné au traitement de l'excès de poids.

**74.** Utilisation d'au moins un composé de formule générale I selon les revendications 1 à 45, pour la fabrication d'un médicament destiné au traitement de l'asthme.

**75.** Utilisation d'au moins un composé de formule générale I selon les revendications 1 à 45, pour la fabrication d'un médicament destiné au traitement de glaucomes.

**76.** Utilisation d'au moins un composé de formule générale I selon les revendications 1 à 45, pour la fabrication d'un médicament destiné au traitement du syndrome hyperkinétique.

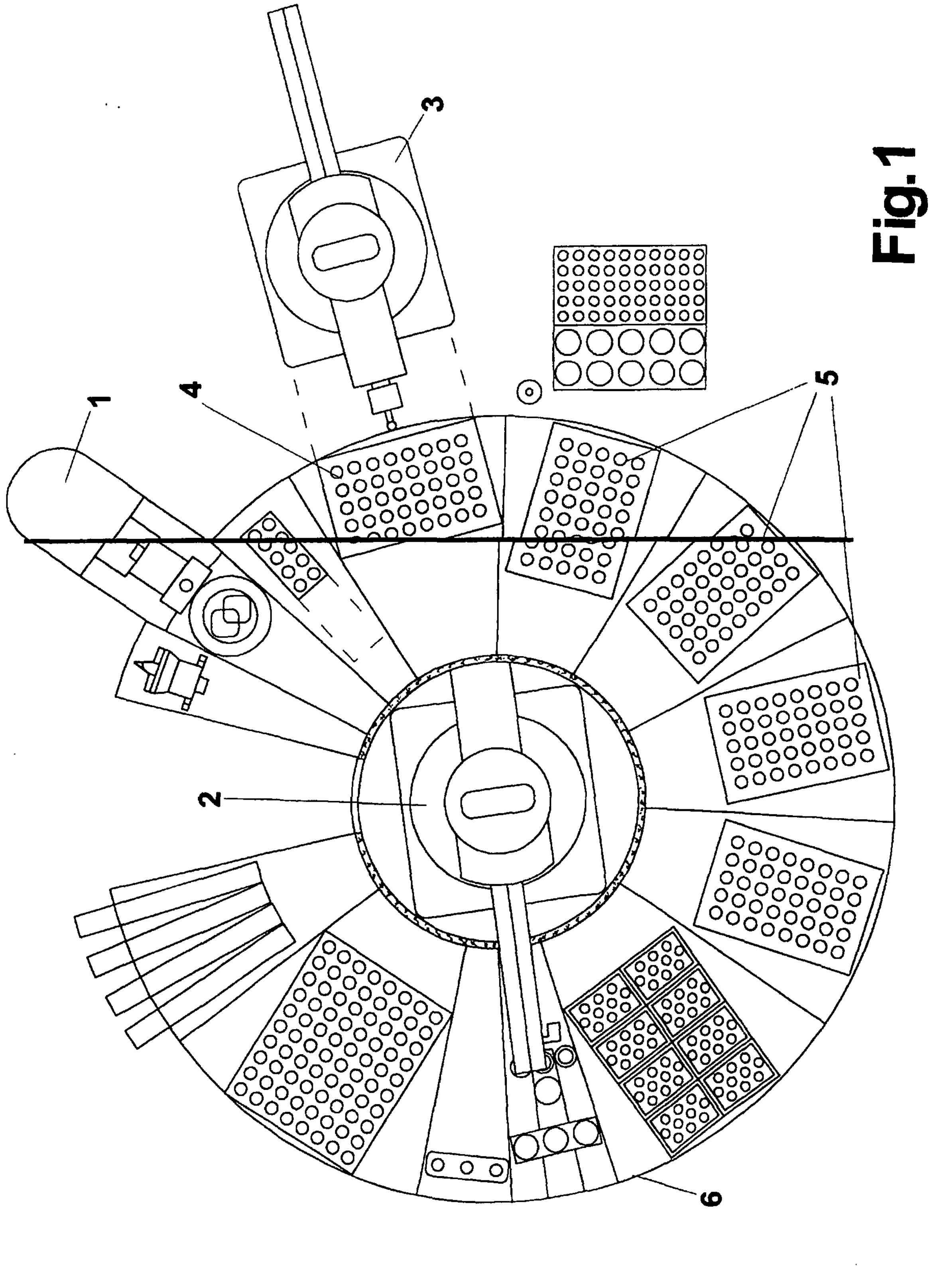


Fig.1

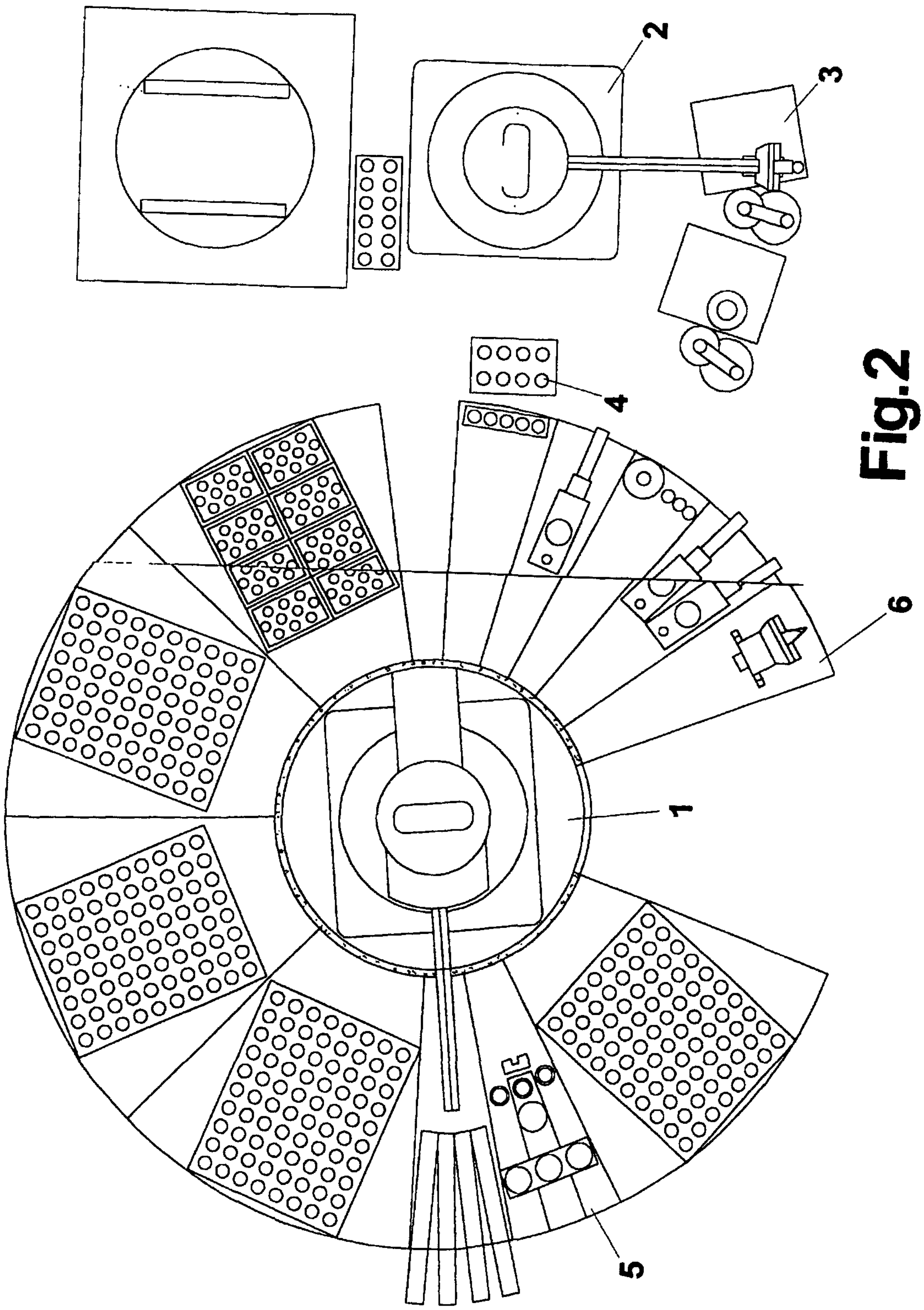


Fig.2